# EUROPÄISCHE PATENTSCHRIFT

(11) **EP 3 140 302 B1**
(45) Veröffentlichungstag und Bekanntmachung des Hinweises auf die Patenterteilung: **21.08.2019**
(21) Anmeldenummer: 15716741.2
(22) Anmeldetag: 01.04.2015
(51) Int. Cl.: C07D 407/14, C07D 409/14, C07D 403/14, H01L 51/50

(54) **MATERIALIEN FÜR ORGANISCHE ELEKTROLUMINESZENZVORRICHTUNGEN**
MATERIALS FOR ORGANIC LIGHT EMITTING DEVICES
MATIÈRES POUR DES DISPOSITIFS ORGANIQUES ÉLECTROLUMINESCENTS

(30) Priorität: 05.05.2014 EP 14001573
(43) Veröffentlichungstag der Anmeldung: 15.03.2017
(73) Patentinhaber: Merck Patent GmbH, 64293 Darmstadt (DE)
(72) Erfinder: PARHAM, Amir Hossain, 60486 Frankfurt am Main (DE); JATSCH, Anja, 60489 Frankfurt am Main (DE); EBERLE, Thomas, 76829 Landau (DE); GROSSMANN, Tobias, 64297 Darmstadt (DE); KROEBER, Jonas Valentin, 60311 Frankfurt am Main (DE); DOBELMANN, Lars, 64295 Darmstadt (DE)
(86) Internationale Anmeldenummer: PCT/EP2015/000705
(87) Internationale Veröffentlichungsnummer: WO 2015/169412

(56) Entgegenhaltungen:
- WO-A1-2014/015931
- WO-A1-2015/014434
- WO-A1-2015/051869
- WO-A2-2011/057706

## Beschreibung

Die vorliegende Erfindung beschreibt Carbazol-, Dibenzofuran-, Dibenzothiophen- und Fluorenderivate, welche mit elektronenarmen Heteroaromaten substituiert sind, insbesondere zur Verwendung als Triplettmatrixmaterialien in organischen Elektrolumineszenzvorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik werden unter anderem Carbazolderivate (z. B. gemäß WO 2014/015931), Indolocarbazolderivate (z. B. gemäß WO 2007/063754 oder WO 2008/056746) oder Indenocarbazolderivate (z. B. gemäß WO 2010/136109 oder WO 2011/000455), insbesondere solche, die mit elektronenarmen Heteroaromaten wie Triazin substituiert sind, als Matrixmaterialien für phosphoreszierende Emitter verwendet. Weiterhin werden beispielsweise Bisdibenzofuranderivate (z. B. gemäß EP 2301926) als Matrixmaterialien für phosphoreszierende Emitter verwendet. Aus der WO 2011/057706 sind Carbazolderivate bekannt, welche mit zwei Triphenyltriazingruppen substituiert sind. Hier sind noch weitere Verbesserungen, insbesondere in Bezug auf das Triplettniveau sowie auf die Sublimationsstabilität wünschenswert. Aus der WO 2011/046182 sind Carbazol-Arylen-Triazin-Derivate bekannt, welche am Triazin mit einer Fluorenylgruppe substituiert sind. Das charakterisierende Merkmal dieser Verbindungen ist die Anwesenheit der Fluorenylgruppe. Verbindungen, die keine Fluorenylgruppe als Substituent aufweisen, sind nicht offenbart. Aus der WO 2013/077352 sind Triazinderivate bekannt, worin die Triazingruppe über eine divalente Arylengruppe an eine Dibenzofurangruppe gebunden ist. Diese Verbindungen sind als Lochblockiermaterialien beschrieben. Eine Verwendung dieser Materialien als Host für phosphoreszierende Emitter ist nicht offenbart. Aus den nicht vorveröffentlichten Anmeldungen WO 2015/014434 und WO 2015/051869 sind Verbindungen bekannt, die den Strukturen der Formel (1) entsprechen.

Generell besteht bei diesen Materialien für die Verwendung als Matrixmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist die Bereitstellung von Verbindungen, welche sich für den Einsatz in einer phosphoreszierenden oder fluoreszierenden OLED eignen, insbesondere als Matrixmaterial. Insbesondere ist es die Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen und die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Es wurde überraschend gefunden, dass Elektrolumineszenzvorrichtungen, die Verbindungen gemäß der folgenden Formel (1) oder Formel (2) enthalten, Verbesserungen gegenüber dem Stand der Technik aufweisen, insbesondere beim Einsatz als Matrixmaterial für phosphoreszierende Dotanden.

Gegenstand der vorliegenden Erfindung ist daher eine Verbindung gemäß der folgenden Formel (1) oder (2), 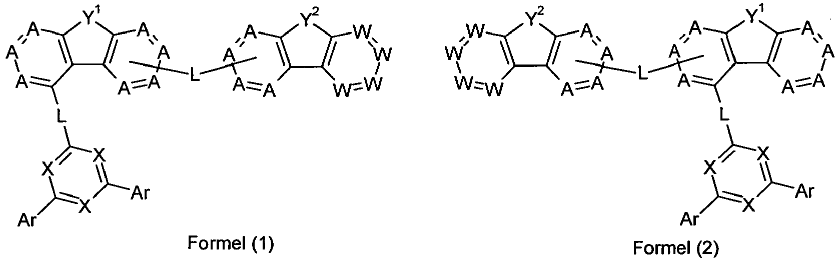 wobei für die verwendeten Symbole und Indizes gilt:
- A: ist bei jedem Auftreten gleich oder verschieden CR oder N, wobei maximal zwei Gruppen A pro Cyclus, bevorzugt maximal eine Gruppe A pro Cyclus, für N stehen und wobei A für C steht, wenn an diese Position eine Gruppe L gebunden ist;
- W: ist bei jedem Auftreten gleich oder verschieden CR oder N, wobei maximal zwei Gruppen W für N stehen, oder zwei benachbarte Gruppen W stehen zusammen für eine Gruppe der folgenden Formel (3), wobei die Verbindung der Formel (1) bzw. Formel (2) maximal eine Gruppe der Formel (3) aufweist, 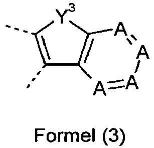 wobei die gestrichelten Bindungen die Verknüpfung dieser Gruppe andeuten und A die oben genannten Bedeutungen aufweist; mit der Maßgabe, dass die Verbindung der Formel (2) keine Gruppe der Formel (3) aufweist, wenn Y¹ für C(R)₂ steht;
- X: ist bei jedem Auftreten gleich oder verschieden CR oder N mit der Maßgabe, dass mindestens eine Gruppe X für N steht;
- Ar: ist bei jedem Auftreten gleich oder verschieden ein aromatisches oder heteroaromatisches Ringsystem mit 5 bis 30 aromatischen Ringatomen, welches durch einen oder mehrere Reste R substituiert sein kann;
- Y¹, Y², Y³: sind bei jedem Auftreten gleich oder verschieden O, NR, S oder C(R)₂, wobei der Rest R, der an N gebunden ist, ungleich H ist;
- L: ist bei jedem Auftreten gleich oder verschieden eine Einfachbindung oder ein aromatisches oder heteroaromatisches Ringsystem mit 5 bis 30 aromatischen Ringatomen, das durch einen oder mehrere Reste R substituiert sein kann;
- R: ist bei jedem Auftreten gleich oder verschieden ausgewählt aus der Gruppe bestehend aus H, D, F, Cl, Br, I, CN, NO₂, N(Ar¹)₂, N(R¹)₂, C(=O)Ar¹, C(=O)R¹, P(=O)(Ar¹)₂, P(Ar¹)₂, B(Ar¹)₂, Si(Ar¹)₃, Si(R¹)₃, einer geradkettigen Alkyl-, Alkoxy- oder Thioalkylgruppe mit 1 bis 20 C-Atomen oder einer verzweigten oder cyclischen Alkyl-, Alkoxyoder Thioalkylgruppe mit 3 bis 20 C-Atomen oder einer Alkenylgruppe mit 2 bis 20 C-Atomen, die jeweils mit einem oder mehreren Resten R¹ substituiert sein kann, wobei eine oder mehrere nichtbenachbarte CH₂-Gruppen durch R¹C=CR¹, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R¹ substituiert sein kann, einer Aryloxy- oder Heteroaryloxygruppe mit 5 bis 40 aromatischen Ringatomen, die mit einem oder mehreren Resten R¹ substituiert sein kann, oder einer Aralkyl- oder Heteroaralkylgruppe mit 5 bis 40 aromatischen Ringatomen, die mit einem oder mehreren Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann;
- Ar¹: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können zwei Reste Ar¹, welche an dasselbe N-Atom, P-Atom oder B-Atom binden, auch durch eine Einfachbindung oder eine Brücke, ausgewählt aus N(R¹), C(R¹)₂, O oder S, miteinander verbrückt sein;
- R¹: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten R¹ miteinander ein mono- oder polycyclisches, aliphatisches Ringsystem bilden;
dabei sind die folgenden Verbindungen von der Erfindung ausgenommen:

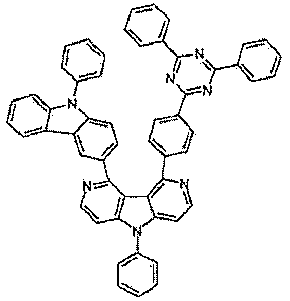

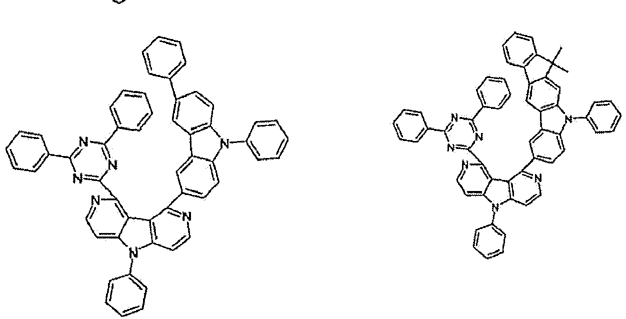

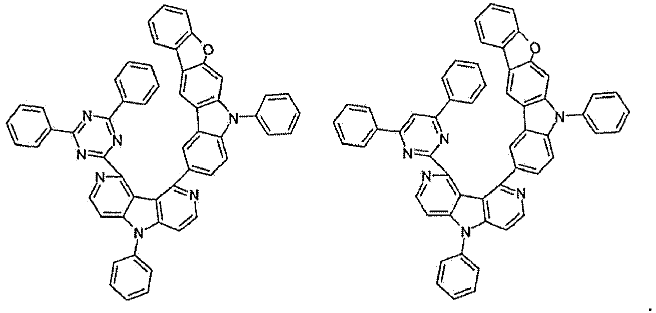

Benachbarte Kohlenstoffatome im Sinne der vorliegenden Erfindung sind Kohlenstoffatome, die direkt miteinander verknüpft sind.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

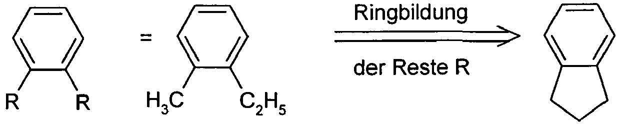

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

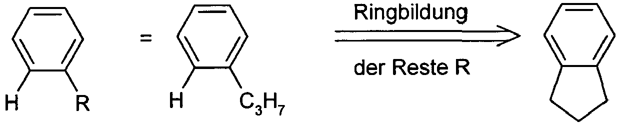

Eine kondensierte Arylgruppe im Sinne der vorliegenden Erfindung ist eine Gruppe, in der zwei oder mehr aromatische Gruppen über eine gemeinsame Kante aneinander ankondensiert, d. h. anelliert, sind, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausführungsform der Erfindung steht W gleich oder verschieden bei jedem Auftreten für CR oder zwei W stehen für eine Gruppe der Formel (3a) und die verbleibenden W stehen für CR, und A steht gleich oder verschieden bei jedem Auftreten für CR. Bevorzugt sind also die Verbindungen der folgenden Formeln (1a), (1b), (2a) bzw. (2b), 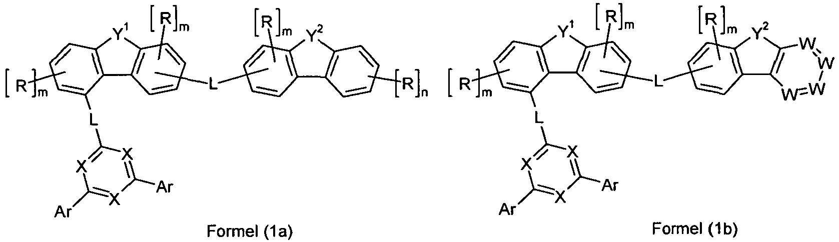 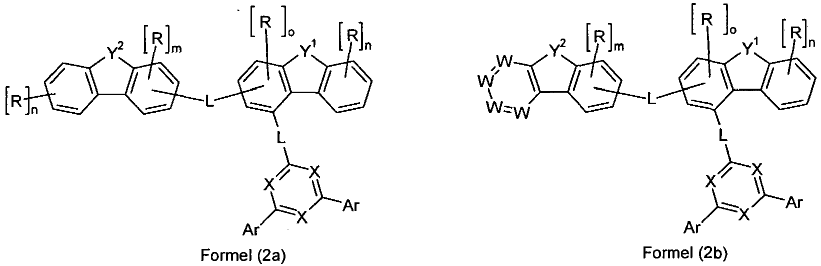 wobei gilt:
zwei benachbarte Gruppen W stehen zusammen für eine Gruppe der folgenden Formel (3a) und die anderen beiden Gruppen W stehen für CR und bevorzugt für CH, 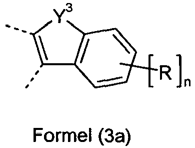 wobei die gestrichelten Bindungen die Verknüpfung dieser Gruppe andeuten;
- n: ist bei jedem Auftreten gleich oder verschieden 0, 1, 2, 3 oder 4;
- m: ist bei jedem Auftreten gleich oder verschieden 0, 1, 2 oder 3;
- o: ist 0, 1 oder 2;
die weiteren verwendeten Symbole weisen die oben genannten Bedeutungen auf;
wobei Verbindungen der Formel (2b), in denen Y¹ für CR₂ steht, von der Erfindung ausgenommen sind.

In einer bevorzugten Ausführungsform der Erfindung stehen mindestens zwei Gruppen X für N, und die gegebenenfalls verbleibende Gruppe X steht für CR, insbesondere für CH. In einer besonders bevorzugten Ausführungsform der Erfindung stehen alle Gruppen X für N. Es handelt sich besonders bevorzugt also um eine Diaryltriazingruppe.

In einer bevorzugten Ausführungsform der Erfindung ist die folgende Gruppe, 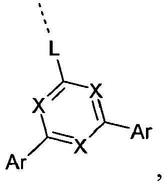 die in Formeln (1) und (2) bzw. den bevorzugten Ausführungsformen gebunden ist, daher ausgewählt aus den folgenden Gruppen (HetAr-1), (HetAr-2) oder (HetAr-3), 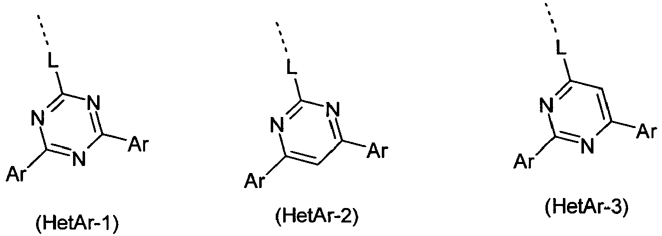 wobei L und Ar die oben genannten Bedeutungen aufweisen und die gestrichelte Bindung die Verknüpfung dieser Gruppe andeutet.

In einer weiteren bevorzugten Ausführungsform der Erfindung stehen Y¹ und Y² gleich oder verschieden bei jedem Auftreten für O, NR, wobei der an den Stickstoff gebundene Rest R ungleich H ist, oder S. Dabei ist es bevorzugt, dass mindestens eine der Gruppen Y¹ und/oder Y² für NR steht und dass in Formel (2) Y¹ für O oder S steht. In einer besonders bevorzugten Ausführungsform der Erfindung stehen Y¹ und Y² gleich oder verschieden für O oder NR, wobei der an den Stickstoff gebundene Rest R ungleich H ist, wobei bevorzugt Y¹ und Y² verschieden sind. Ganz besonders bevorzugt steht Y¹ für O und Y² für NR, wobei der an den Stickstoff gebundene Rest R ungleich H ist.

In einer weiteren bevorzugten Ausführungsform der Erfindung steht Y³, falls die Verbindung eine Gruppe der Formel (3) enthält, für O, NR, wobei der an den Stickstoff gebundene Rest R ungleich H ist, oder C(R)₂, besonders bevorzugt für NR, wobei der an den Stickstoff gebundene Rest R ungleich H ist, oder C(R)₂ und ganz besonders bevorzugt für C(R)₂.

Wenn die erfindungsgemäße Verbindung eine Gruppe der Formel (3) enthält, so kann diese in verschiedenen Positionen gebunden sein. Dies wird im Folgenden schematisch anhand von bevorzugten Ausführungsformen, in denen die Gruppen A und die anderen Gruppen W für CR stehen, durch die Formeln (A) bis (F) dargestellt: 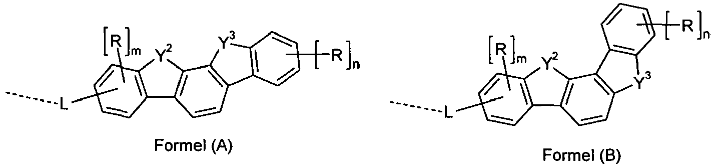 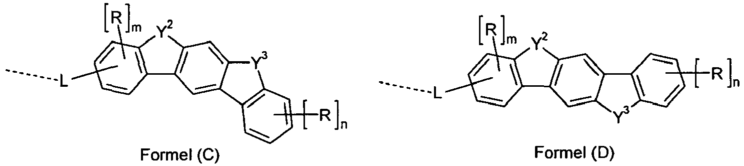 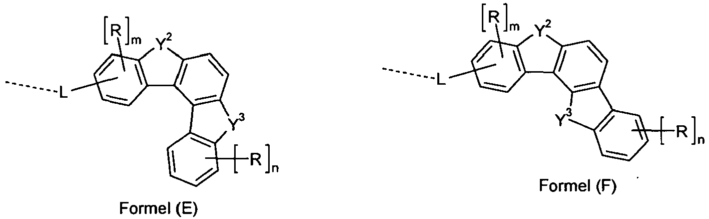 wobei die verwendeten Symbole und Indizes die oben genannten Bedeutungen aufweisen und die gestrichelte Bindung die Verknüpfung in der erfindungsgemäßen Verbindung darstellt.

In einer weiteren bevorzugten Ausführungsform der Erfindung steht L gleich oder verschieden bei jedem Auftreten für eine Einfachbindung oder ein aromatisches oder heteroaromatisches Ringsystem mit 5 bis 24 aromatischen Ringatomen, das durch einen oder mehrere Reste R substituiert sein kann. Besonders bevorzugt steht L gleich oder verschieden bei jedem Auftreten für eine Einfachbindung oder ein aromatisches Ringsystem mit 6 bis 12 aromatischen Ringatomen oder ein heteroaromatisches Ringsystem mit 6 bis 13 aromatischen Ringatomen, das jeweils durch einen oder mehrere Reste R substituiert sein kann, bevorzugt aber unsubstituiert ist. Ganz besonders bevorzugt steht L für eine Einfachbindung. Beispiele für geeignete aromatische oder heteroaromatische Ringsysteme L sind ausgewählt aus der Gruppe bestehend aus ortho-, meta- oder para-Phenylen, Biphenyl, Fluoren, Pyridin, Pyrimidin, Triazin, Dibenzofuran, Dibenzothiophen und Carbazol, die jeweils durch einen oder mehrere Reste R substituiert sein können, bevorzugt aber unsubstituiert sind.

In einer weiteren bevorzugten Ausführungsform der Erfindung steht Ar gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste R substituiert sein kann, bevorzugt aber unsubstituiert ist. Beispiele für geeignete Gruppen Ar sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R substituiert sein können, bevorzugt aber unsubstituiert sind.

Beispiele für geeignete Gruppen Ar sind die im Folgenden aufgeführten Strukturen Ar-1 bis Ar-19, 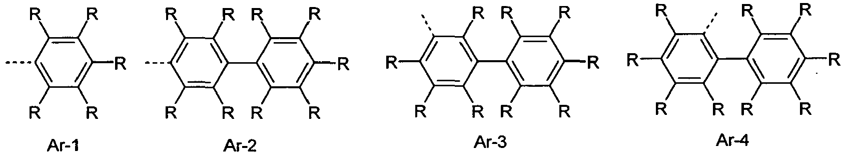 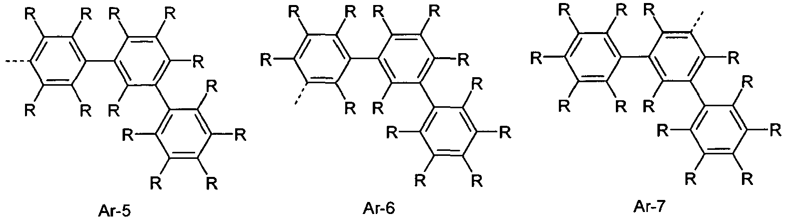 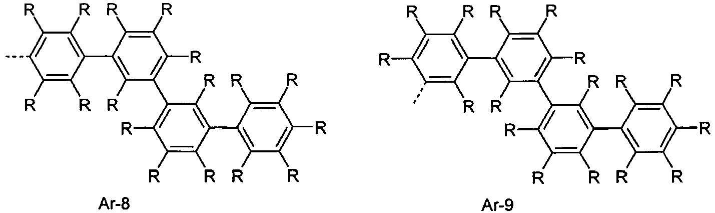 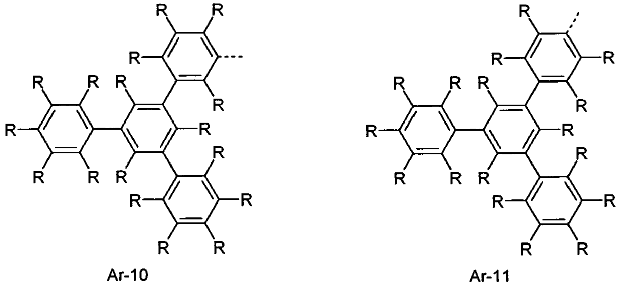 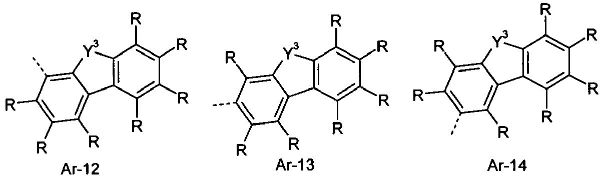 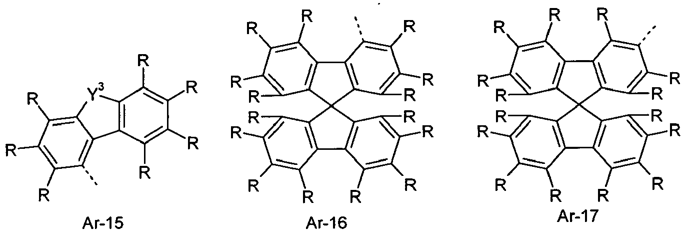 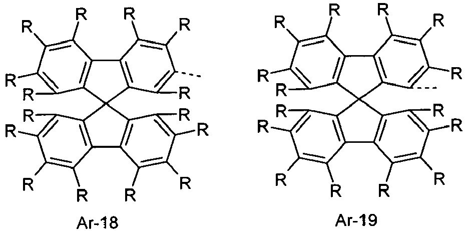 wobei Y³ und R die oben genannten Bedeutungen aufweisen und die gestrichelte Bindung die Bindung an die Sechsring-Heteroarylgruppe in Formel (1) bzw. Formel (2) darstellt.

In einer weiteren bevorzugten Ausführungsform der Erfindung ist in Verbindungen der Formel (1a) bzw. (2a) der Index n = 0, 1, 2 oder 3, besonders bevorzugt 0, 1 oder 2 und ganz besonders bevorzugt 0 oder 1.

In nochmals einer weiteren bevorzugten Ausführungsform der Erfindung ist in Verbindungen der Formel (1a) bzw. (2a) der Index m = 0, 1 oder 2, besonders bevorzugt 0 oder 1 und ganz besonders bevorzugt 0.

In nochmals einer weiteren bevorzugten Ausführungsform der Erfindung ist in Verbindungen der Formel (1a) bzw. (2a) der Index o = 0 oder 1, besonders bevorzugt 0.

Im Folgenden werden bevorzugte Substituenten R beschrieben. Dabei hängen die bevorzugten Substituenten davon ab, ob sie an A bzw. W oder an Ar oder an Y¹, Y² oder Y³ gebunden sind und hängen auch davon ab, wie Y¹, Y² bzw. Y³ gewählt sind.

Wenn A für CR steht bzw. wenn W für CR steht bzw. wenn die Gruppen Ar durch Substituenten R substituiert sind, dann sind diese Substituenten R bevorzugt gewählt aus der Gruppe bestehend aus H, D, F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R¹ substituiert sein kann, bevorzugt aber unsubstituiert ist, oder einer Aralkyl- oder Heteroaralkylgruppe mit 5 bis 25 aromatischen Ringatomen, die mit einem oder mehreren Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann.

Besonders bevorzugt sind diese Substituenten R ausgewählt aus der Gruppe bestehend aus H, D, F, CN, N(Ar¹)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten R¹ substituiert sein kann, bevorzugt aber unsubstituiert ist; dabei können optional zwei Substituenten R, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R¹ substituiert sein kann, bevorzugt aber unsubstituiert ist.

Ganz besonders bevorzugt sind die Substituenten R ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten R¹ substituiert sein kann, bevorzugt aber unsubstituiert ist. Beispiele für geeignete Substituenten R sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R¹ substituiert sein können, bevorzugt aber unsubstituiert sind. Dabei sind geeignete Strukturen R die gleichen Strukturen, wie sie vorne für Ar-1 bis Ar-19 abgebildet sind, wobei diese Strukturen durch R¹ statt R substituiert sind. Für Substituenten R an den Gruppen Ar sind außer den oben genannten Gruppen auch geradkettige Alkylgruppen mit 1 bis 4 C-Atomen oder verzweigte oder cyclische Alkylgruppen mit 3 bis 6 C-Atomen besonders bevorzugt.

Wenn Y¹ bzw. Y² bzw. Y³ für NR steht, ist es bevorzugt, wenn der Rest R, der an dieses Stickstoffatom gebunden ist, bei jedem Auftreten gleich oder verschieden für ein aromatisches oder heteroaromatisches Ringsystem mit 5 bis 24 aromatischen Ringatomen steht, das jeweils mit einem oder mehreren Resten R¹ substituiert sein kann, besonders bevorzugt für ein aromatisches oder heteroaromatisches Ringsystem mit 6 bis 18 aromatischen Ringatomen, das mit einem oder mehreren Resten R¹ substituiert sein kann. Beispiele für geeignete Substituenten R sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1,3,5-Triazinyl, 4,6-Diphenyl-1,3,5-triazinyl, 1-, 2-, 3- oder 4-Dibenzofuranyl, 1-, 2-, 3- oder 4-Dibenzothienyl und 1-, 2-, 3- oder 4-Carbazolyl, wobei die Carbazolylgruppe am Stickstoffatom durch einen Rest R¹ ungleich H oder D substituiert ist. Dabei können diese Gruppen jeweils durch einen oder mehrere Reste R¹ substituiert sein, sind bevorzugt aber unsubstituiert. Dabei sind geeignete Strukturen R die gleichen Strukturen, wie sie vorne für Ar-1 bis Ar-18 abgebildet sind, wobei diese Strukturen durch R¹ statt R substituiert sind.

Wenn Y¹ bzw. Y² bzw. Y³ für C(R)₂ steht, ist es bevorzugt, wenn die Reste R, die an dieses Kohlenstoffatom gebunden sind, bei jedem Auftreten gleich oder verschieden für eine geradkettigen Alkylgruppe mit 1 bis 10 C-Atomen oder eine verzweigte oder cyclische Alkylgruppe mit 3 bis 10 C-Atomen oder eine Alkenylgruppe mit 2 bis 10 C-Atomen stehen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, oder für ein aromatisches oder heteroaromatisches Ringsystem mit 5 bis 24 aromatischen Ringatomen, das jeweils mit einem oder mehreren Resten R¹ substituiert sein kann; dabei können optional die beiden Substituenten R ein monocyclisches oder polycyclisches, aliphatisches, aromatisches oder heteroaromatisches Ringsystem bilden, das mit einem oder mehreren Resten R¹ substituiert sein kann. Durch Ringbildung der beiden Substituenten R wird ein Spirosystem aufgespannt, beispielsweise ein Spirobifluoren bzw. Derivat eines Spirobifluorens, wenn die Gruppen R für Phenylgruppen stehen.

In einer weiteren bevorzugten Ausführungsform der Erfindung ist R¹ bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Wenn die erfindungsgemäße Verbindung mit aromatischen oder heteroaromatischen Gruppen R bzw. R¹ bzw. Ar bzw. Ar¹ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Die oben genannten Bevorzugungen können einzeln oder gemeinsam auftreten. Es ist bevorzugt, wenn die oben genannten Bevorzugungen gemeinsam auftreten.

Bevorzugt sind somit Verbindungen der oben genannten Formel (1), (2), (1a), (1b), (2a) bzw. (2b) für die gilt:
- X: ist gleich oder verschieden bei jedem Auftreten CR oder N, wobei mindestens zwei Gruppen X für N stehen und die gegebenenfalls verbleibende Gruppe X für CR , insbesondere für CH, steht;
- Y¹, Y²: stehen gleich oder verschieden bei jedem Auftreten für O, NR, wobei der an den Stickstoff gebundene Rest R ungleich H ist, oder S; dabei steht bevorzugt mindestens eine der Gruppen Y¹ und/oder Y² für NR und Y¹ in Formeln (2), (2a) und (2b) für O oder S;
- Y³: steht für O, NR, wobei der an den Stickstoff gebundene Rest R ungleich H ist, oder CR₂;
- L: steht gleich oder verschieden bei jedem Auftreten für eine Einfachbindung oder ein aromatisches oder heteroaromatisches Ringsystem mit 5 bis 24 aromatischen Ringatomen, insbesondere mit 6 bis 12 aromatischen Ringatomen, das durch einen oder mehrere Reste R substituiert sein kann, bevorzugt aber unsubstituiert ist;
- Ar: steht gleich oder verschieden bei jedem Auftreten für ein aromatisches oder heteroaromatisches Ringsystem mit 6 bis 24 aromatischen Ringatomen, das durch einen oder mehrere Reste R substituiert sein kann, bevorzugt aber unsubstituiert ist;
- n: ist in Formel (1a), (1b), (2a) bzw. (2b) gleich oder verschieden bei jedem Auftreten 0, 1, 2 oder 3, bevorzugt 0, 1 oder 2;
- m: ist in Formel (1a), (1b), (2a) bzw. (2b) gleich oder verschieden bei jedem Auftreten 0, 1 oder 2, bevorzugt 0 oder 1;
- o: ist in Formel (1a), (1b), (2a) bzw. (2b) 0 oder 1;
- R: ist gleich oder verschieden bei jedem Auftreten gewählt aus der Gruppe bestehend aus H, D, F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R¹ substituiert sein kann, oder einer Aralkyl- oder Heteroaralkylgruppe mit 5 bis 25 aromatischen Ringatomen, die mit einem oder mehreren Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann;
- R¹: ist wie oben definiert.

Besonders bevorzugt sind Verbindungen der oben genannten Formeln (1), (2), (1a), (1b), (2a) bzw. (2b), für die gilt:
- X: ist N;
- Y¹, Y²: stehen gleich oder verschieden bei jedem Auftreten für O oder NR, wobei der an den Stickstoff gebundene Rest R ungleich H ist; dabei gilt bevorzugt: Y¹ steht für O und Y² steht für NR, oder Y¹ steht für NR und Y² steht für O, oder Y¹ und Y² stehen für NR;
- Y³: steht für NR, wobei der an den Stickstoff gebundene Rest R ungleich H ist, oder CR₂;
- L: steht für eine Einfachbindung;
- Ar: steht gleich oder verschieden bei jedem Auftreten für ein aromatisches oder heteroaromatisches Ringsystem mit 6 bis 18 aromatischen Ringatomen, bevorzugt mit 6 bis 13 aromatischen Ringatomen, das jeweils durch einen oder mehrere Reste R substituiert sein kann, bevorzugt aber unsubstituiert ist;
- n: ist in Formeln (1a) bis (2b) bei jedem Auftreten gleich oder verschieden 0 oder 1;
- m: ist in Formeln (1a) bis (2b) 0;
- o: ist in Formeln (1a) bis (2b) 0;
- R: ist gleich oder verschieden bei jedem Auftreten ausgewählt aus der Gruppe bestehend aus H, D, F, CN, N(Ar¹)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 12 aromatischen Ringatomen, das jeweils mit einem oder mehreren nicht-aromatischen Resten R¹ substituiert sein kann, bevorzugt aber unsubstituiert ist; dabei können optional zwei Substituenten R, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R¹ substituiert sein kann, bevorzugt aber unsubstituiert ist;
- R¹: ist wie oben definiert.

Bei den Gruppen enthaltend Y¹ und Y² handelt es sich für Y¹ bzw. Y² = O um Dibenzofuranderivate, für Y¹ bzw. Y² = NR um Carbazolderivate, für Y¹ bzw. Y² = S um Dibenzothiophenderivate und für Y¹ bzw. Y² = C(R)₂ um Fluorenderivate. Wenn die beiden Reste R in der CR₂-Gruppe miteinander einen Ring bilden, entsteht hieraus ein Spirosystem, beispielsweise ein Spirobifluorenderivat, wenn die Reste R für Phenyl stehen.

Diese Gruppen können in verschiedenen Positionen miteinander verknüpft werden und können in verschiedenen Positionen substituiert sein. Die Nummerierung der Positionen der einzelnen Gruppen ist im Folgenden durch die Formeln (4) und (5) schematisch dargestellt, wobei sich die erfindungsgemäßen Verbindungen durch Verknüpfung der Formeln (4) und (5) miteinander über eine Gruppe L ergeben:

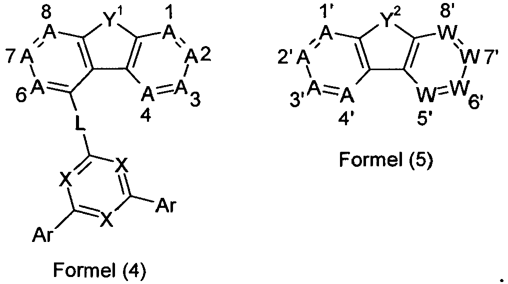

Für Verbindungen der Formel (1) eignen sich somit die Verknüpfungen 1-1', 1-2', 1-3', 1-4', 2-1', 2-2', 2-3', 2-4', 3-1', 3-2', 3-3', 3-4', 4-1', 4-2', 4-3' und 4-4'. Dabei bedeutet beispielsweise eine Verknüpfung 1-2', dass die Gruppe der Formel (4) über die 1-Position und die Gruppe der Formel (5) über die 2'-Position miteinander über eine Gruppe L verknüpft sind.

Für Verbindungen der Formel (2) eignen sich die Verknüpfungen 6-1', 6-2', 6-3', 6-4', 7-1', 7-2', 7-3', 7-4', 8-1', 8-2', 8-3' und 8-4'.

Dabei können prinzipiell alle oben genannten Verknüpfungsmuster mit den oben genannten Bevorzugungen für die Symbole und Indizes kombiniert werden. Das bevorzugte Verknüpfungsmuster hängt von der Wahl von Y¹ bzw. Y² ab.

Wenn Y¹ für O, NR oder S steht, ist die Gruppe der Formel (4) bevorzugt über die 1-Position, die 2-Position oder die 3-Position in Formel (1) bzw. über die 8-Position in Formel (2) verknüpft. Für Verbindungen der Formel (1) mit Y¹ = O, NR oder S sind somit die Verknüpfungen 1-1', 1-2', 1-3', 1-4', 2-1', 2-2', 2-3', 2-4', 3-1', 3-2', 3-3' und 3-4' und für Verbindungen der Formel (2) die Verknüpfungen 8-1', 8-2', 8-3' und 8-4' bevorzugt.

Bevorzugte Ausführungsformen der erfindungsgemäßen Verbindungen sind somit die Verbindungen der folgenden Formeln (6), (7), (8) und (9) 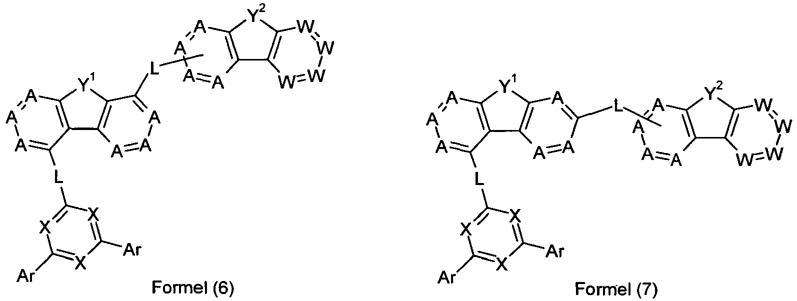 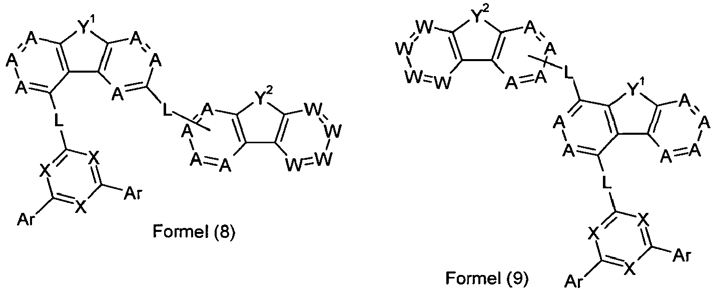 wobei Y¹ für O, NR oder S steht, die weiteren verwendeten Symbole die oben genannten Bedeutungen aufweisen und A bevorzugt gleich oder verschieden für CR steht und W bevorzugt gleich oder verschieden bei jedem Auftreten für CR steht oder zwei Gruppen W stehen für eine Gruppe der oben genannten Formel (3) und die anderen Gruppen W stehen für CR. Besonders bevorzugt stehen A und W gleich oder verschieden bei jedem Auftreten für CR.

Wenn Y¹ für C(R)₂ steht, ist die Gruppe der Formel (4) bevorzugt über die 2-Position in Formel (1) bzw. über die 7-Position in Formel (2) verknüpft. Für Verbindungen der Formel (1) mit Y¹ = C(R)₂ sind somit die Verknüpfungen 2-1', 2-2', 2-3' und 2-4' und für Verbindungen der Formel (2) die Verknüpfungen 7-1', 7-2', 7-3' und 7-4' bevorzugt. Insbesondere handelt es sich für Y = C(R)₂ um eine Verbindung der Formel (1) und nicht um eine Verbindung der Formel (2). Eine bevorzugte Ausführungsform der erfindungsgemäßen Verbindungen ist für Y¹ = CR₂ somit die Verbindung der folgenden Formel (10), 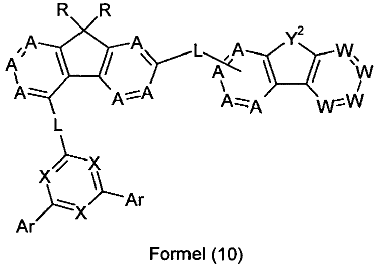 wobei die verwendeten Symbole die oben genannten Bedeutungen aufweisen und wobei A und W bevorzugt gleich oder verschieden bei jedem Auftreten für CR stehen.

Wenn Y² für O steht, ist die Gruppe der Formel (5) bevorzugt über die 1'-, 3'- oder die 4'-Position verknüpft. Für Verbindungen der Formel (1) sind somit die Verknüpfungen 1-1', 1-3', 1-4', 2-1', 2-3', 2-4', 3-1', 3-3', 3-4', 4-1', 4-3' und 4-4' bevorzugt. Für Verbindungen der Formel (2) sind die Verknüpfungen 6-1', 6-3', 6-4', 7-1', 7-3', 7-4', 8-1', 8-3' und 8-4' bevorzugt.

Wenn Y² für NR steht, ist die Gruppe der Formel (5) bevorzugt über die 2'- oder die 3'-Position verknüpft. Für Verbindungen der Formel (1) sind somit die Verknüpfungen 1-2', 1-3', 2-2', 2-3', 3-2', 3-3', 4-2' und 4-3' bevorzugt. Für Verbindungen der Formel (2) sind die Verknüpfungen 6-2', 6-3', 7-2', 7-3', 8-2' und 8-3' bevorzugt.

Wenn Y² für S steht, ist die Gruppe der Formel (5) bevorzugt über die 1-, die 2-, die 3- oder die 4-Position verknüpft. Für Verbindungen der Formel (1) sind somit die Verknüpfungen 1-1', 1-2 ,1-3', 1-4', 2-1', 2-2', 2-3', 2-4', 3-1', 3-2', 3-3', 3-4', 4-1', 4-2', 4-3' und 4-4' bevorzugt. Für Verbindungen der Formel (2) sind die Verknüpfungen 6-1', 6-2', 6-3', 6-4', 7-1', 7-2', 7-3', 7-4', 8-1', 8-2', 8-3' und 8-4' bevorzugt.

Wenn Y² für C(R)₂ steht, ist die Gruppe der Formel (5) bevorzugt über die 1'-, die 2'- oder die 4'-Position verknüpft, besonders bevorzugt über die 2'- oder die 4'-Position. Für Verbindungen der Formel (1) sind somit die Verknüpfungen 1-1', 2-1', 3-1', 4-1', 1-2', 2-2', 3-2', 4-2', 1-4', 2-4', 3-4' und 4-4' und für Verbindungen der Formel (2) die Verknüpfungen 6-1', 7-1', 8-1', 6-2', 7-2', 8-2', 6-4', 7-4' und 8-4' bevorzugt.

Dabei hängt das genaue bevorzugte Verknüpfungsmuster für die oben angegebenen Gruppen Y² jeweils von der verwendeten Gruppe Y¹ ab, wie oben beschrieben.

Bevorzugt sind Verbindungen der oben genannten Formel (8), in denen Y² für NR, O oder S steht und die Gruppe der Formel (5) über die 3'-Position verknüpft ist, gemäß der folgenden Formel (8a) und bevorzugt gemäß der folgenden Formel (8b), 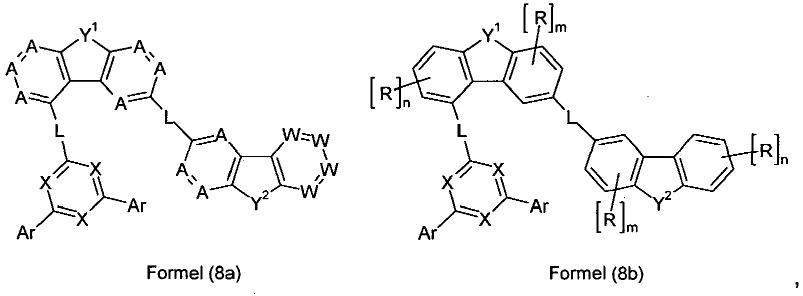 wobei Y¹ für O, NR oder S, Y² für NR, O oder S steht und die weiteren verwendeten Symbole und Indizes die oben genannten Bedeutungen aufweisen und insbesondere die oben als bevorzugt und besonders bevorzugt genannten Bedeutungen aufweisen.

Besonders bevorzugt sind somit die Verbindungen der folgenden Formel (8c), 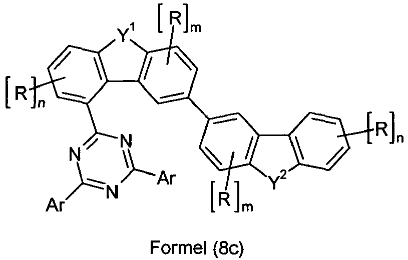 wobei für die verwendeten Symbole und Indizes gilt:
- Y¹, Y²: stehen gleich oder verschieden bei jedem Auftreten für O, NR, wobei der an den Stickstoff gebundene Rest R ungleich H ist, oder S, mit der Maßgabe, dass mindestens eine der Gruppen Y¹ und/oder Y² für NR steht, und stehen bevorzugt für O oder NR, wobei der an den Stickstoff gebundene Rest R ungleich H ist;
- Ar: steht gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, das jeweils durch einen oder mehrere Reste R substituiert sein kann, bevorzugt aber unsubstituiert ist;
- n: ist gleich oder verschieden bei jedem Auftreten 0, 1, 2 oder 3, bevorzugt 0, 1 oder 2, besonders bevorzugt 0 oder 1;
- m: ist gleich oder verschieden bei jedem Auftreten 0, 1 oder 2, bevorzugt 0 oder 1, besonders bevorzugt 0;
- o: ist 0 oder 1, besonders bevorzugt 0;
- R: ist gleich oder verschieden bei jedem Auftreten gewählt aus der Gruppe bestehend aus H, D, F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R¹ substituiert sein kann, oder einer Aralkyl- oder Heteroaralkylgruppe mit 5 bis 25 aromatischen Ringatomen, die mit einem oder mehreren Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann; bevorzugt ist R gleich oder verschieden bei jedem Auftreten ausgewählt aus der Gruppe bestehend aus H, D, F, CN, N(Ar¹)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 12 aromatischen Ringatomen, das jeweils mit einem oder mehreren nicht-aromatischen Resten R¹ substituiert sein kann, bevorzugt aber unsubstituiert ist; dabei können optional zwei Substituenten R, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R¹ substituiert sein kann, bevorzugt aber unsubstituiert ist.

Dabei hat R¹ die oben genannten Bedeutungen und wenn Y¹ und/oder Y² für NR steht, so ist dieser Rest R bevorzugt aus den Substituenten ausgewählt, wie sie oben als bevorzugte Substituenten an R angegeben sind.

Ganz besonders bevorzugt ist eine Verbindung der folgenden Formel (8d), 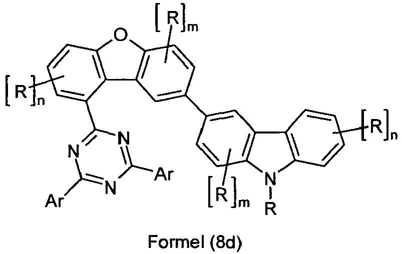 wobei die verwendeten Symbole und Indizes die unter Formel (8c) genannten Bedeutungen aufweisen und der an den Stickstoff gebundene Rest R für ein aromatisches oder heteroaromatisches Ringsystem mit 5 bis 24 aromatischen Ringatomen steht, das jeweils mit einem oder mehreren Resten R¹ substituiert sein kann, bevorzugt für ein aromatisches oder heteroaromatisches Ringsystem mit 6 bis 18 aromatischen Ringatomen, das mit einem oder mehreren Resten R¹ substituiert sein kann.

Beispiele für geeignete erfindungsgemäße Verbindungen sind die nachstehend gezeigten Strukturen.

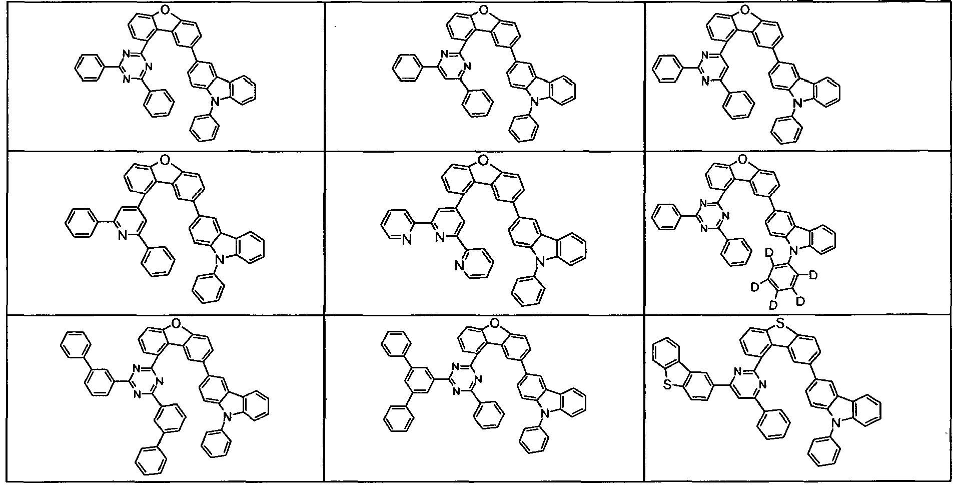

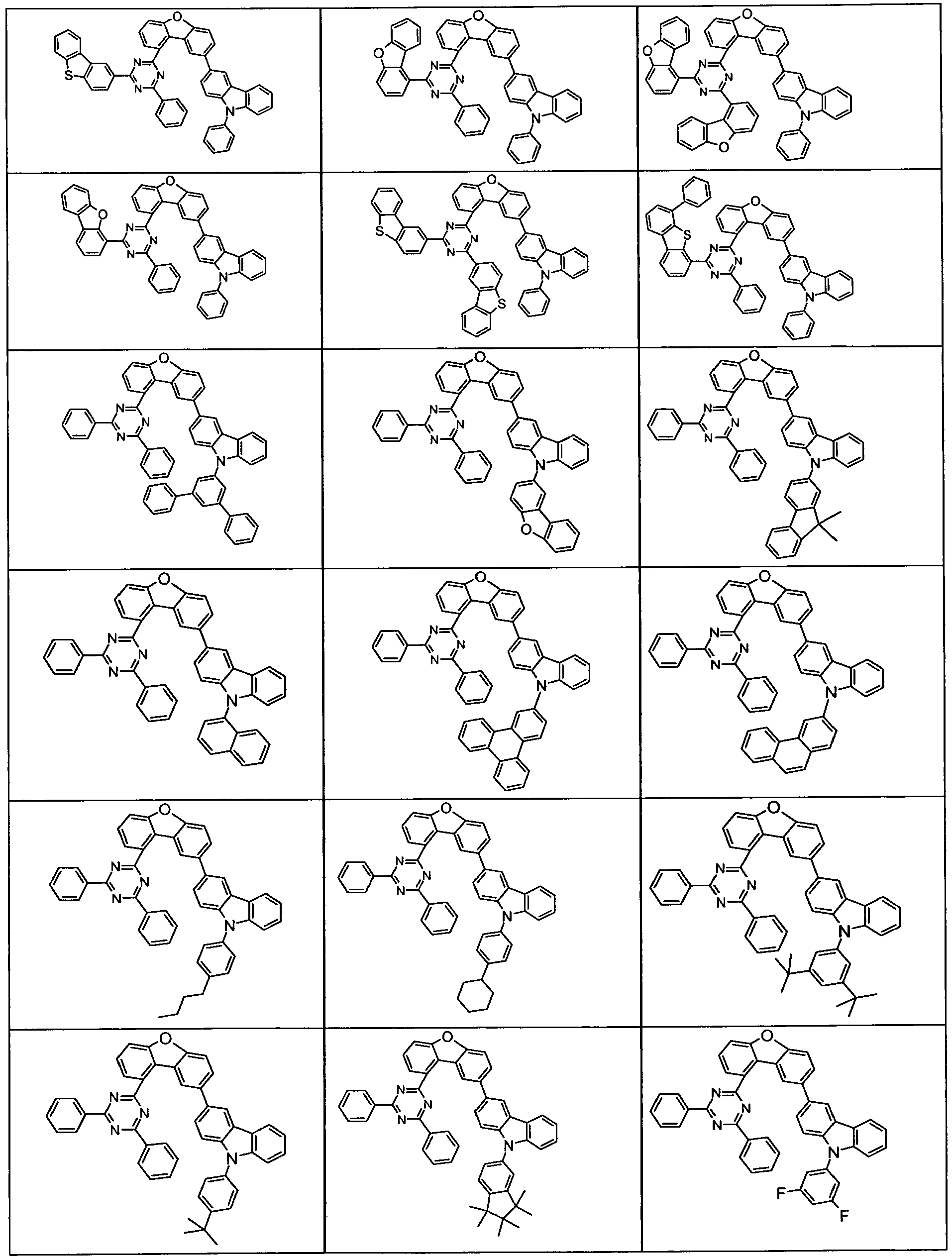

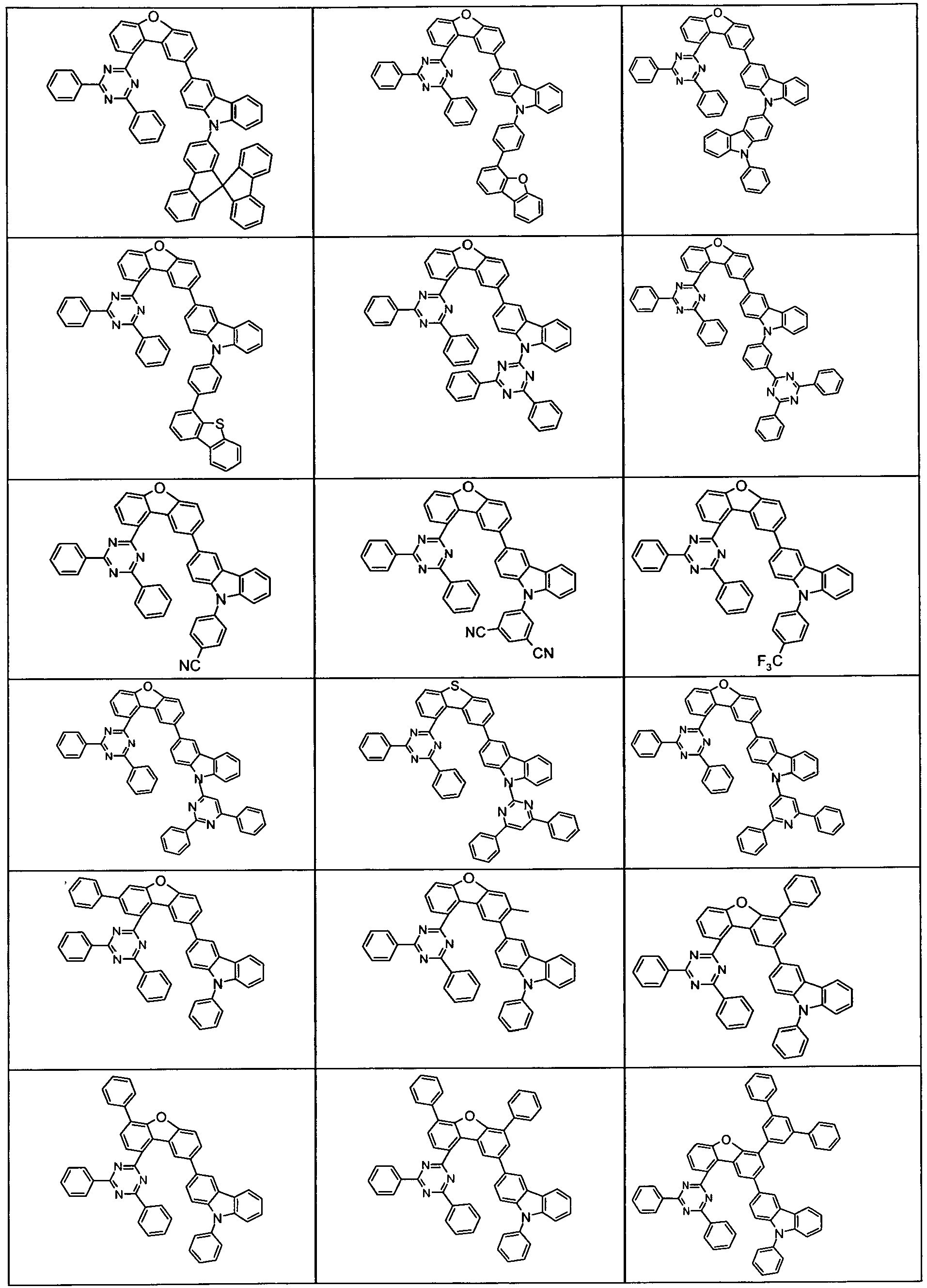

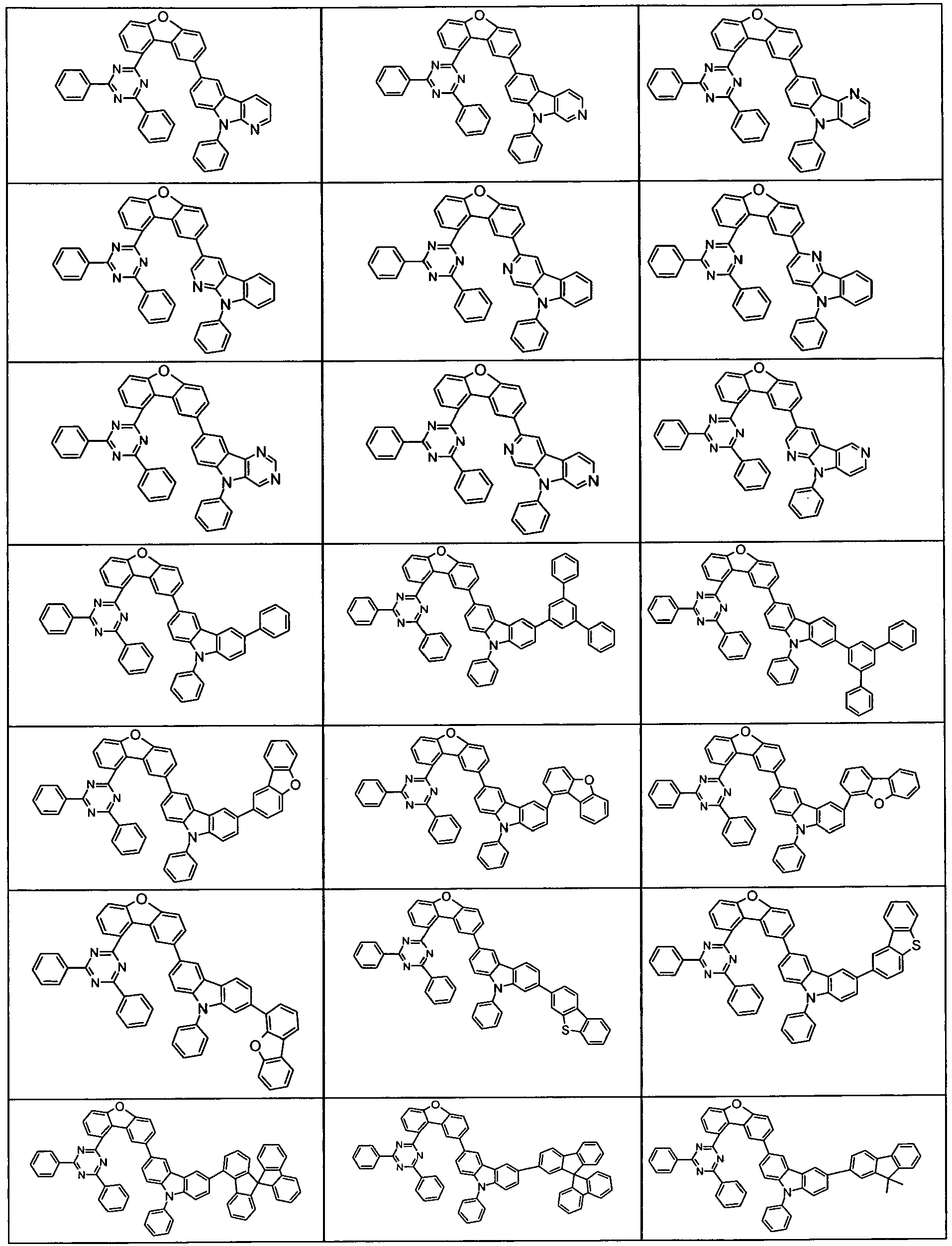

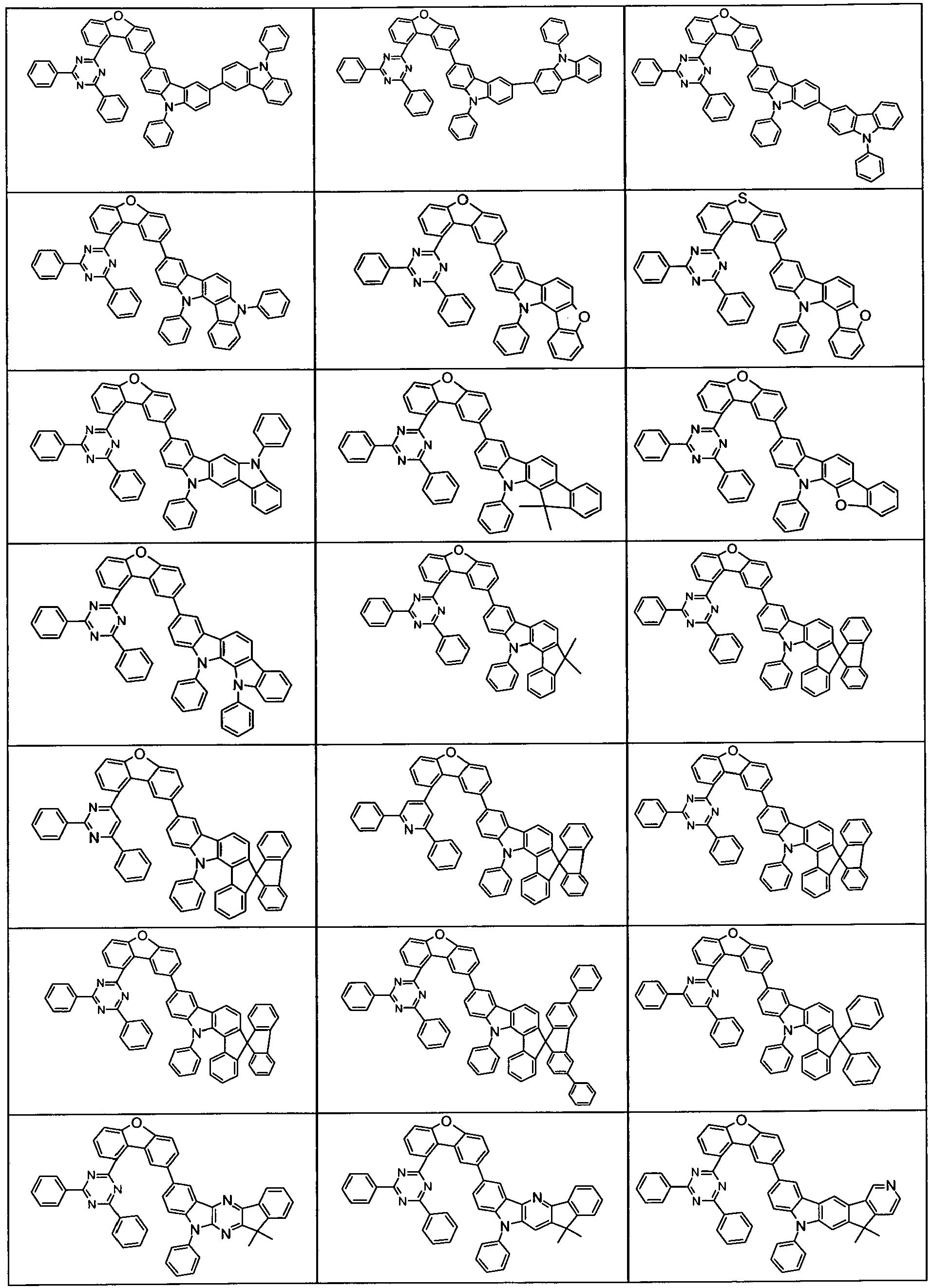

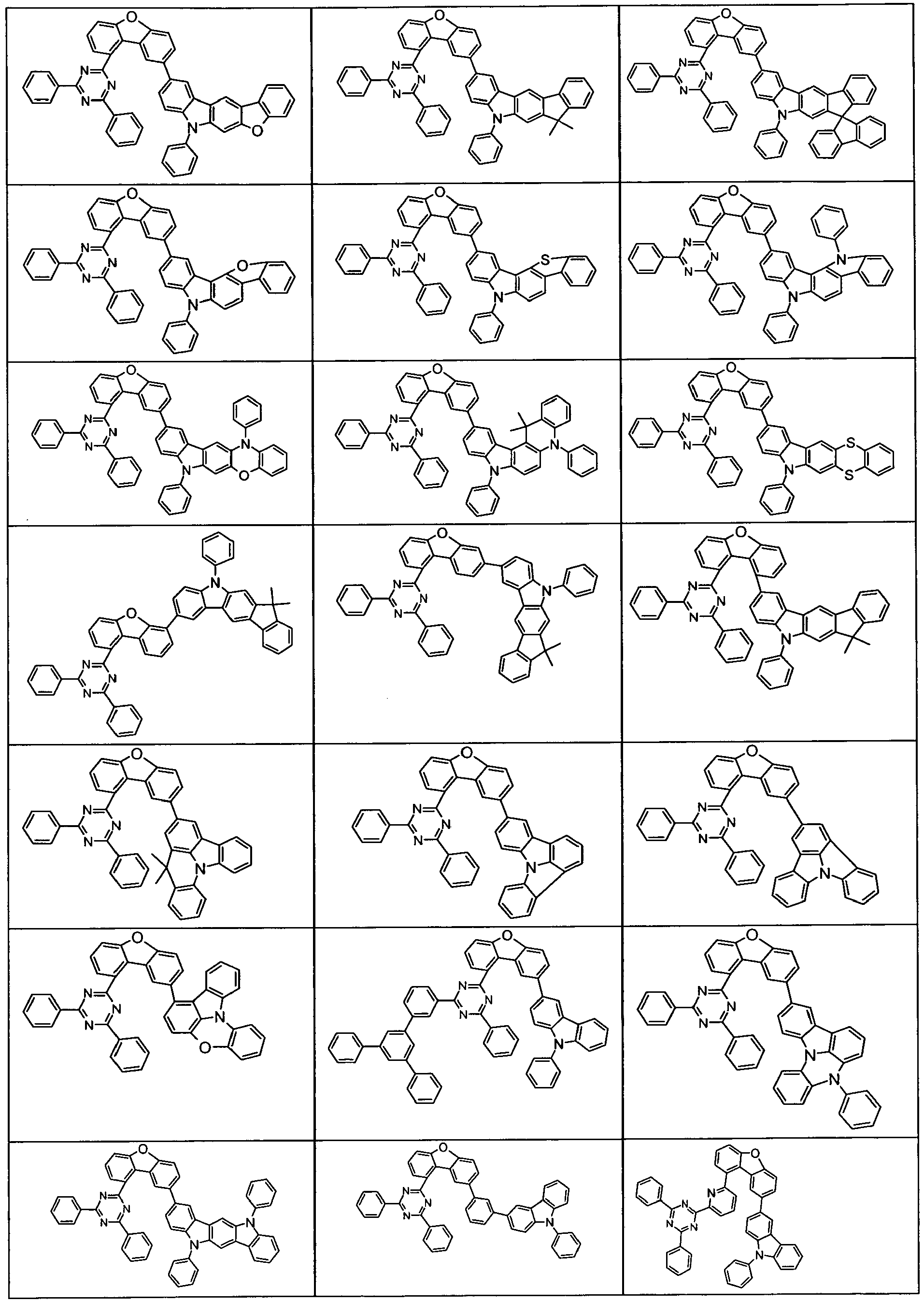

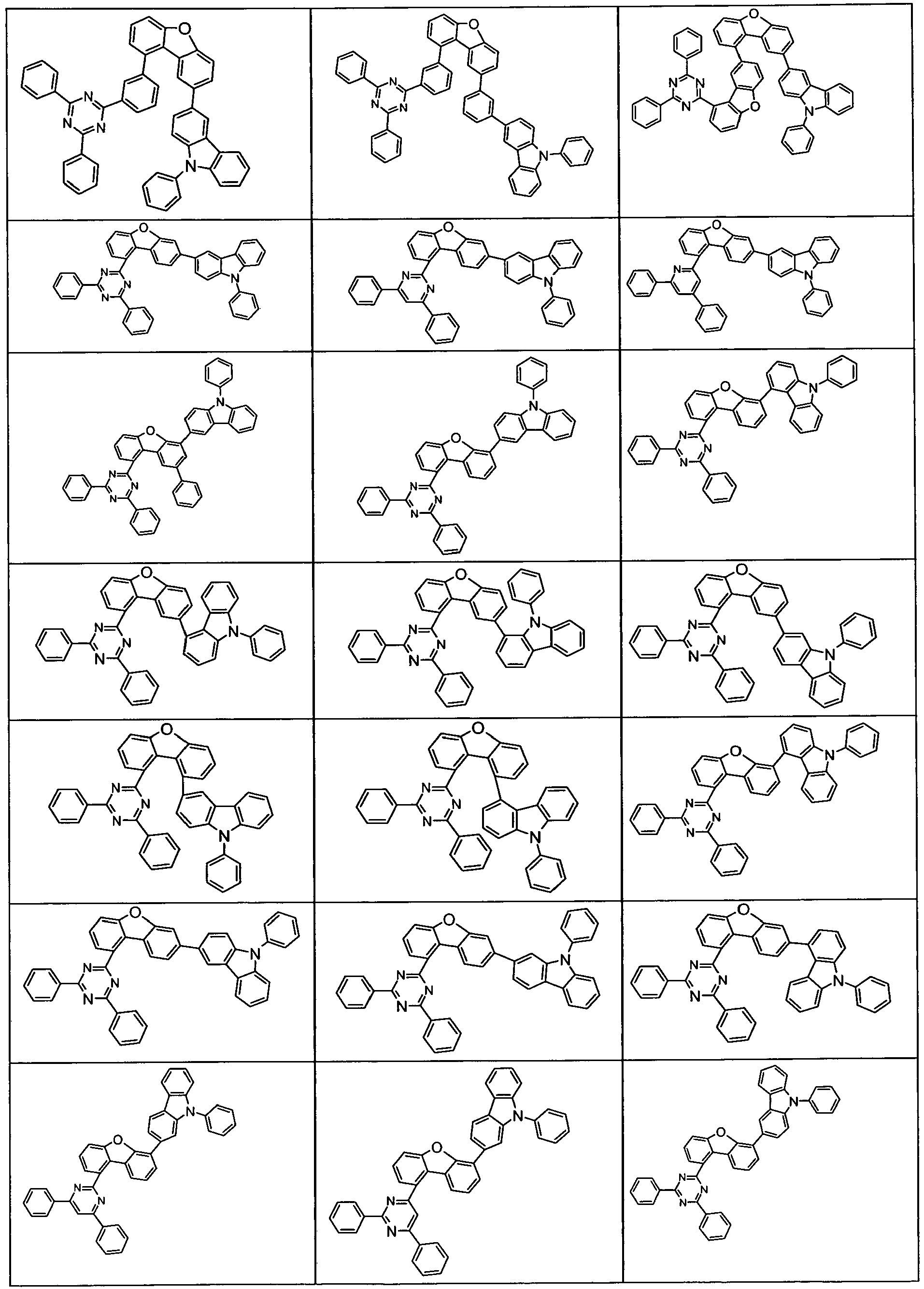

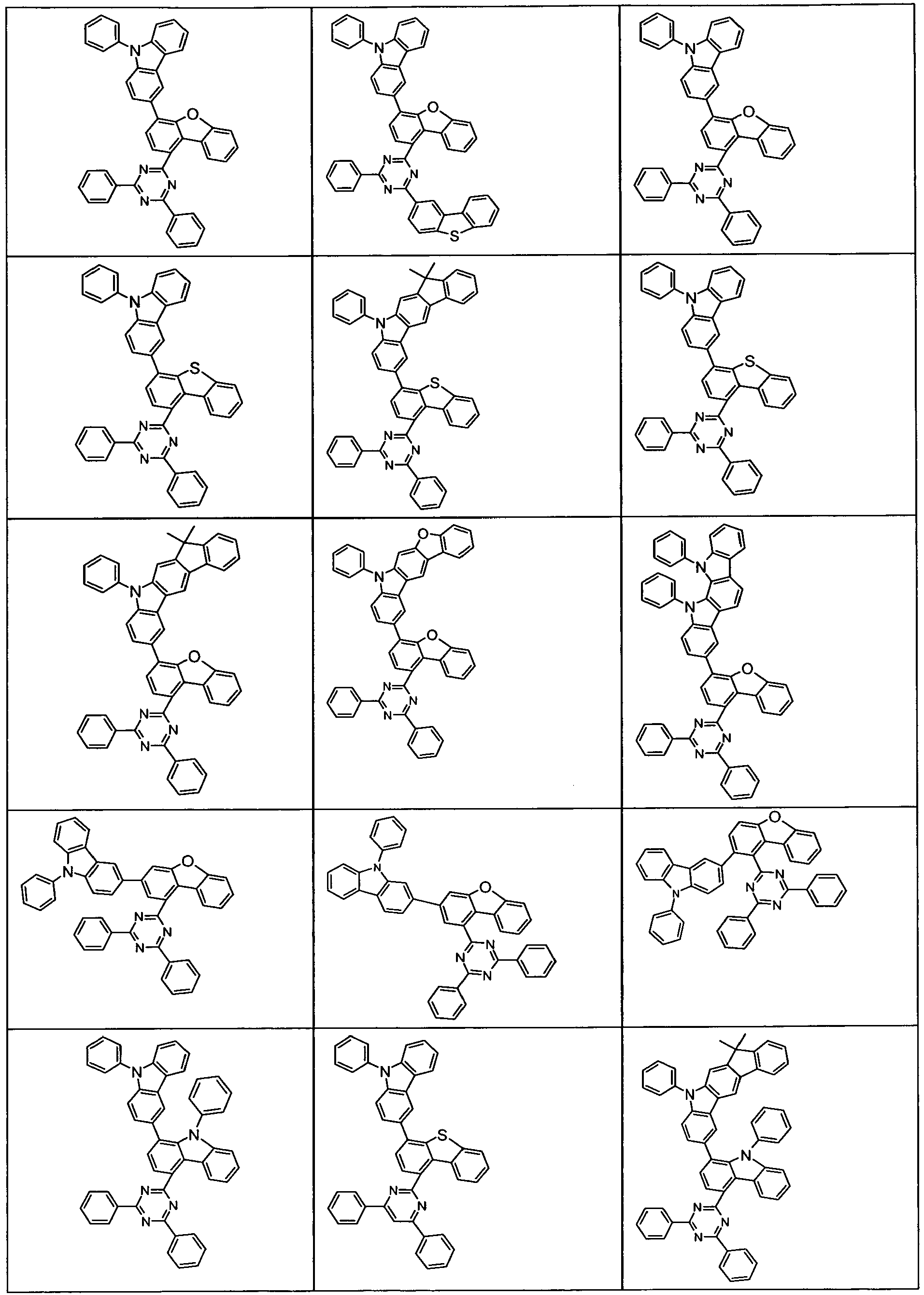

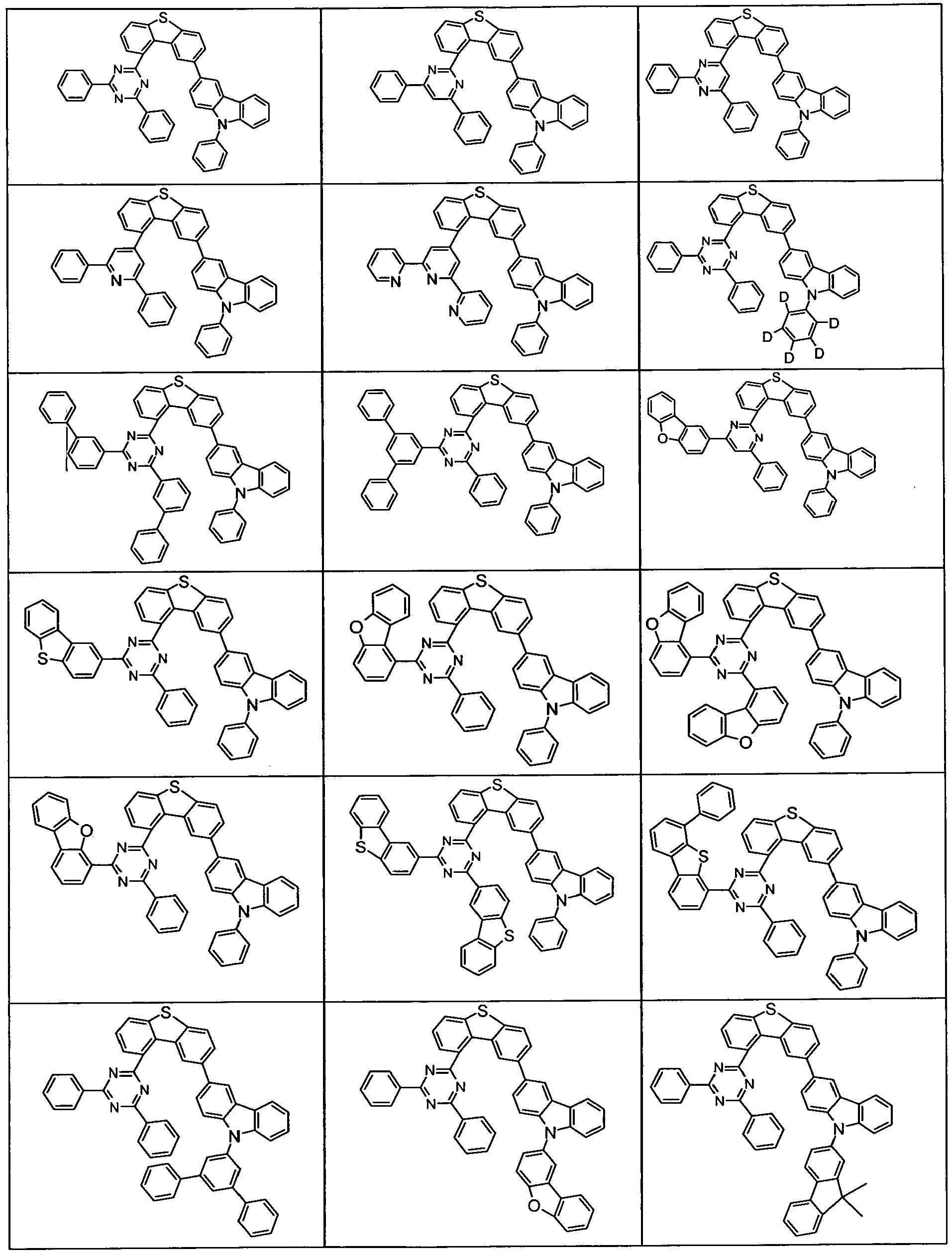

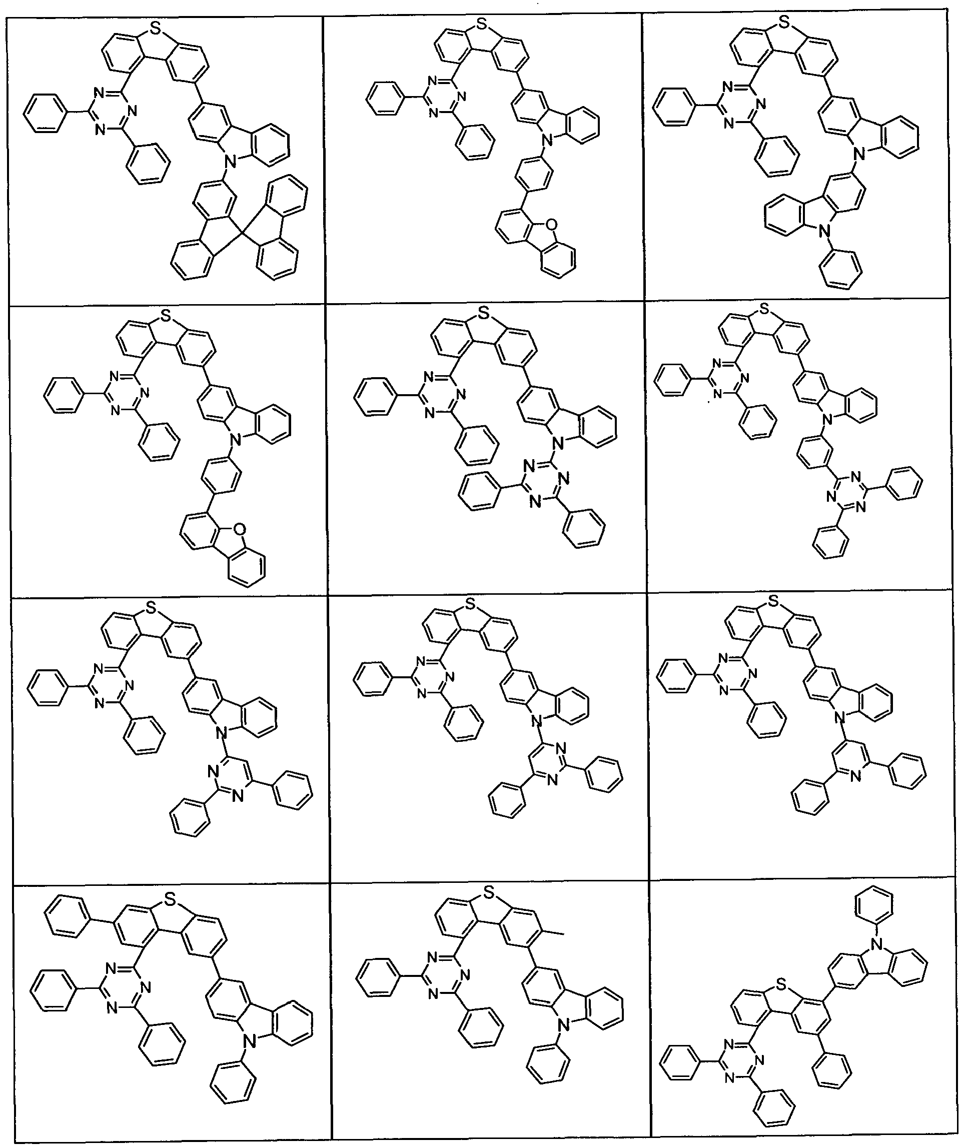

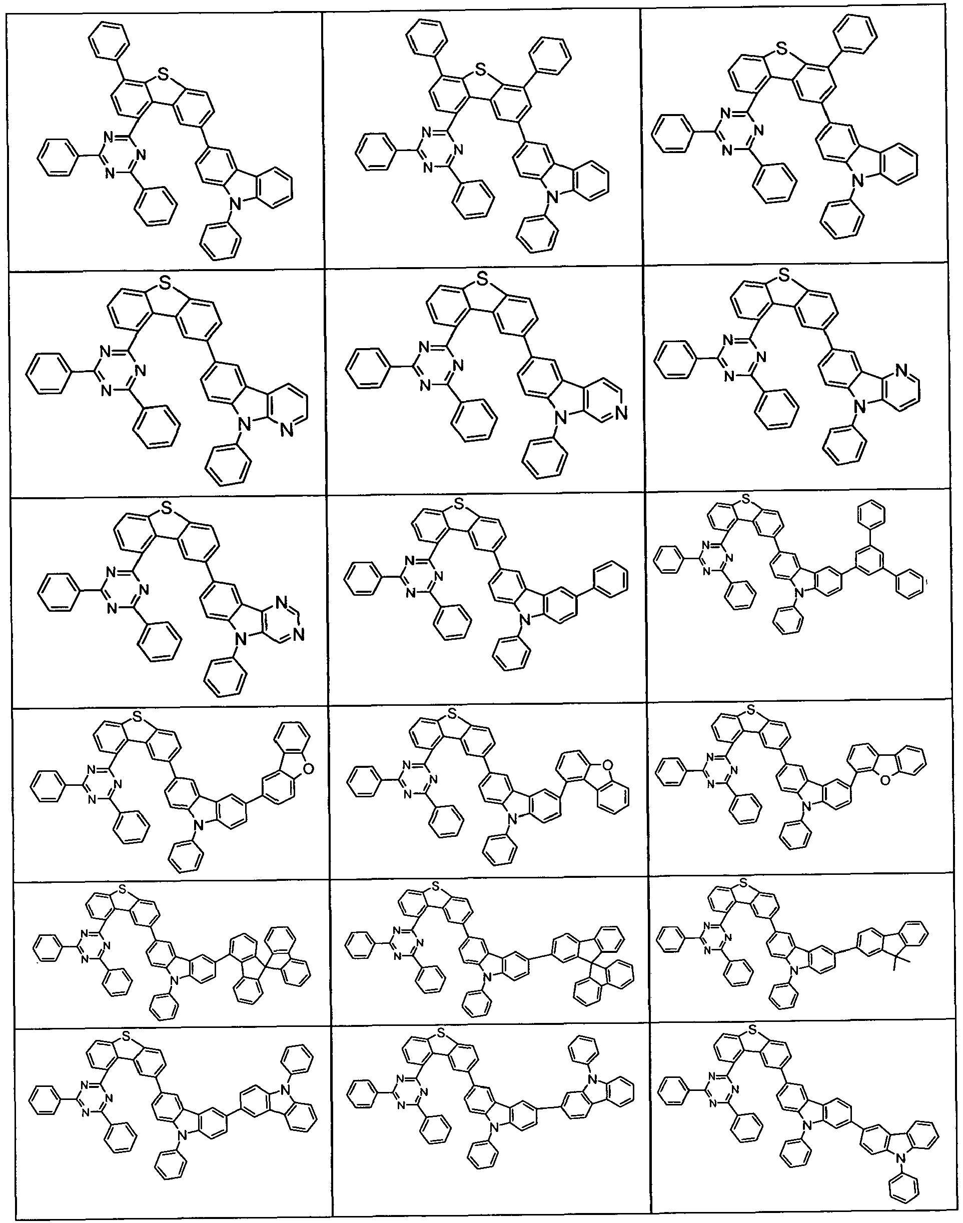

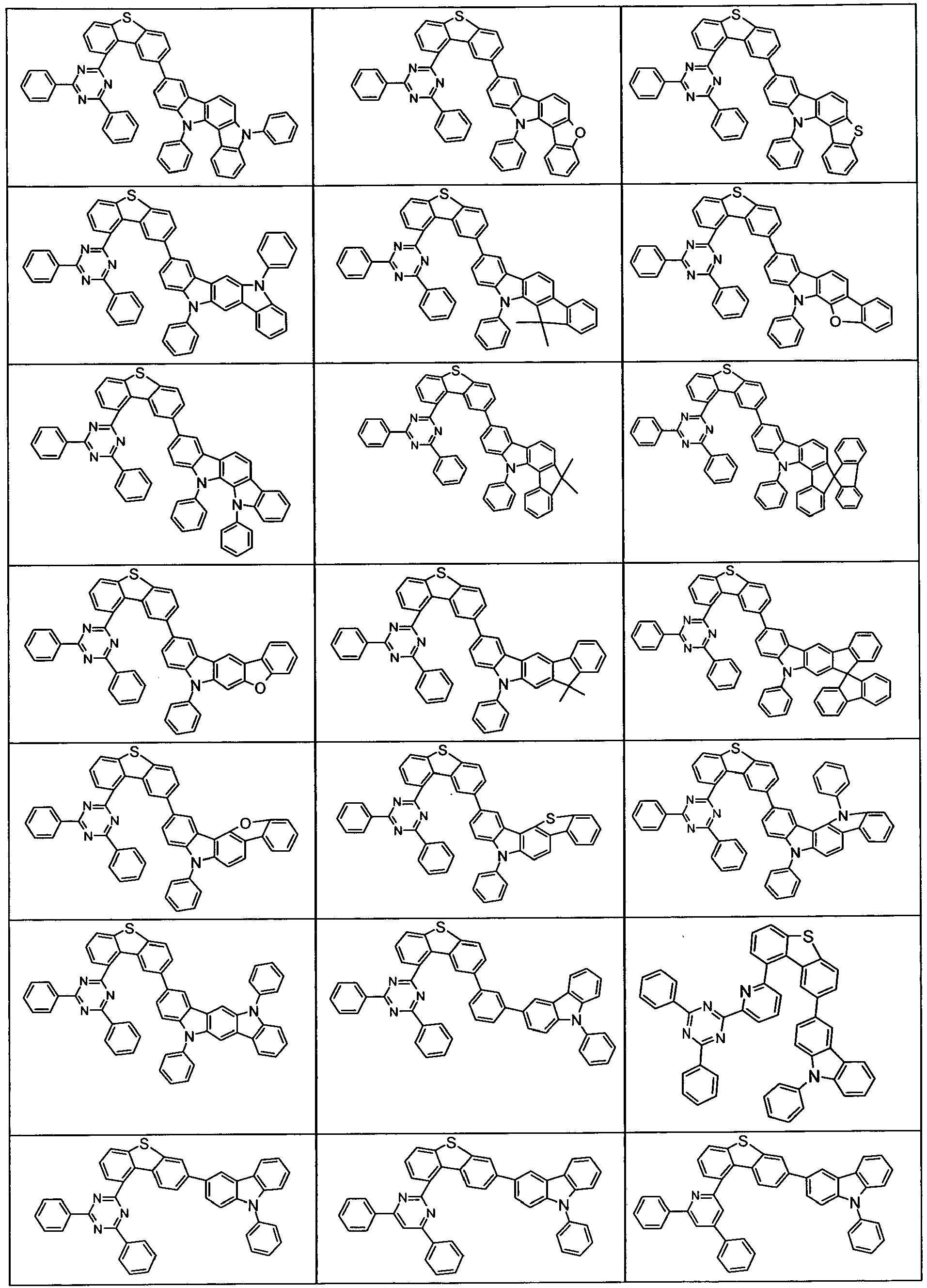

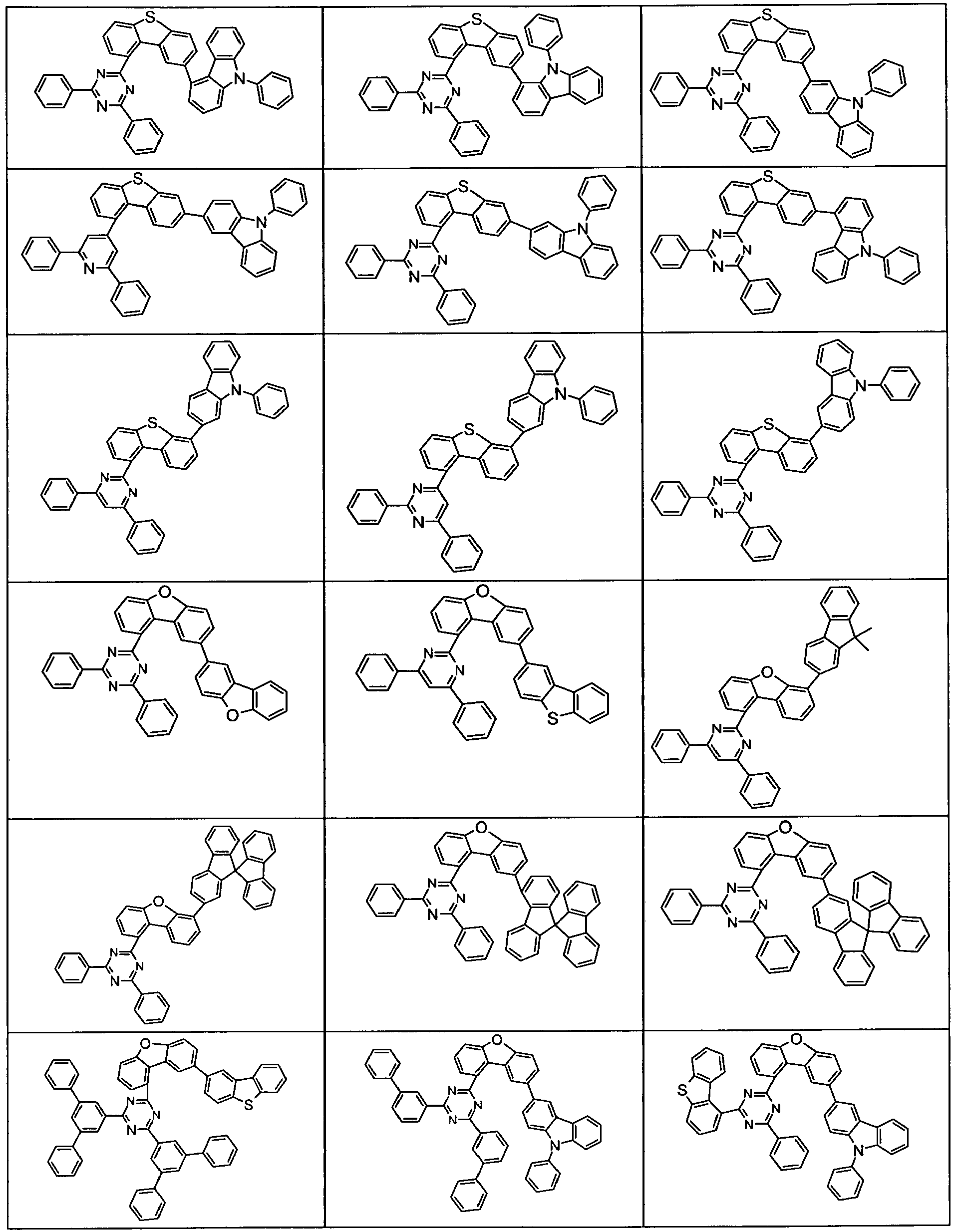

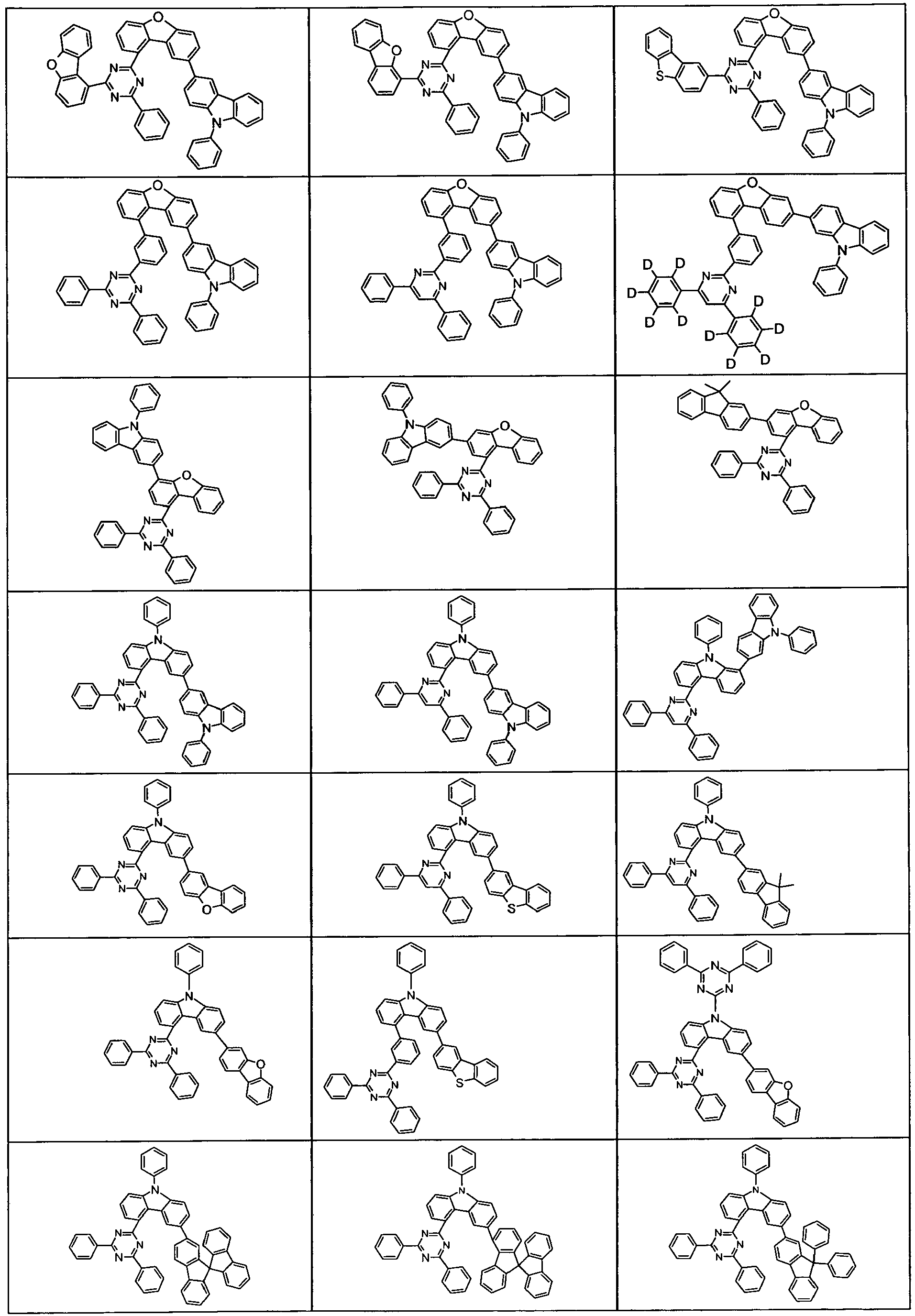

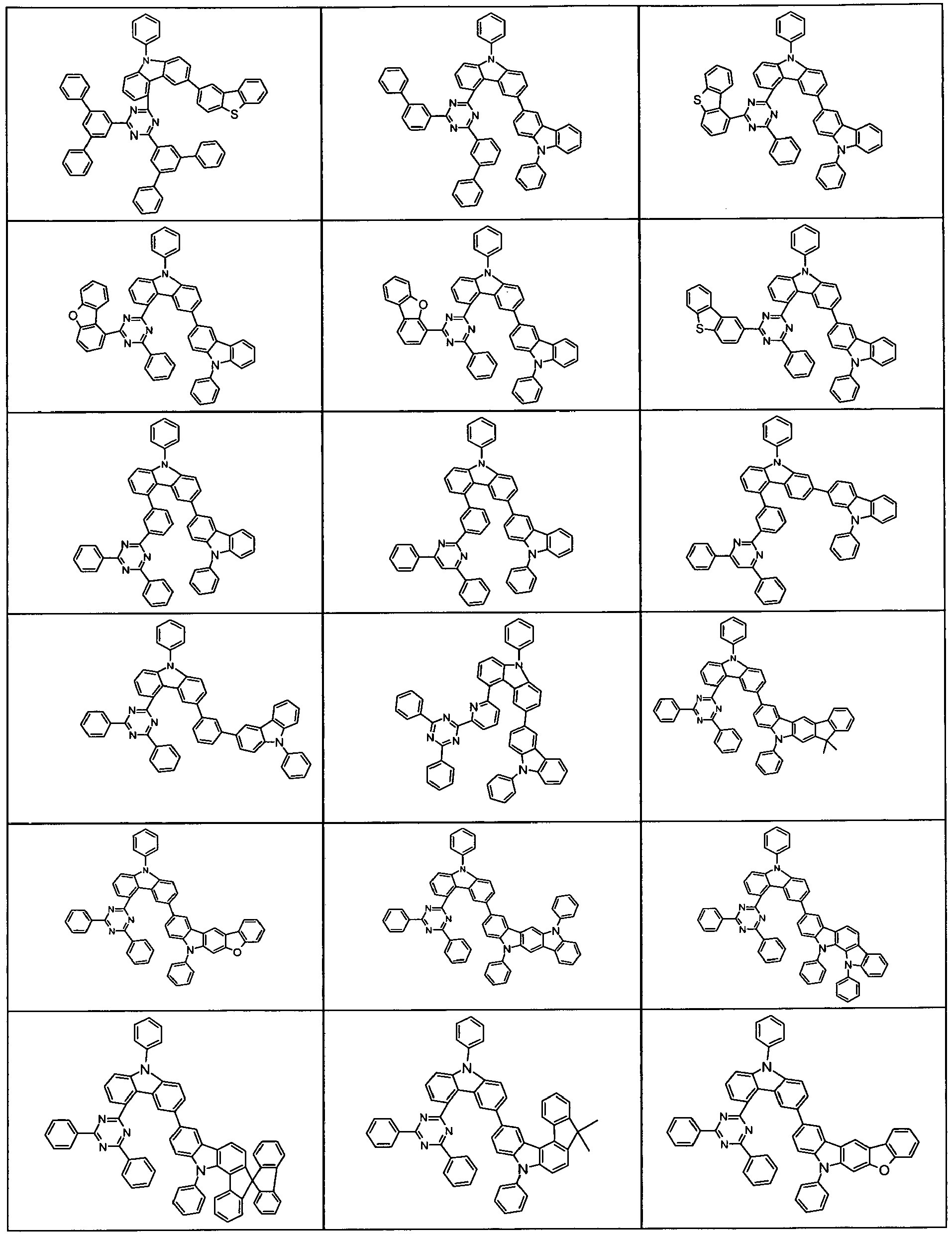

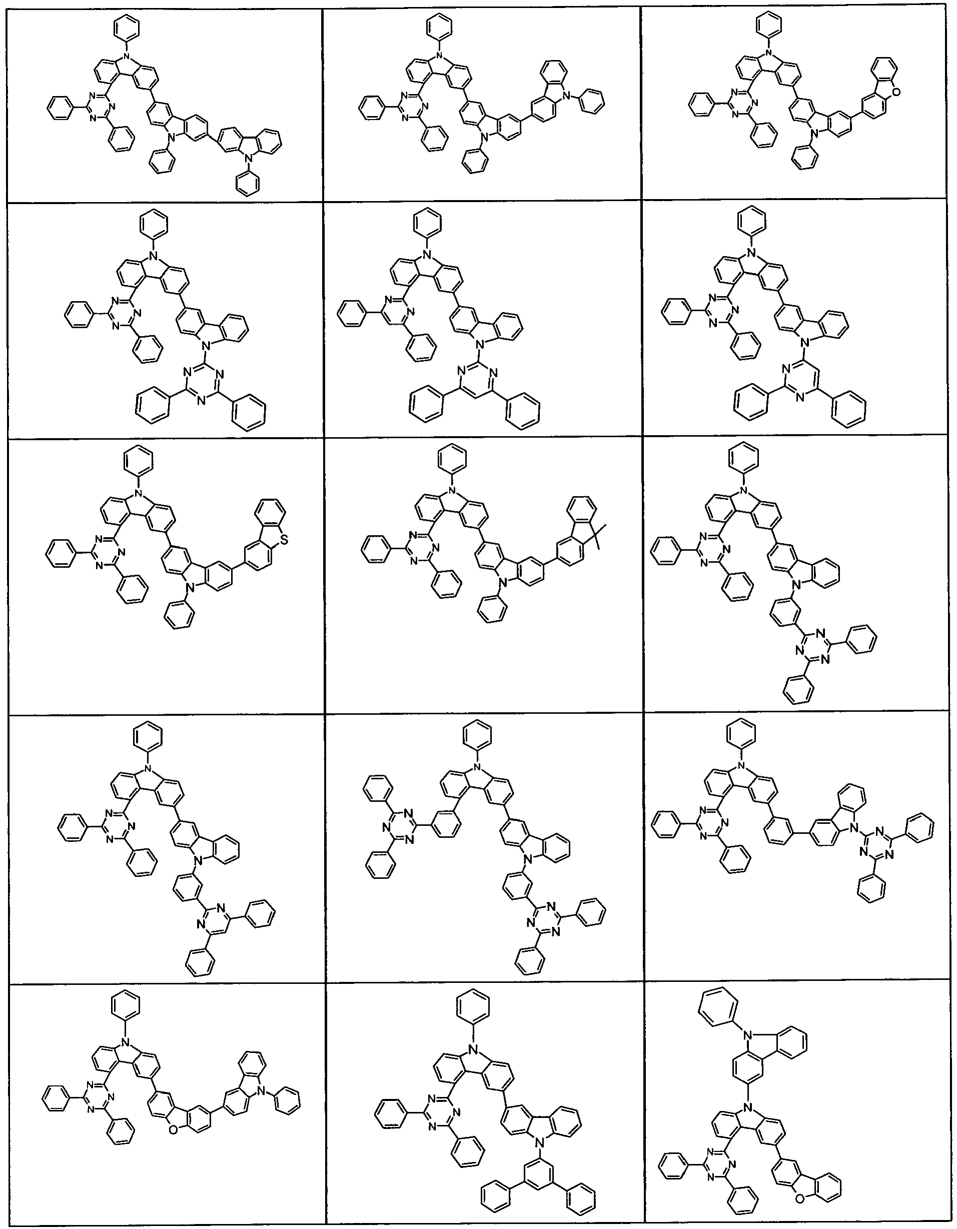

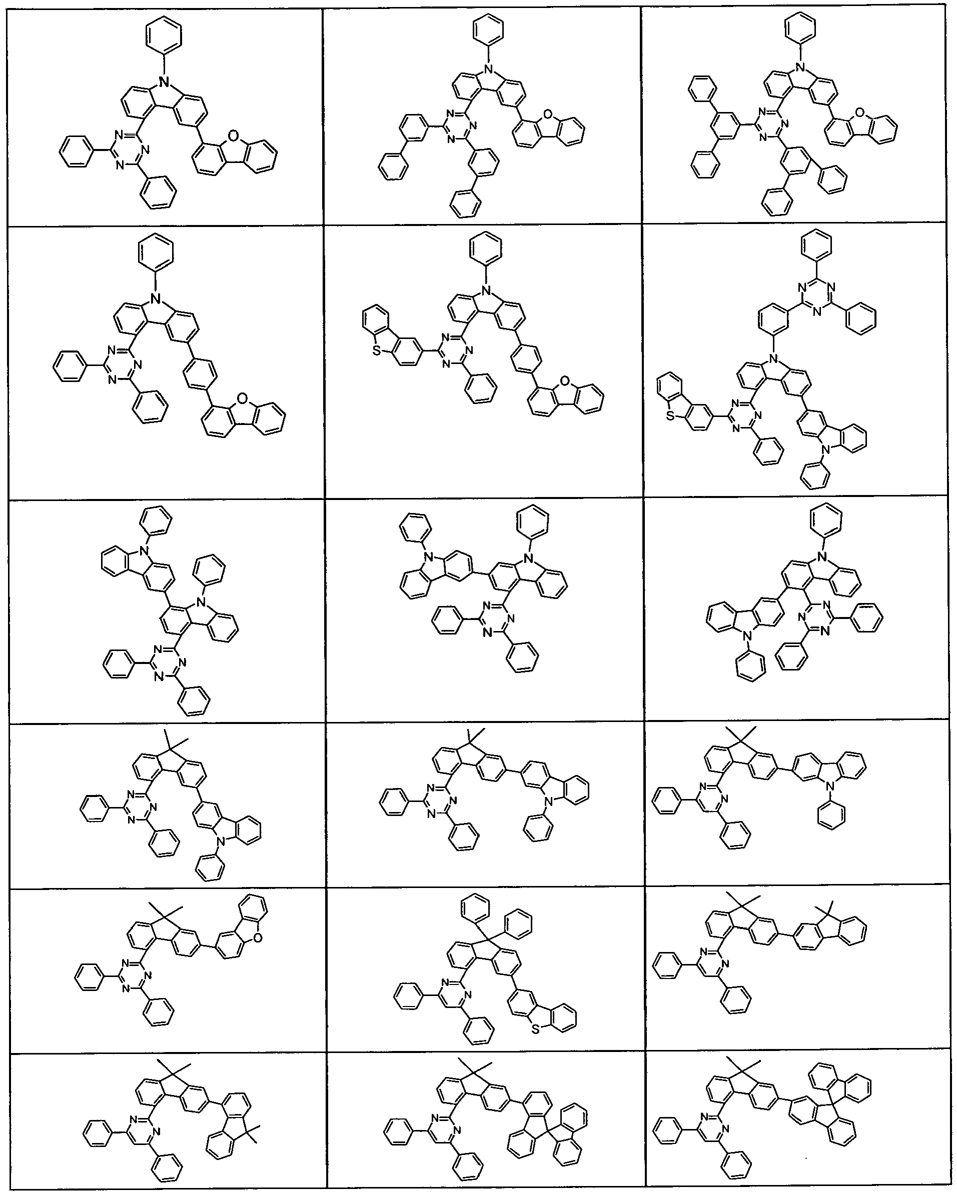

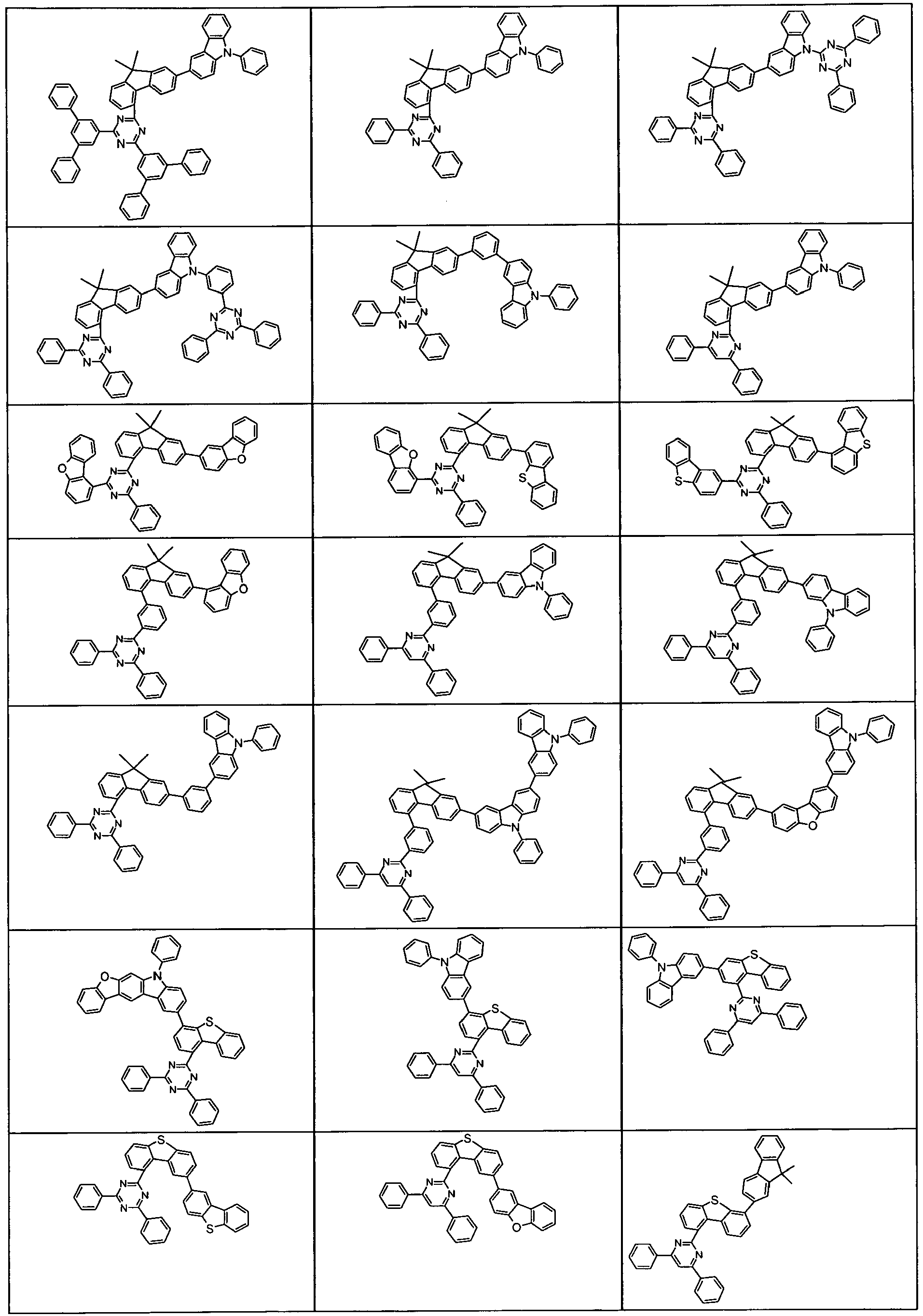

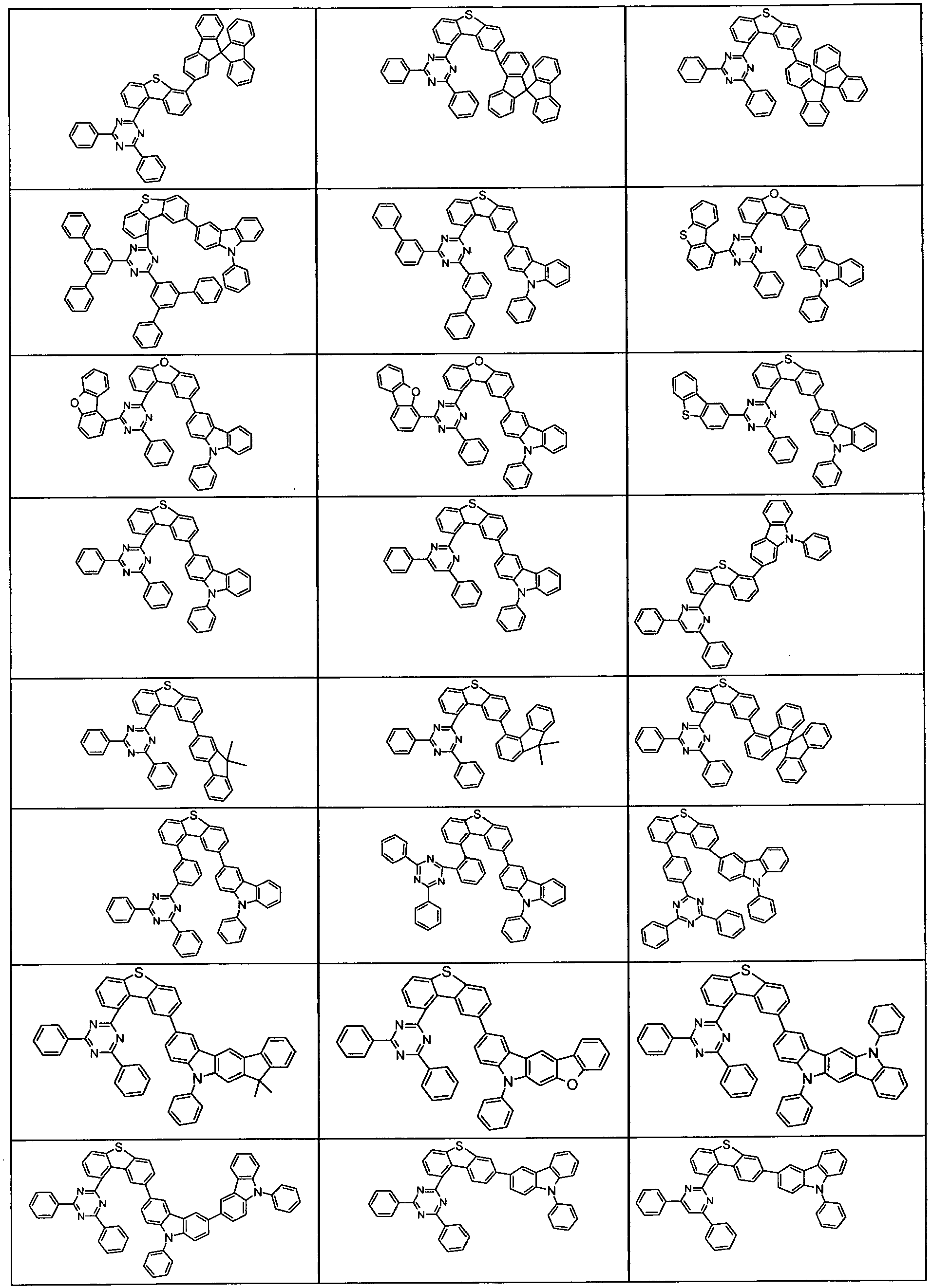

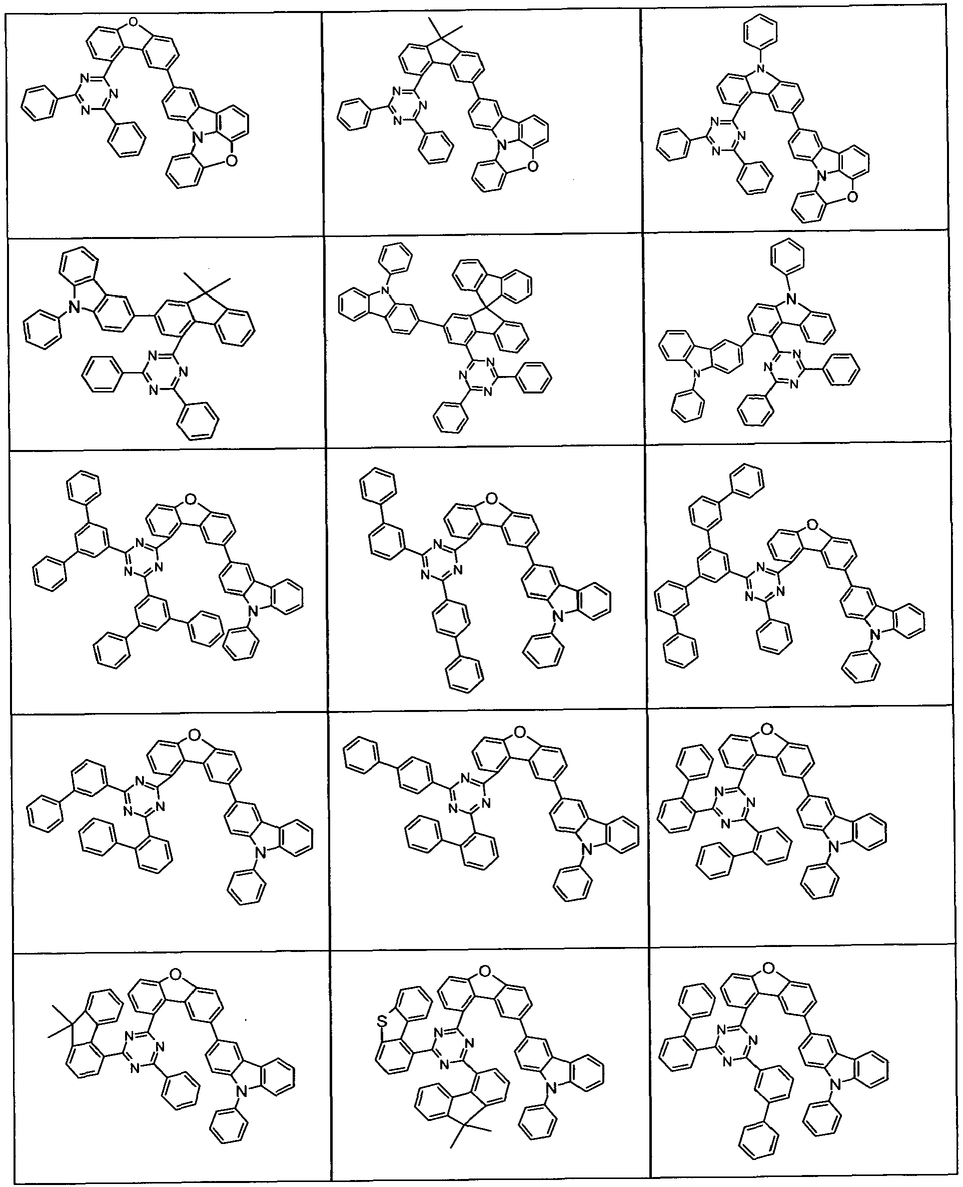

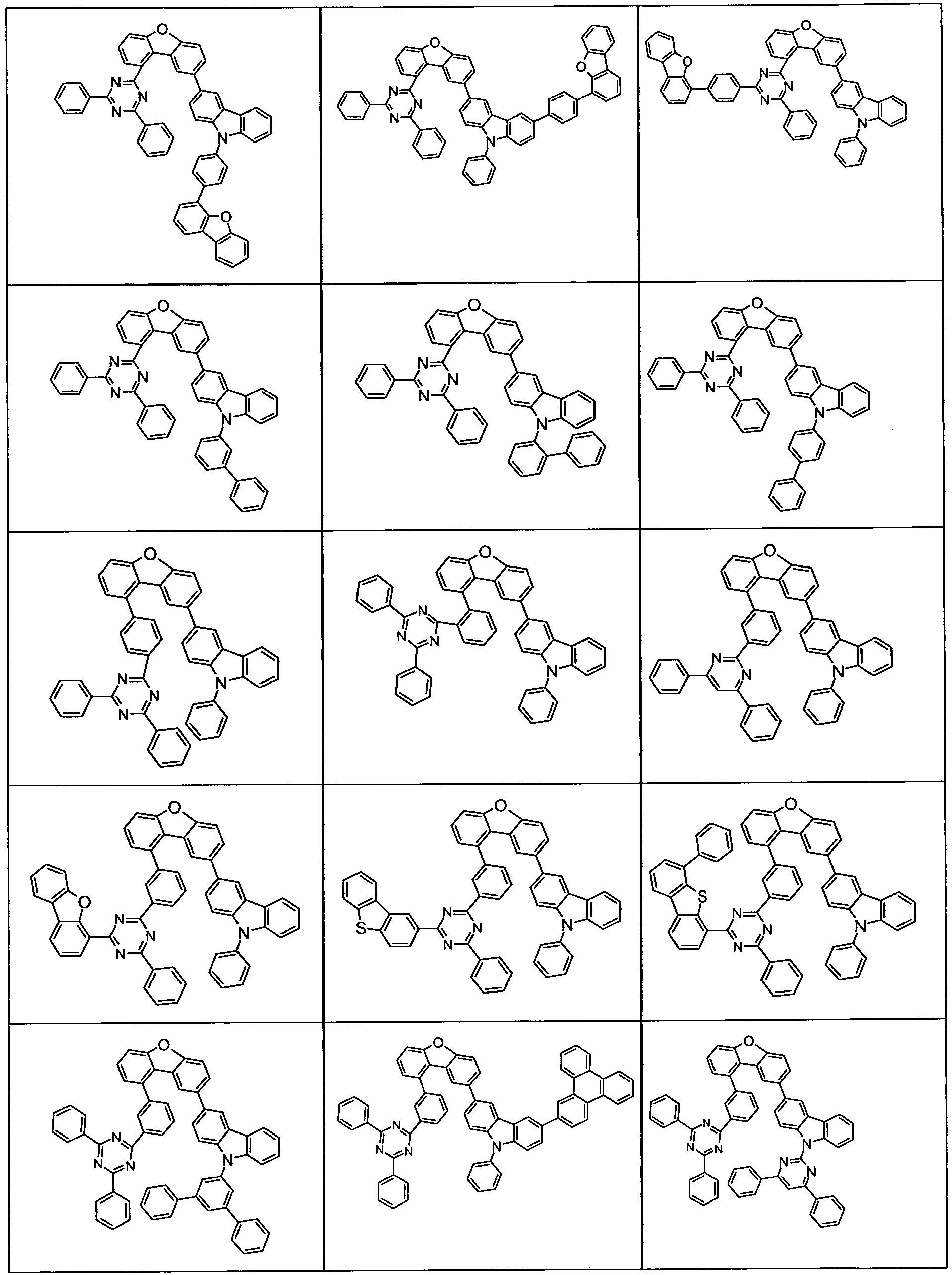

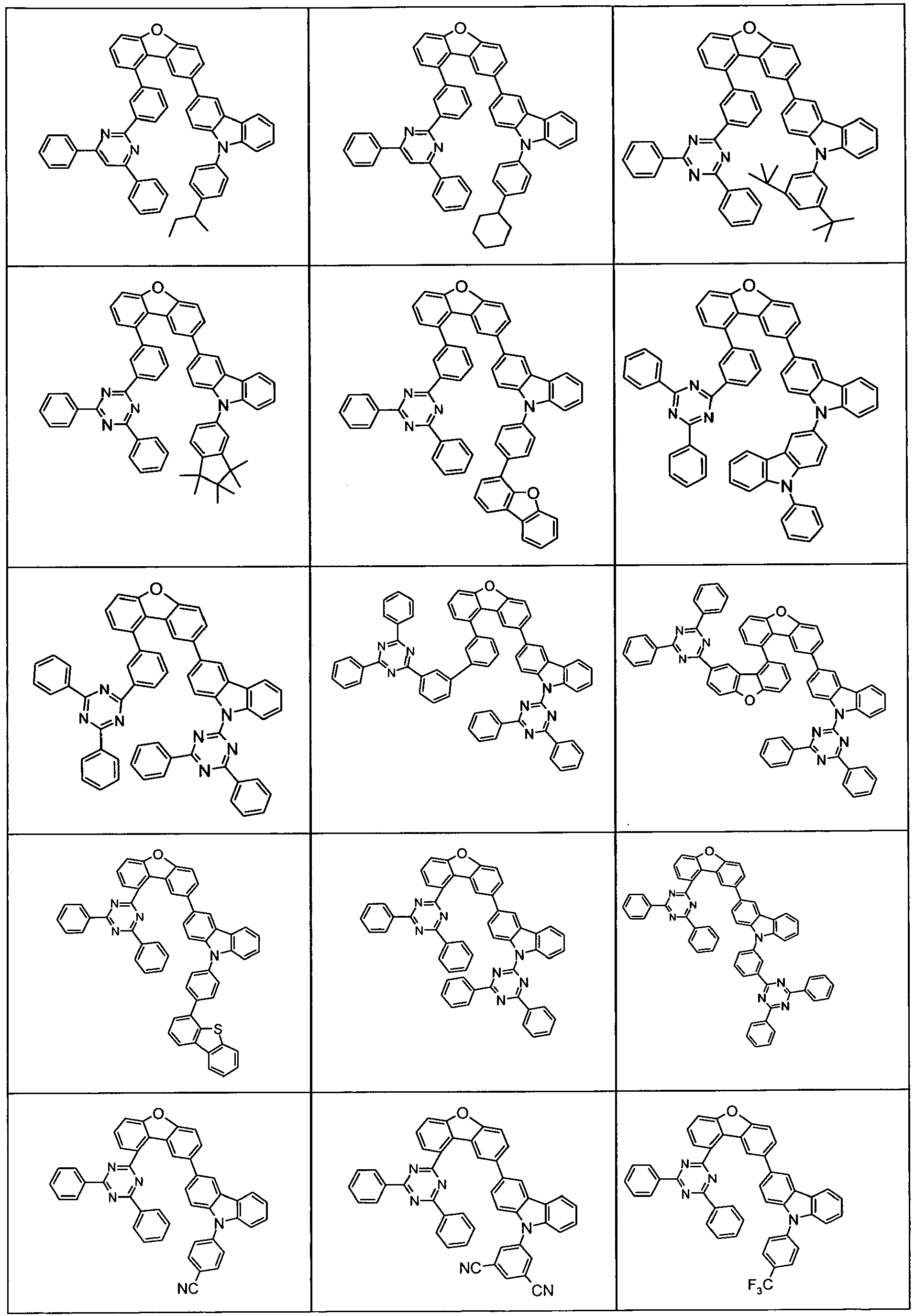

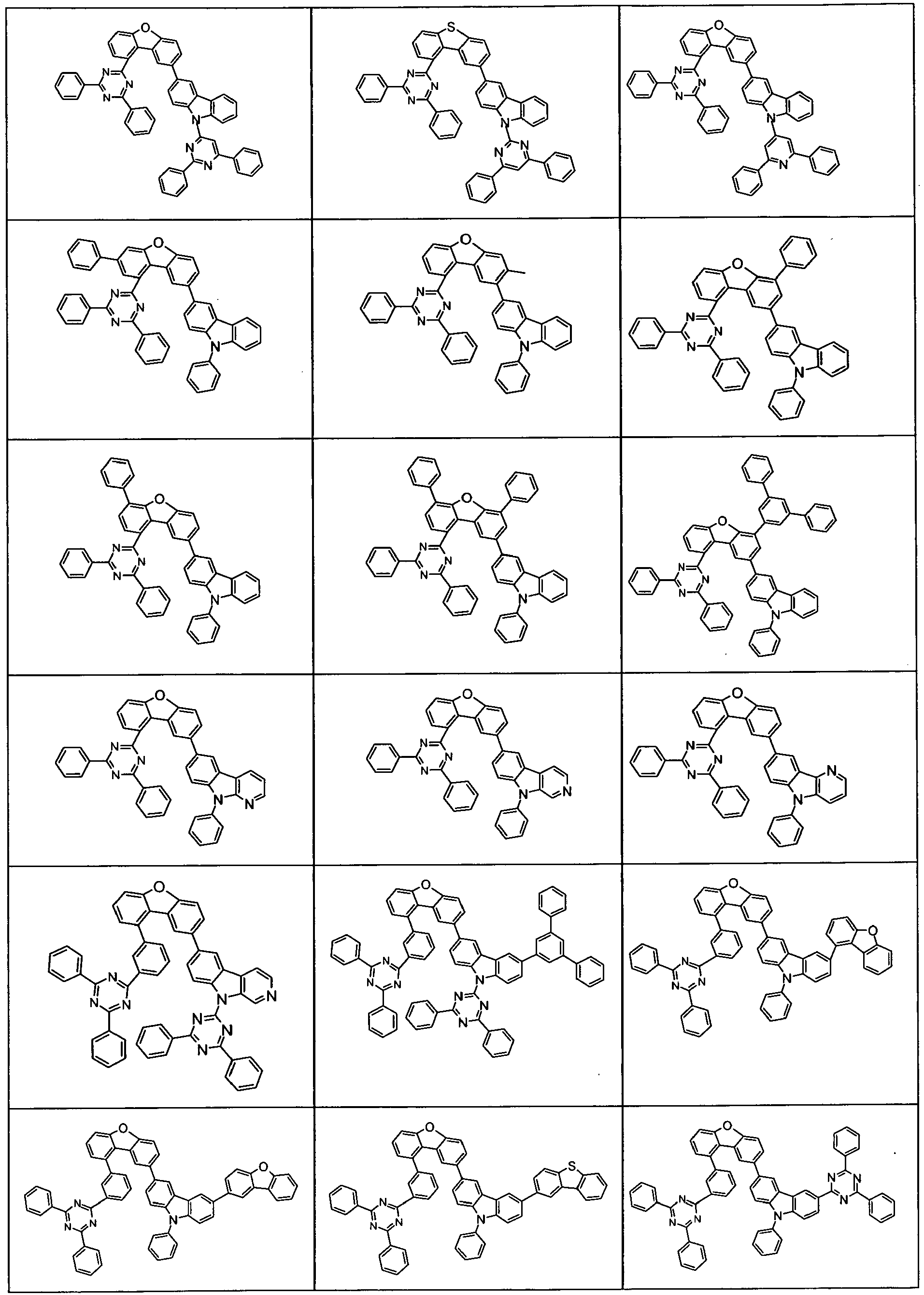

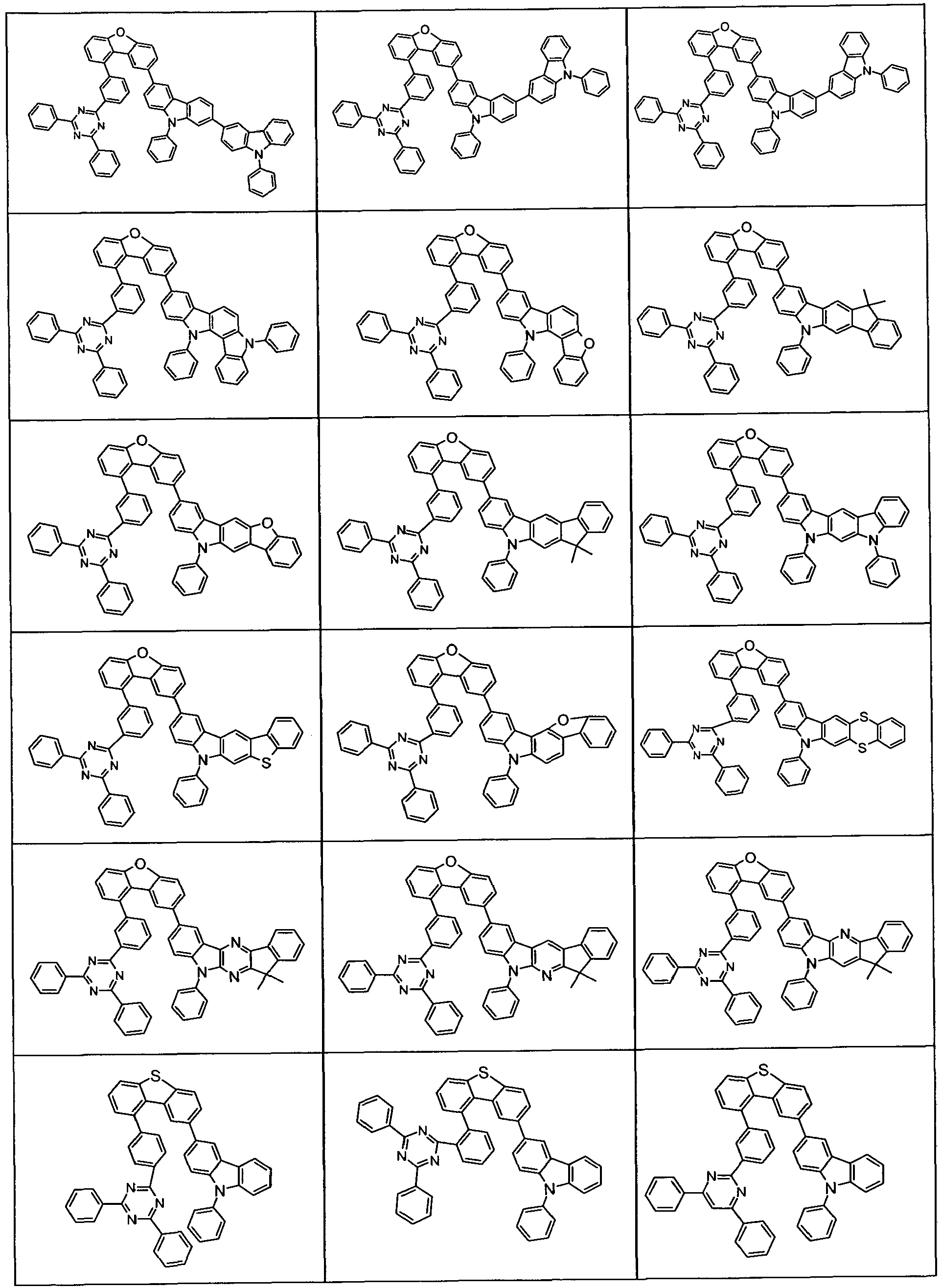

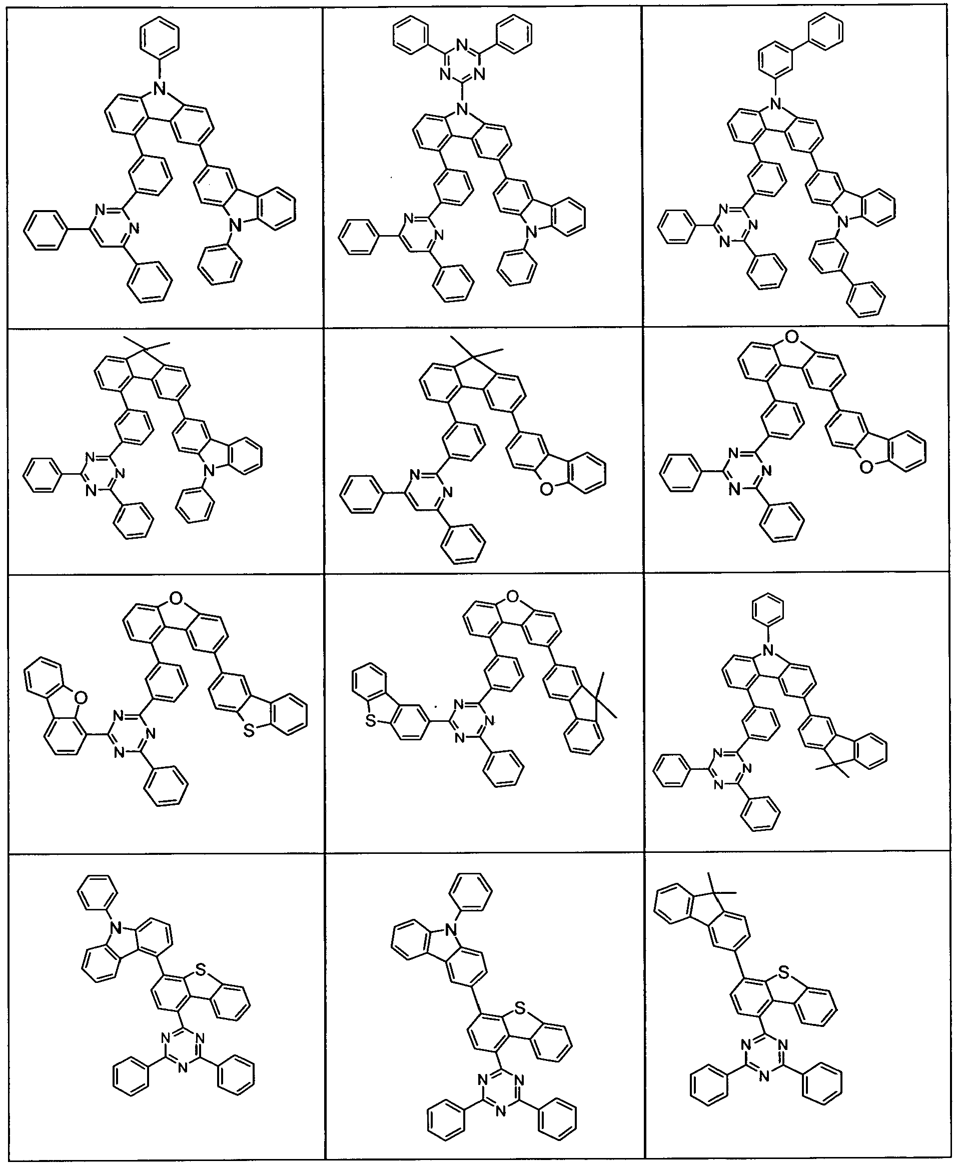

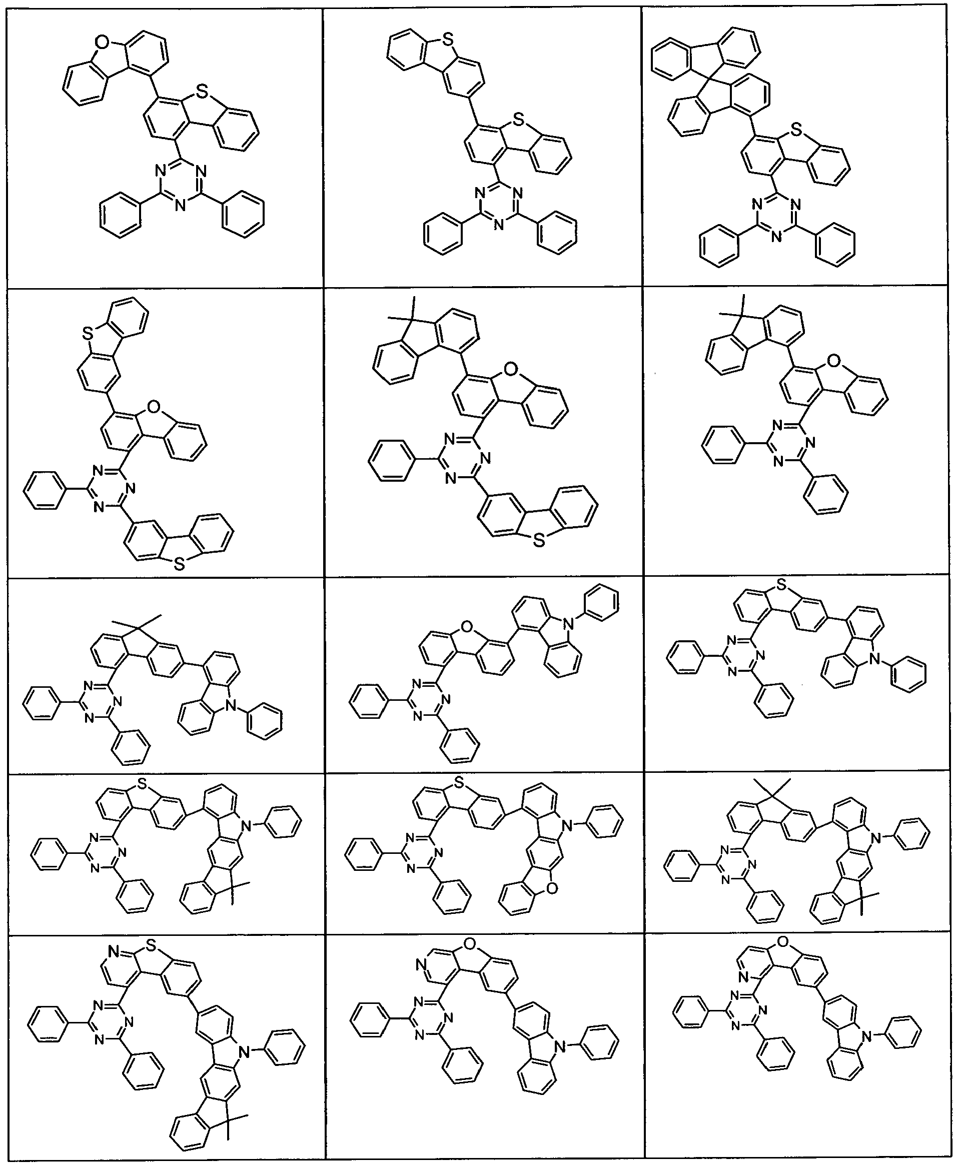

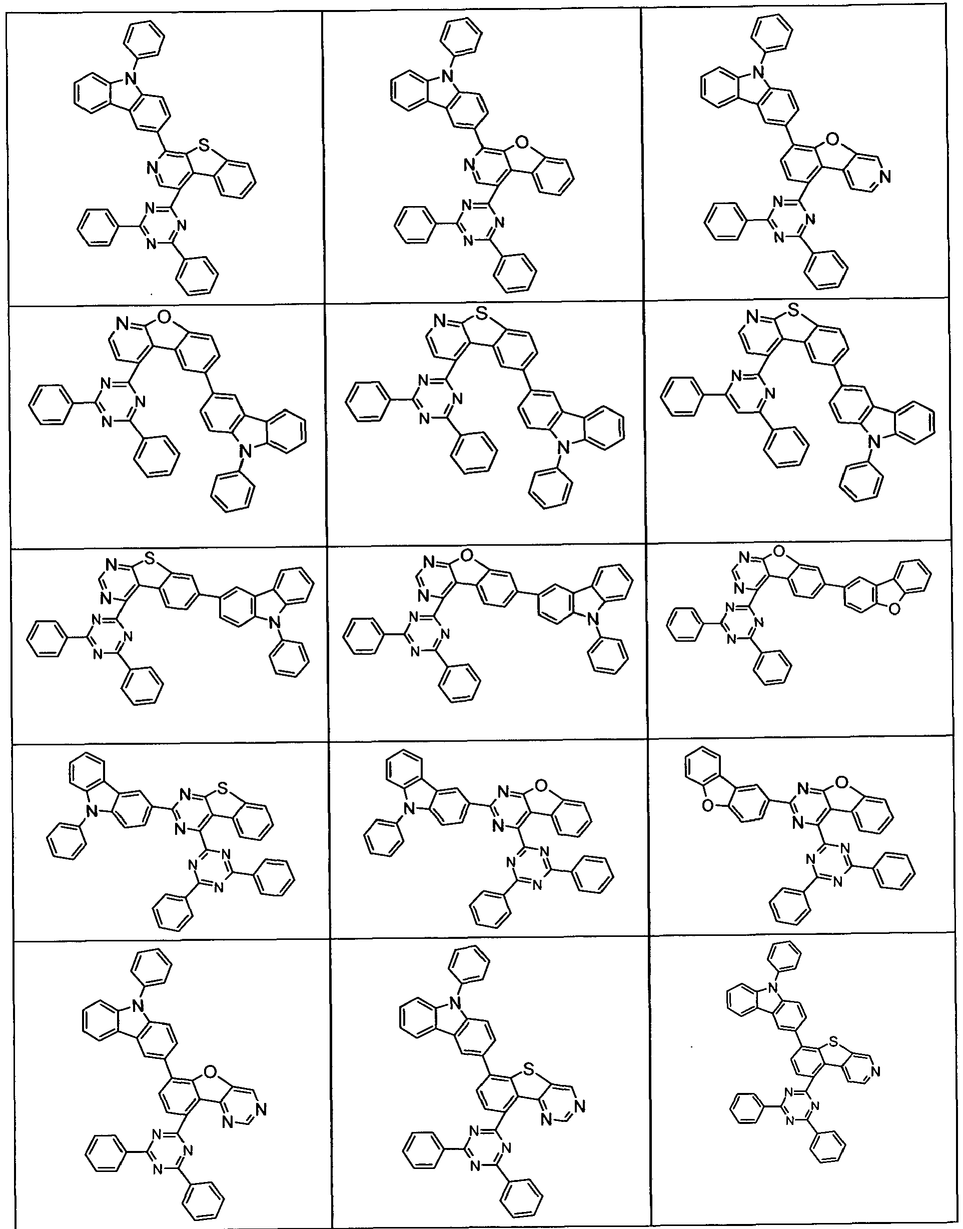

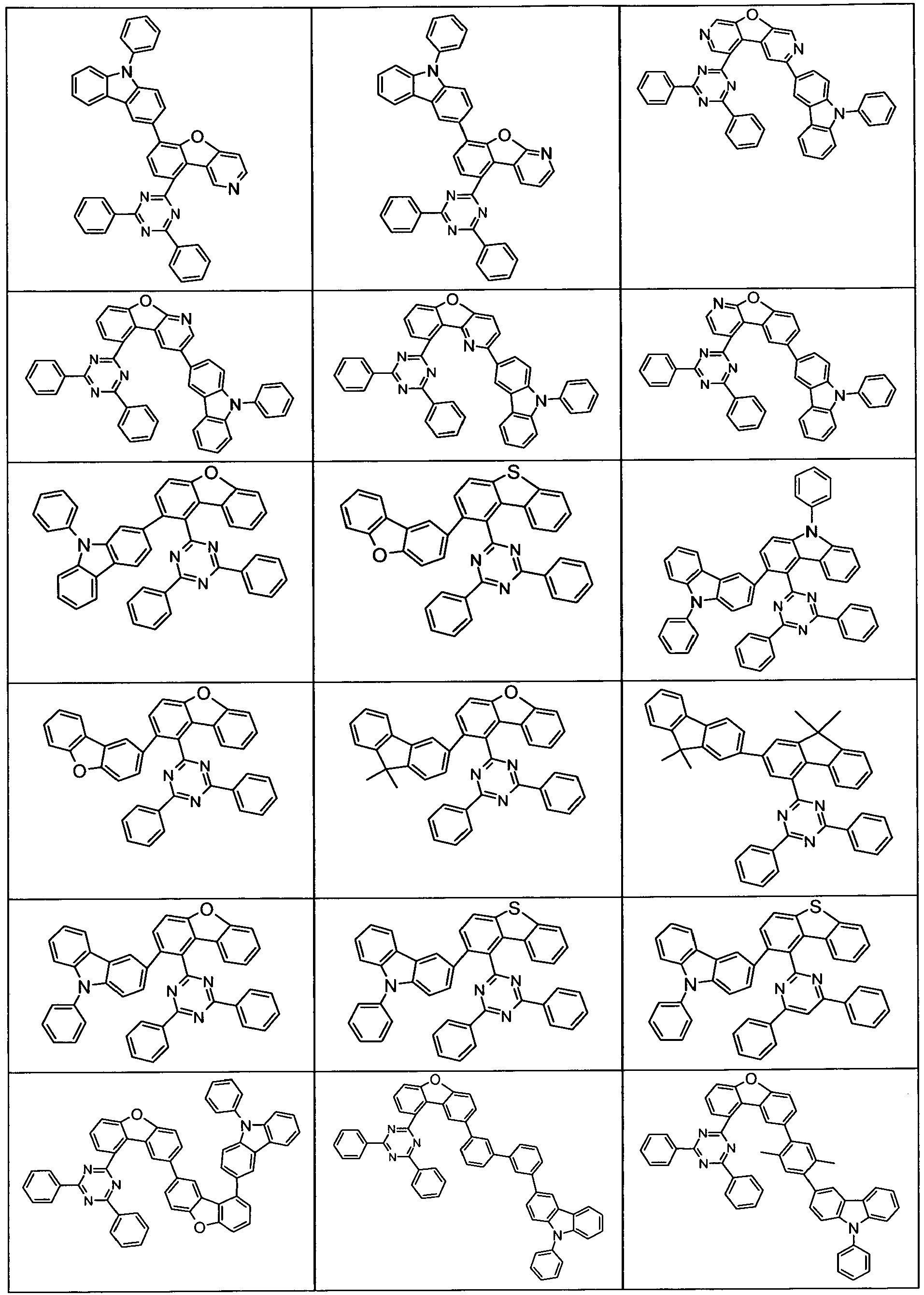

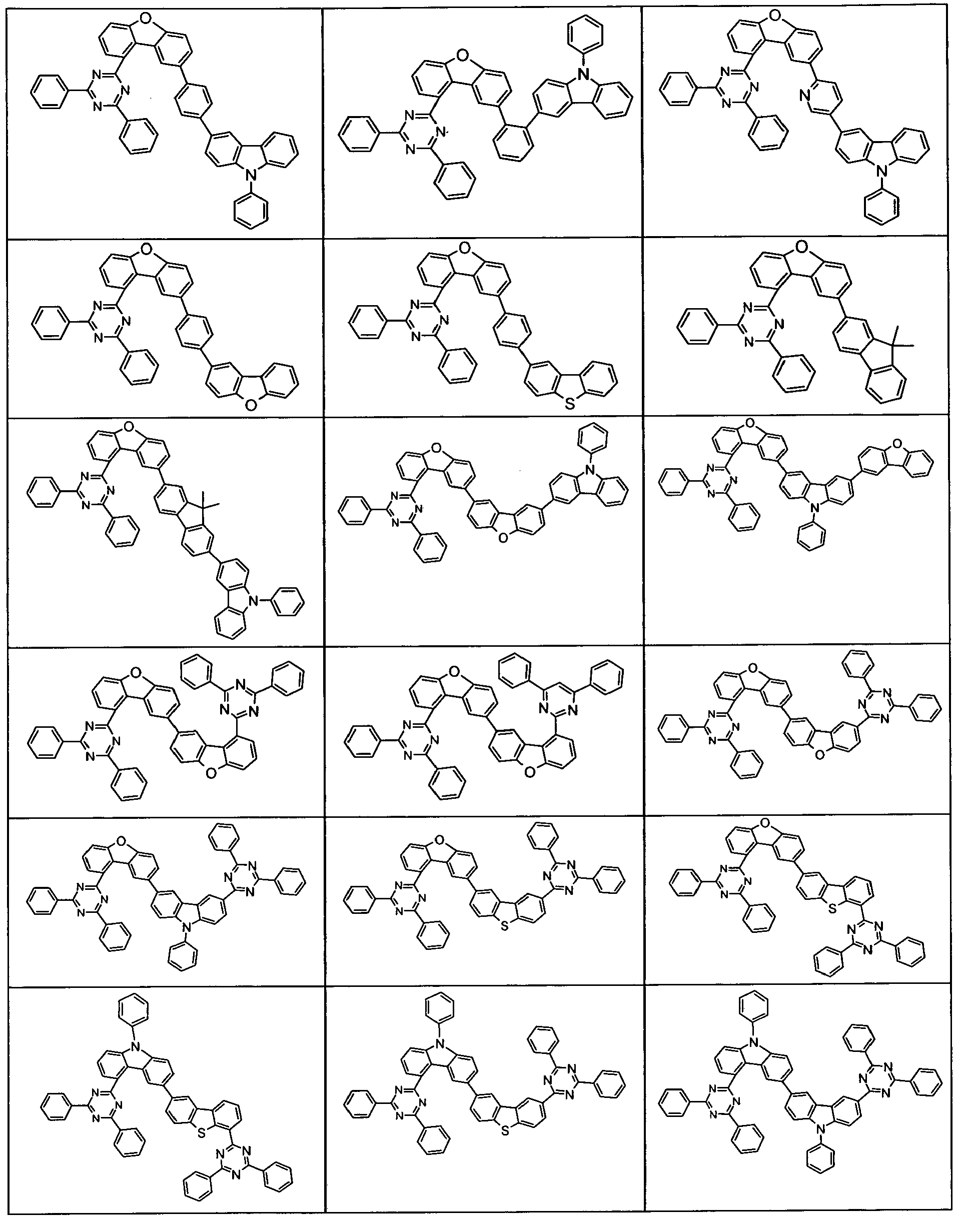

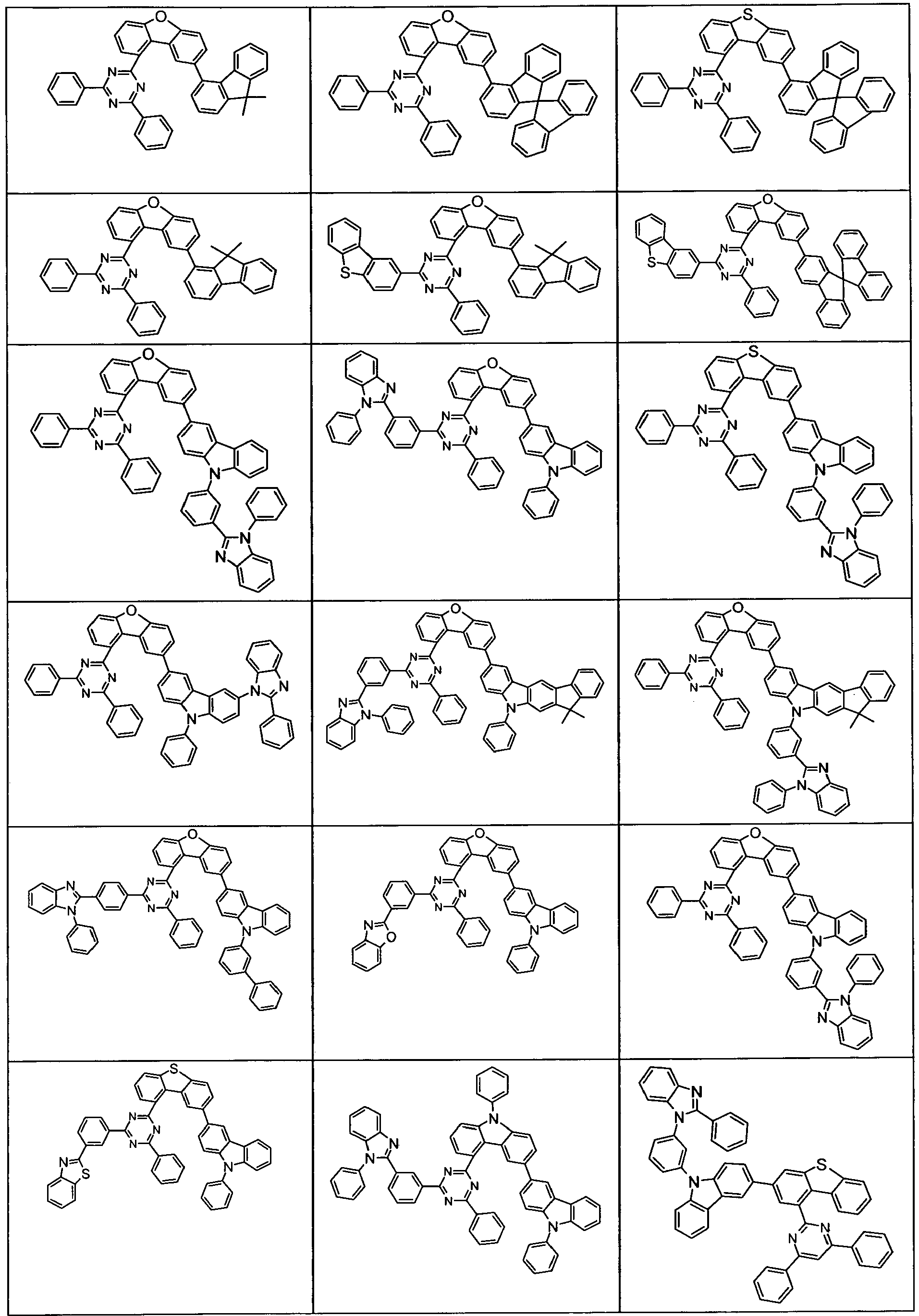

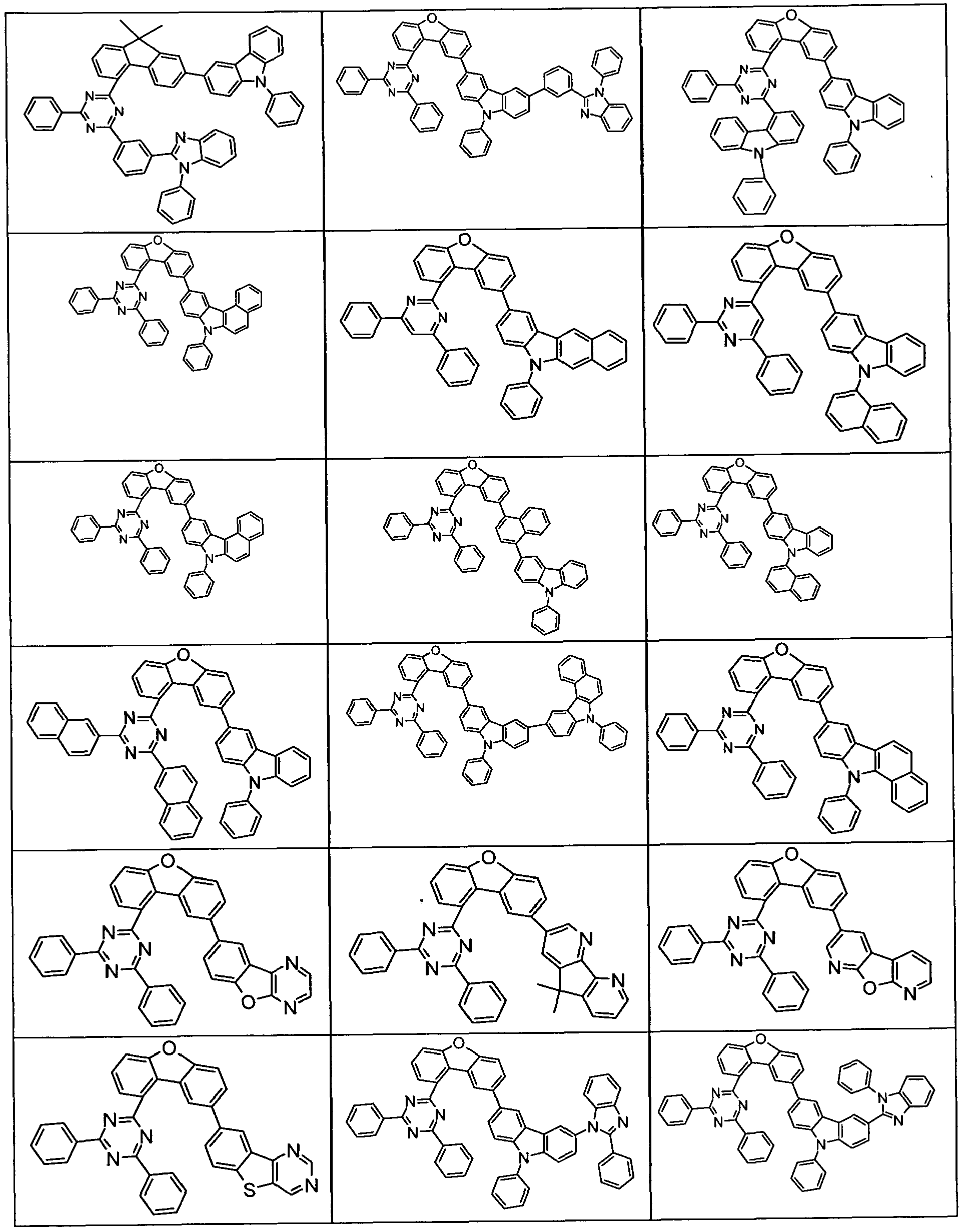

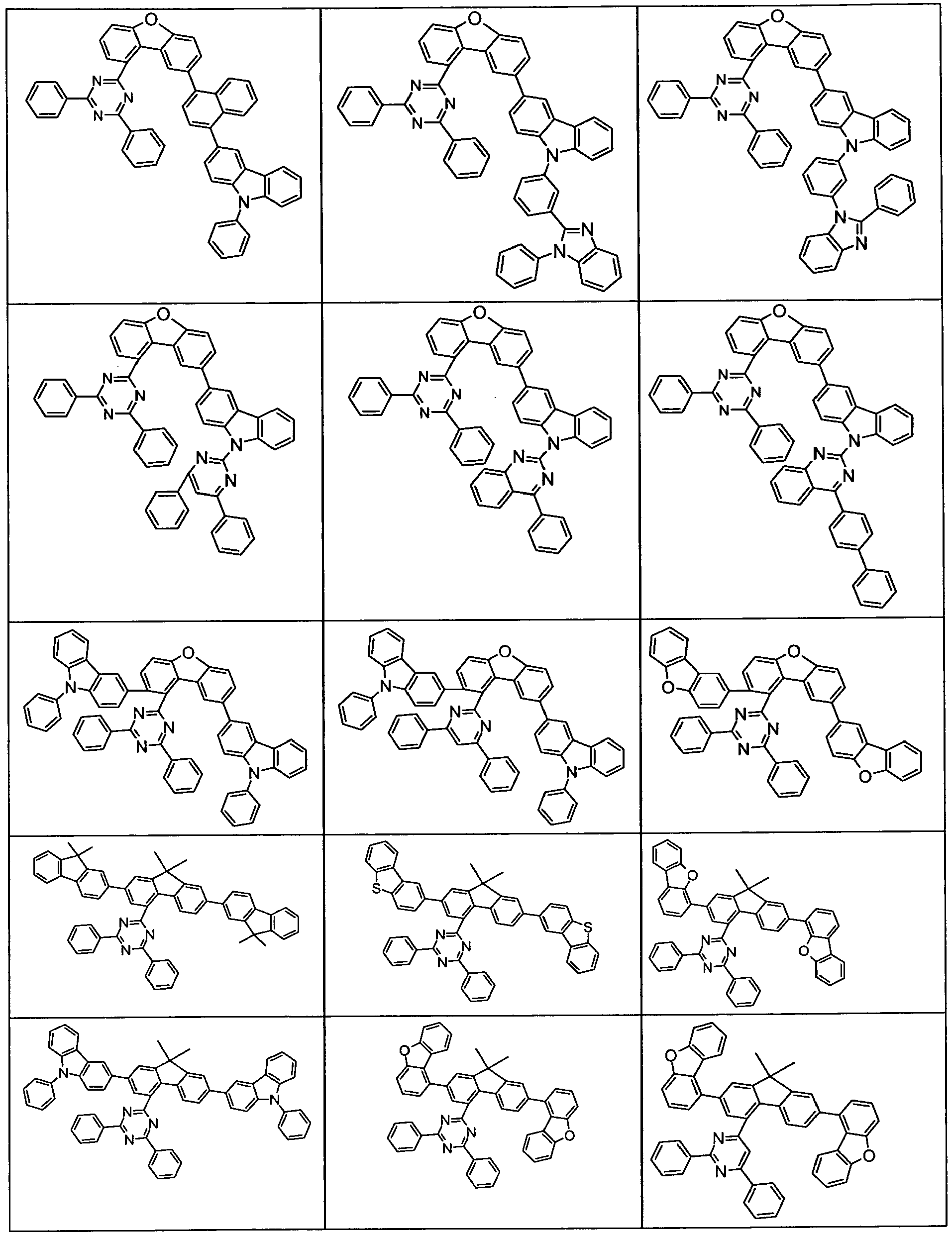

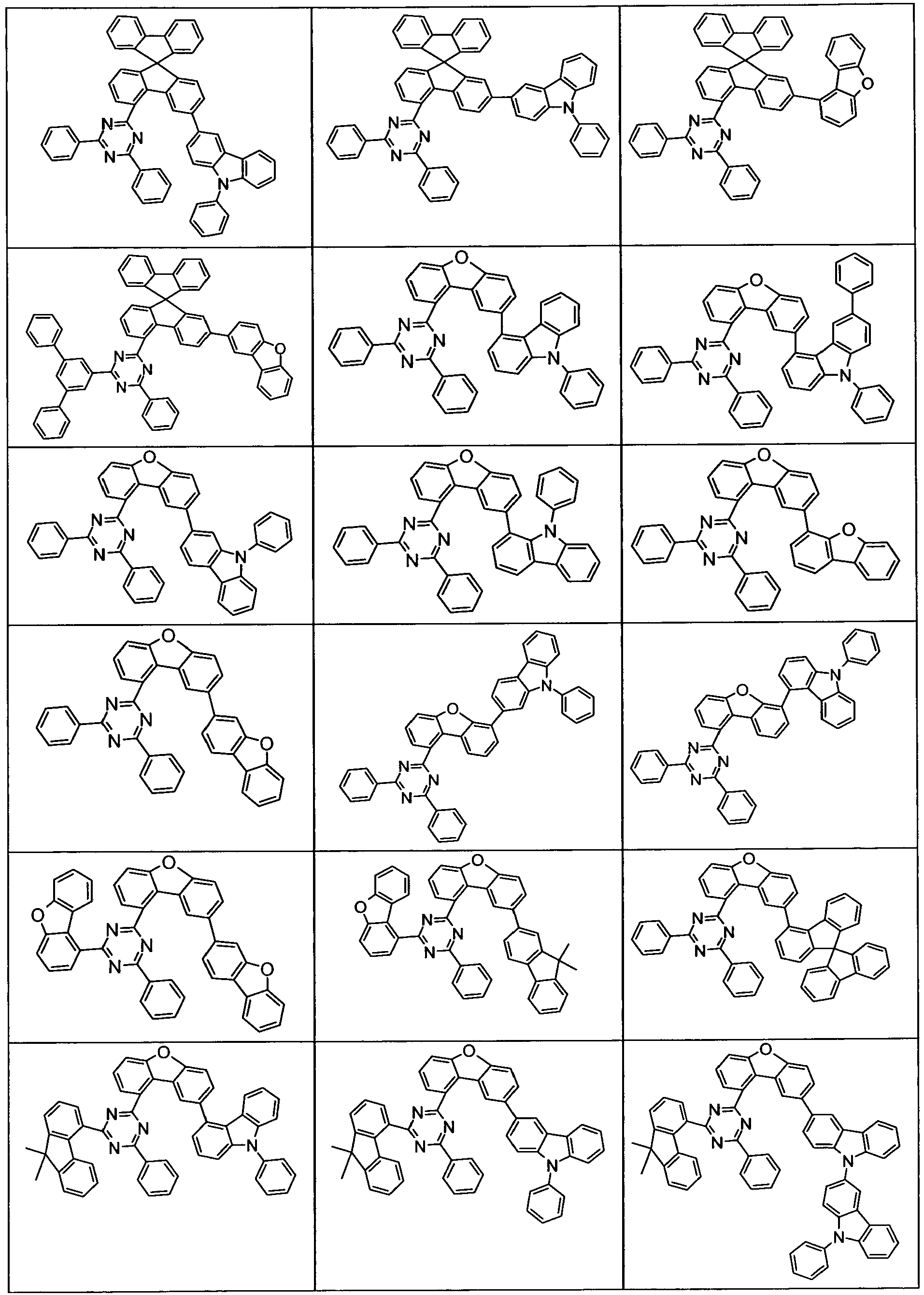

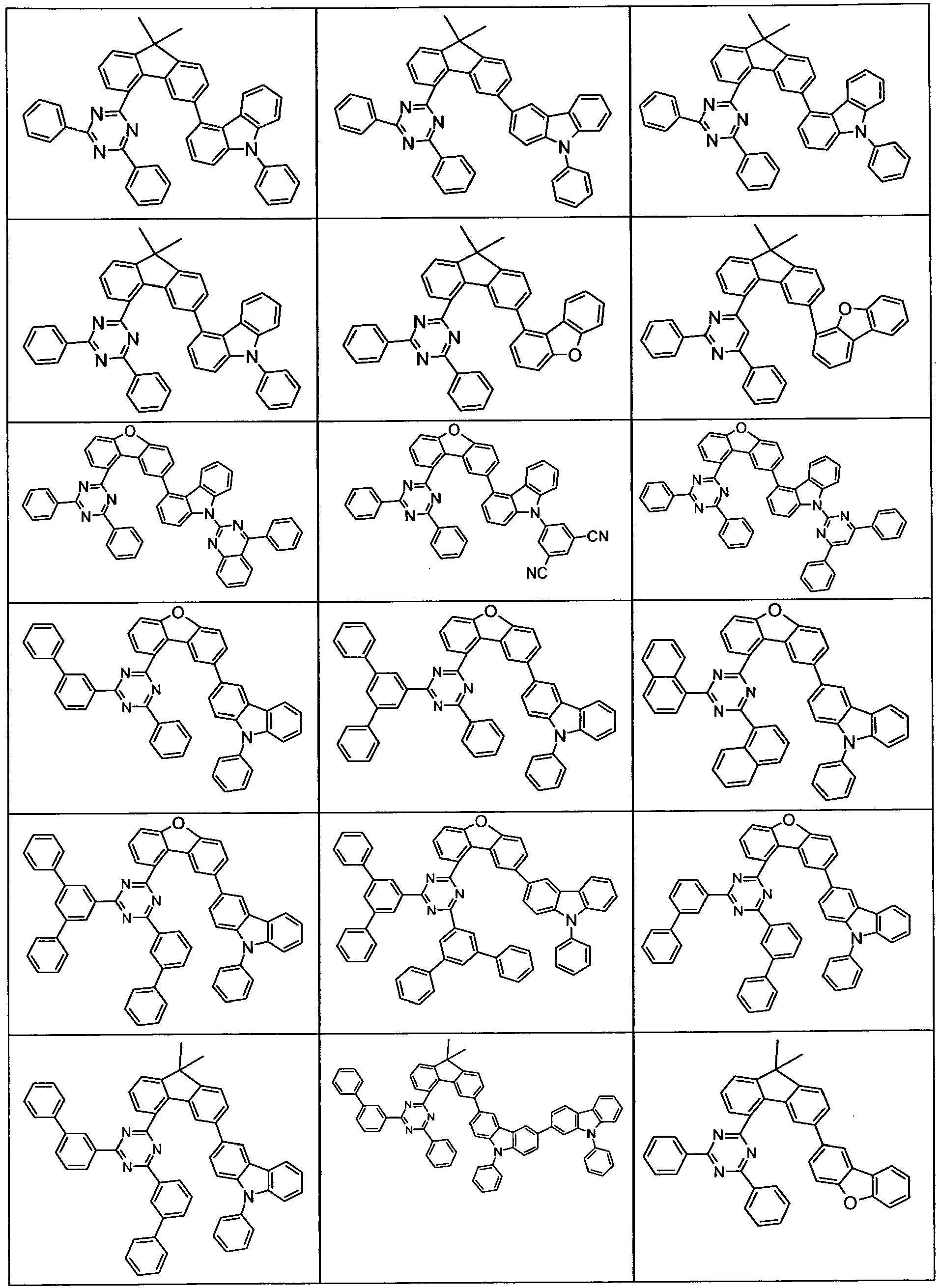

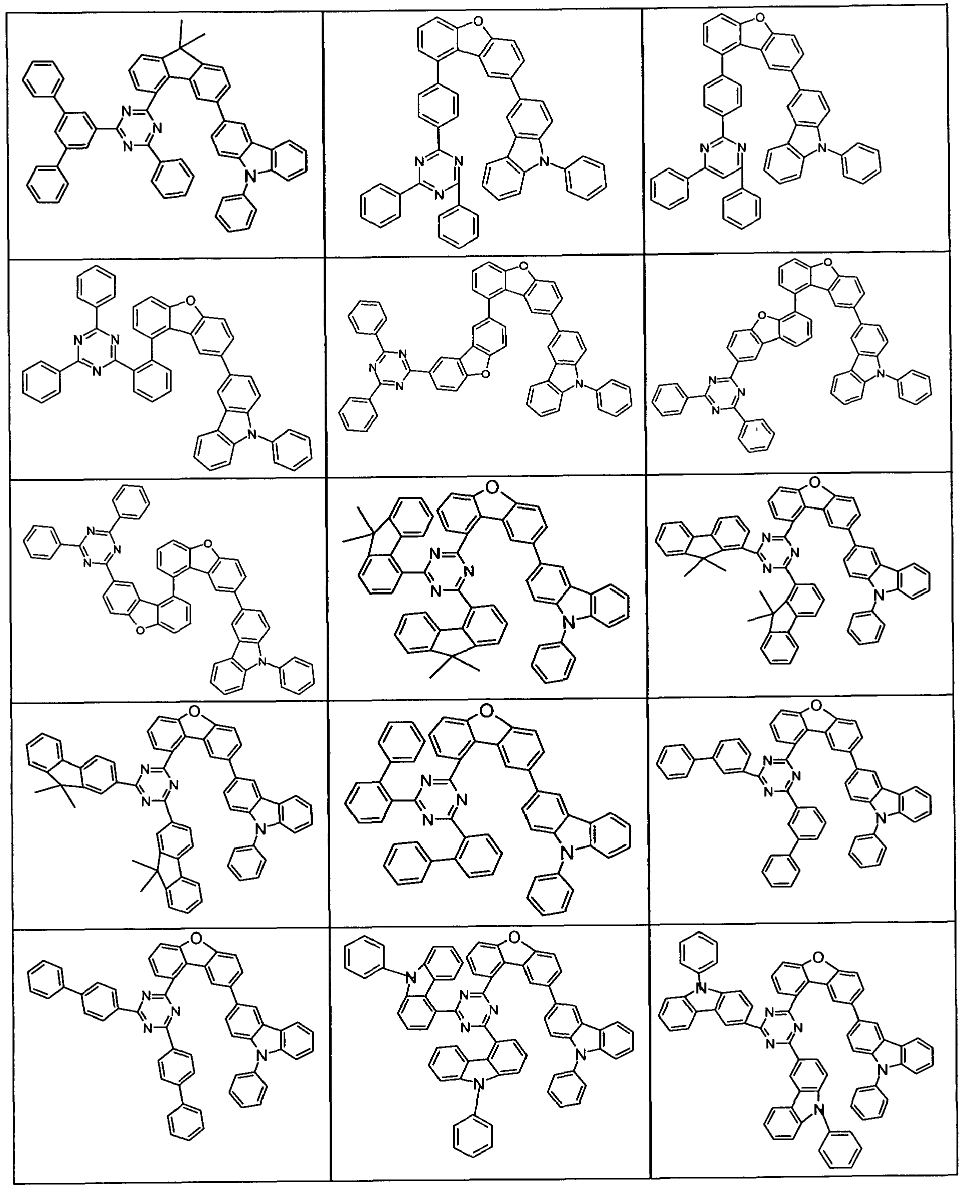

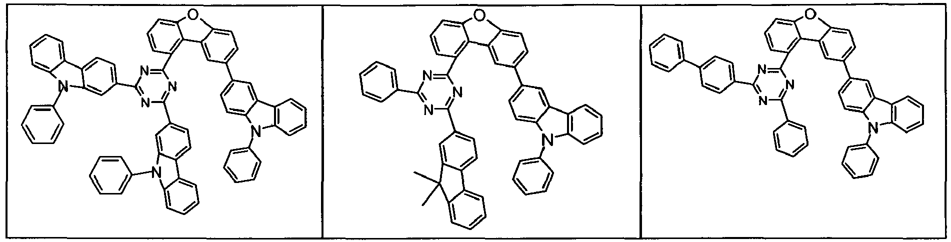

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Ein geeignetes Syntheseverfahren ist allgemein im folgenden Schema 1 dargestellt.

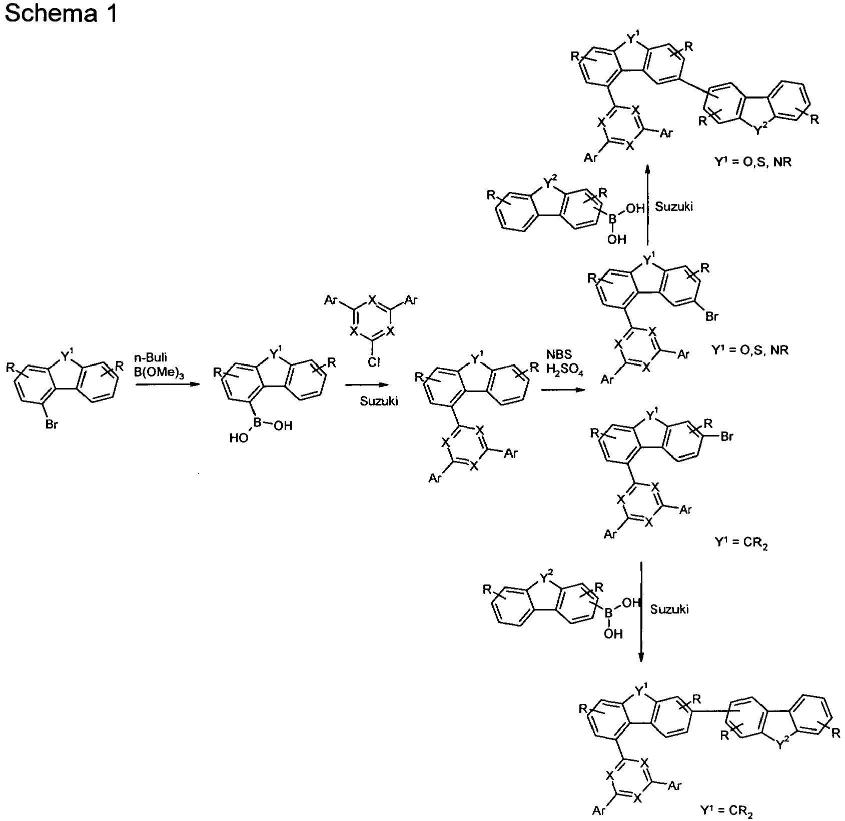

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden. Eine weiß emittierende Elektrolumineszenzvorrichtung kann beispielsweise für Beleuchtungsanwendungen, aber auch in Kombination mit einem Farbfilter für Vollfarb-Displays verwendet werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. Formel (2) oder gemäß den bevorzugten Ausführungsformen als Matrixmaterial für fluoreszierende oder phosphoreszierende Emitter, insbesondere für phosphoreszierende Emitter, und/oder in einer Elektronentransportschicht und/oder in einer elektronenblockierenden bzw. exzitonenblockierenden Schicht und/oder in einer Lochtransportschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. Formel (2) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. Formel (2) bzw. gemäß den bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der Verbindung gemäß Formel (1) bzw. Formel (2) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial. Werden die Verbindungen aus Lösung verarbeitet, so werden statt der oben angegebenen Mengen in Vol.-% bevorzugt die entsprechenden Mengen in Gew.-% verwendet.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960 und den noch nicht offen gelegten Anmeldungen EP 13004411.8, EP 14000345.0, EP 14000417.7 und EP 14002623.8 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Eine weitere bevorzugte Ausführungsform der vorliegenden Erfindung ist der Einsatz der Verbindung gemäß Formel (1) bzw. Formel (2) bzw. gemäß den bevorzugten Ausführungsformen als Matrixmaterial für einen phosphoreszierenden Emitter in Kombination mit einem weiteren Matrixmaterial. In einer bevorzugten Ausführungsform der Erfindung ist das weitere Matrixmaterial eine lochtransportierende Verbindung. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindund mit großem Bandabstand, das nicht oder nicht in wesentlichem Umfang am Loch- und Elektronentransport in der Schicht beteiligt ist.

Geeignete Matrixmaterialien, welche in Kombination mit den Verbindungen gemäß Formel (1) bzw. Formel (2) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, oder 4-Spirocarbazol-Derivate, z. B. gemäß WO 2014/094963 oder der noch nicht offen gelegten Anmeldung EP 14002104.9. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (11), 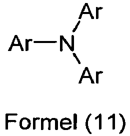 wobei Ar gleich oder verschieden bei jedem Auftreten die oben genannten Bedeutungen aufweist. Bevorzugt sind die Gruppen Ar gleich oder verschieden bei jedem Auftreten ausgewählt aus den oben genannten Gruppen Ar-1 bis Ar-19.

In einer bevorzugten Ausführungsform der Verbindungen der Formel (11) ist mindestens eine Gruppe Ar ausgewählt aus einer Biphenylgruppe, wobei es sich um eine ortho-, meta- oder para-Biphenylgruppe handeln kann. In einer weiteren bevorzugten Ausführungsform der Verbindungen der Formel (11) ist mindestens eine Gruppe Ar ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (11) ist mindestens eine Gruppe Ar ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (11) ist eine Gruppe Ar ausgewählt aus einer Fluoren- oder Spirobifluorengruppe, insbesondere einer 4-Fluoren- bzw. 4-Spirobifluorengruppe, und eine Gruppe Ar ist ausgewählt aus einer Biphenylgruppe, insbesondere einer para-Biphenylgruppe, oder einer Fluorengruppe, insbesondere einer 2-Fluorengruppe, und die dritte Gruppe Ar ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (12), 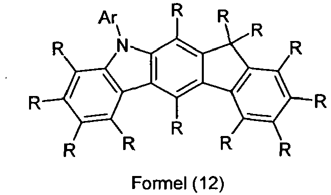 wobei Ar und R die oben aufgeführten Bedeutungen aufweisen. Dabei sind bevorzugte Ausführungsformen der Gruppe Ar die oben aufgeführten Strukturen Ar-1 bis Ar-19.

Eine bevorzugte Ausführungsform der Verbindungen der Formel (12) sind die Verbindungen der folgenden Formel (12a), 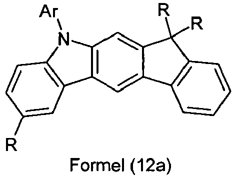 wobei Ar und R die oben genannten Bedeutungen aufweisen. Dabei stehen die beiden Gruppen R, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen R, die an das Indenokohlenstoffatom gebunden sind, für Methylgruppen. Weiterhin bevorzugt steht der Substituent R, der in Formel (12a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (13), 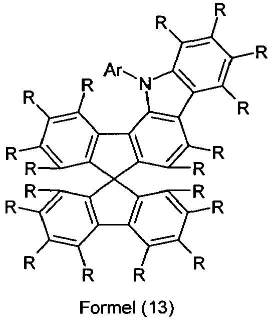 wobei Ar und R die oben aufgeführten Bedeutungen aufweisen. Dabei sind bevorzugte Ausführungsformen der Gruppe Ar die oben aufgeführten Strukturen Ar-1 bis Ar-19.

Eine bevorzugte Ausführungsform der Verbindungen der Formel (13) sind die Verbindungen der folgenden Formel (13a), 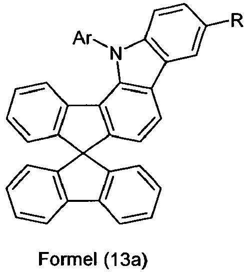 wobei Ar und R die oben genannten Bedeutungen aufweisen.

Bevorzugte Lactame, die als Co-Host-Materialien zusammen mit den erfindungsgemäßen Verbindungen eingesetzt werden, sind ausgewählt aus den Verbindungen der folgenden Formel (14), 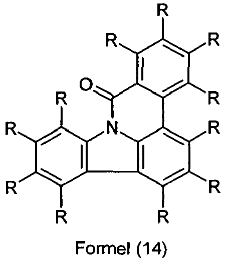 wobei R die oben aufgeführten Bedeutungen aufweist.

Eine bevorzugte Ausführungsform der Verbindungen der Formel (14) sind die Verbindungen der folgenden Formel (14a), 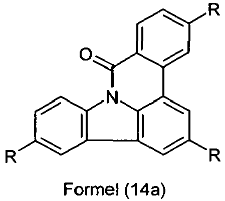 wobei R die oben genannten Bedeutungen aufweist. Dabei steht R bevorzugt gleich oder verschieden bei jedem Auftreten für H oder ein aromatisches oder heteroaromatisches Ringsystem mit 5 bis 40 aromatischen Ringatomen, das mit einem oder mehreren Resten R¹ substituiert sein kann. Ganz besonders bevorzugt sind die Substituenten R ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten R¹ substituiert sein kann, bevorzugt aber unsubstituiert ist. Beispiele für geeignete Substituenten R sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R¹ substituiert sein können, bevorzugt aber unsubstituiert sind. Dabei sind geeignete Strukturen R die gleichen Strukturen, wie sie vorne für Ar-1 bis Ar-19 abgebildet sind, wobei diese Strukturen durch R¹ statt R substituiert sind.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochblockier- oder Elektronentransportschicht einzusetzen. Dies gilt insbesondere für erfindungsgemäße Verbindungen, die keine Carbazolstruktur aufweisen. Diese können bevorzugt auch mit einer oder mehreren weiteren elektronentransportierenden Gruppen substituiert sein, beispielsweise Benzimidazolgruppen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. Formel (2) bzw. gemäß den bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren aufgebracht werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation aufgebracht werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Die Erfindung wird nun durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### Synthese beispiele

### a)Triazinsynthese: 2,4-Bis-biphenyl-3-yl-6-chlor-[1,3,5]triazin

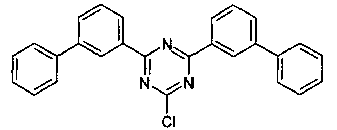

5.2 g Magnesium (0.215 mol) werden in einem 500 mL Vierhalskolben vorgelegt, und eine Lösung aus 50 g Brombiphenyl (214 mmol) in 200 ml THF wird langsam zugetropft. Die Reaktionsmischung wird 1.5 h zum Sieden erhitzt und anschließend auf Raumtemperatur abgekühlt. In einem zweiten Kolben wird Cyanchlorid (17.2 g, 93 mmol) in 150 ml THF vorgelegt und auf 0 °C gekühlt. Bei dieser Temperatur wird das abgekühlte Grignard-Reagenz zugetropft und 12 h bei Raumtemperatur gerührt. Nach dieser Zeit wird die Reaktionsmischung mit 150 ml HCl versetzt und die wässrige Phase dreimal mit Dichlormethan extrahiert. Die vereinigten organischen Phasen werden mit Wasser gewaschen, über Na₂SO₄ getrocknet und eingeengt. Der Rückstand wird aus EtOH umkristallisiert. Die Ausbeute beträgt 32.8 g (78 mmol, 84%).

### b) 4-Brom-9-methyl-9-phenyl-9H-fluoren

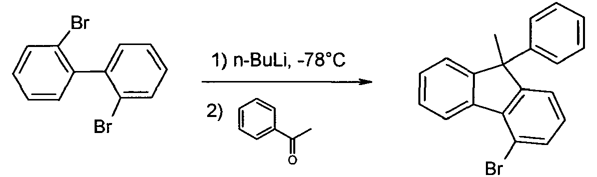

30 g (94 mmol) 2,2'-Dibrom-biphenyl werden in einem ausgeheizten Kolben in 200 mL getrocknetem THF gelöst. Die Reaktionsmischung wird auf -78 °C gekühlt. Bei dieser Temperatur werden 37.7 mL einer 2.5 M-Lösung n-Butyllithium in Hexan (94 mmol) langsam zugetropft (Dauer: ca 1 h). Der Ansatz wird 1 h bei -70 °C nachgerührt. Anschließend werden 11.1 mL Acetophenon (94 mmol) in 100 ml THF gelöst und bei -70 °C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 300 ml Essigsäure versetzt, und anschließend werden 50 ml rauchende HCl zugegeben. Der Ansatz wird 6 h auf 75 °C erhitzt. Dabei fällt ein weißer Feststoff aus. Der Ansatz wird auf Raumtemperatur abgekühlt, der ausgefallene Feststoff wird abgesaugt und mit Methanol nachgewaschen. Der Rückstand wird im Vakuum bei 40 °C getrocknet. Die Ausbeute beträgt 25.3 g (75 mmol) (80% der Theorie)

### c) 4-Brom-9,9-diphenyl-9H-fluoren

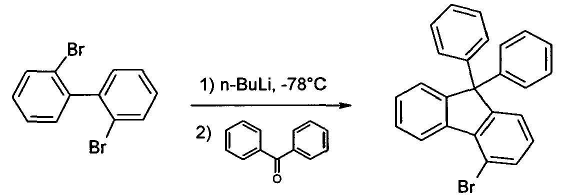

37 g (152 mmol) 2,2'-Dibrom-biphenyl werden in einem ausgeheizten Kolben in 300 mL getrocknetem THF gelöst. Die Reaktionsmischung wird auf -78 °C gekühlt. Bei dieser Temperatur werden 75 mL einer 15%-igen Lösung n-Butyllithium in Hexan (119 mmol) langsam zugetropft (Dauer: ca 1 h). Der Ansatz wird 1 h bei -70 °C nachgerührt. Anschließend werden 21.8 g Benzophenon (119 mmol) in 100 ml THF gelöst und bei -70 °C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 510 ml Essigsäure versetzt und anschließend werden 100 ml rauchende HCl zugegeben. Der Ansatz wird 4 h auf 75 °C erhitzt. Dabei fällt ein weißer Feststoff aus. Der Ansatz wird dann auf Raumtemperatur abgekühlt, der ausgefallene Feststoff wird abgesaugt und mit Methanol nachgewaschen. Der Rückstand wird im Vakuum bei 40 °C getrocknet. Ausbeute beträgt 33.2 g (83 mmol) (70% der Theorie).

Analog dazu werden die folgenden bromierten Verbindungen hergestellt:

| | **Edukt 1** | **Edukt 2** | **Produkt** | **Ausbeute** |
|---|---|---|---|---|
| c1 | 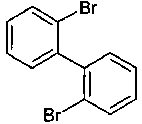 | 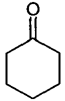 | 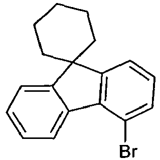 | 78% |
| c2 | 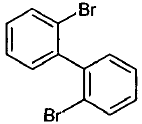 | 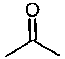 | 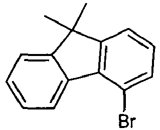 | 70% |
| c3 | 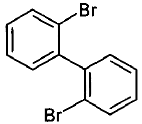 | 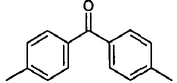 | 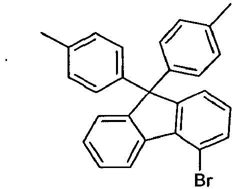 | 82% |

### d) 6-Brom-2-fluor-2'-methoxy-biphenyl

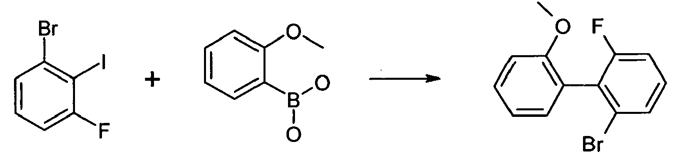

200 g (664 mmol) 1-Brom-3-fluor-2-iodbenzol, 101 g (664 mmol) 2-Methoxyphenylboronsäure und 137.5 g (997 mmol) Natriumtetraborat werden in 1000 mL THF und 600 ml Wasser gelöst und entgast. Es wird mit 9.3 g (13.3 mmol) Bis(triphenylphosphin)palladium(II)chlorid und 1 g (20 mmol) Hydraziniumhydroxid versetzt. Die Reaktionsmischung wird anschließend bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 155 g (553 mmol), 83 % der Theorie.

Analog dazu werden die folgenden Verbindungen hergestellt:

| | **Edukt 1** | **Edukt 2** | **Produkt** | **Ausbeute** |
|---|---|---|---|---|
| d1 | 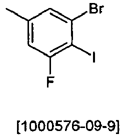 | 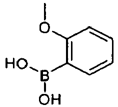 | 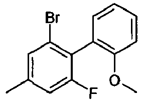 | 77% |
| d2 | 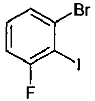 | 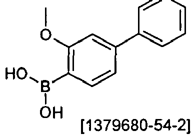 | 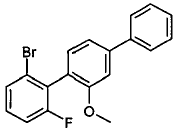 | 74% |
| d3 | 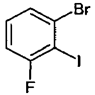 | 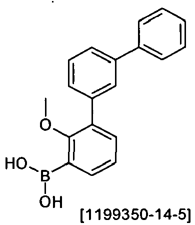 | 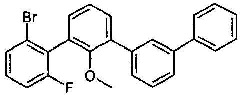 | 76% |
| d4 | 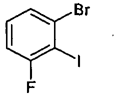 | 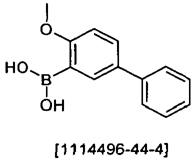 | 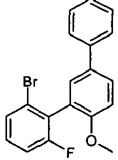 | 71% |

### e) 6'-Brom-2'fluor-biphenyl-2-ol

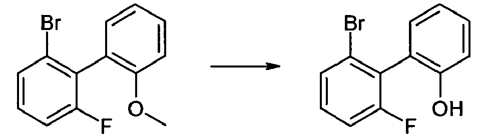

112 g (418 mmol) 6-Brom-2-fluor-2'-methoxy-biphenyl werden in 2 L Dichloromethan gelöst und auf 5 °C gekühlt. Zu dieser Lösung wird innerhalb von 90 min. 41.01 ml (431 mmol) Bortribromid zugetropft und über Nacht weiter gerührt. Das Gemisch wird im Anschluss langsam mit Wasser versetzt, die organische Phase dreimal mit Wasser gewaschen, über Na₂SO₄ getrocknet, einrotiert und chromatographisch gereinigt. Ausbeute: 104 g (397 mmol), 98 % der Theorie.

Analog dazu werden die folgenden Verbindungen hergestellt:

| | **Edukt 1** | **Produkt** | **Ausbeute** |
|---|---|---|---|
| e1 | 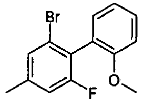 | 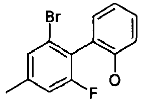 | 92% |
| e2 | 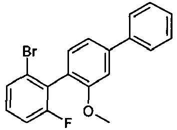 | 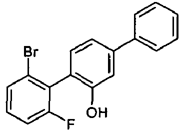 | 90% |
| e3 | 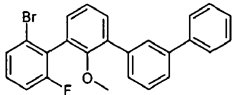 | 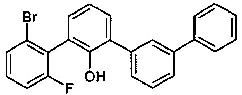 | 93% |
| e4 | 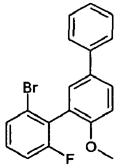 | 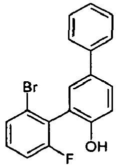 | 94% |

### f) 1-Brom-dibenzofuran

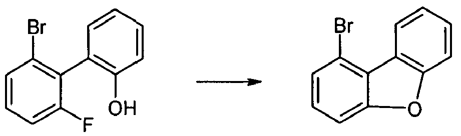

111 g (416 mmol) 6'-Brom-2'-fluor-biphenyl-2-ol wird in 2 L DMF (max. 0.003 % H₂O)SeccoSolv® gelöst und auf 5 °C gekühlt. Zu dieser Lösung wird portionsweise 20 g (449 mmol) Natriumhydrid (60% Suspension in Paraffinöl) zugegeben, nach beendeter Zugabe 20 min. nachgerührt und dann für 45 min. auf 100 °C erhitzt. Das Gemisch wird nach dem Abkühlen langsam mit 500 ml Ethanol versetzt, einrotiert und dann chromatographisch gereinigt. Ausbeute: 90 g (367 mmol), 88.5 % der Theorie.

Analog dazu werden die folgenden Verbindungen hergestellt:

| | **Edukt 1** | **Produkt** | **Ausbeute** |
|---|---|---|---|
| f1 | 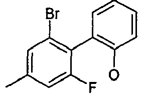 | 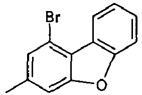 | 81% |
| f2 | 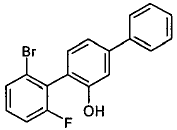 | 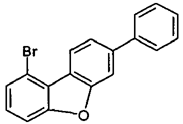 | 78% |
| f3 | 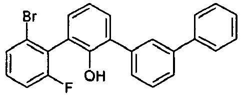 | 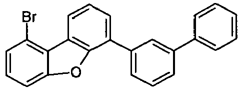 | 73% |
| f4 | 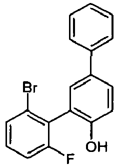 | 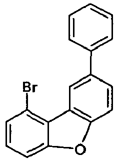 | 79% |

### g) Dibenzofuran-1-boronsäure und Boronsäureester

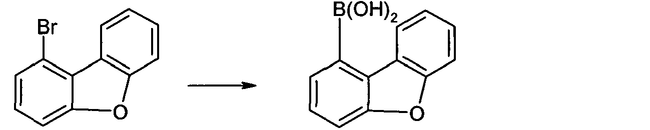

180 g (728 mmol) 1-Brom-dibenzofuran werden in 1500 mL trockenem THF gelöst und auf -78 °C gekühlt. Bei dieser Temperatur wird mit 305 mL (764 mmol / 2.5 M in Hexan) n-Butyllithium innerhalb von ca. 5 min. versetzt und anschließend für 2.5 h bei -78 °C nachgerührt. Bei dieser Temperatur wird mit 151 g (1456 mmol) Borsäuretrimethylester möglichst zügig versetzt und die Reaktion langsam auf Raumtemperatur kommen gelassen (ca. 18 h). Die Reaktionslösung wird mit Wasser gewaschen und der ausgefallene Feststoff abfiltriert und die organische Phase mit Toluol azeotrop getrocknet. Das Rohprodukt wird mit Toluol/Methylenchlorid bei ca. 40 °C ausgerührt und abgesaugt. Ausbeute: 146 g (690 mmol), 95 % der Theorie.

Analog dazu werden die folgenden Verbindungen hergestellt:

| | **Edukt 1** | **Produkt** | **Ausbeute** |
|---|---|---|---|
| g1 | 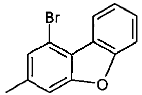 | 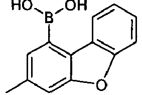 | 81% |
| g2 | 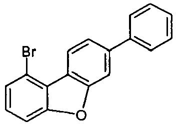 | 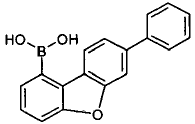 | 78% |
| g3 | 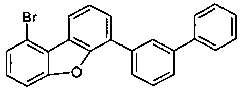 | 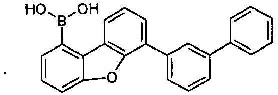 | 73% |
| g4 | 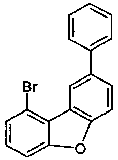 | 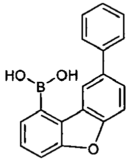 | 79% |
| g5 | 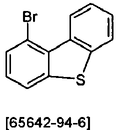 | 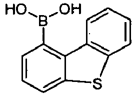 | 73% |
| g6 | 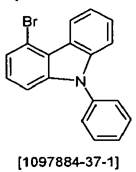 | 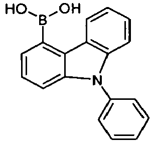 | 70% |
| g7 | 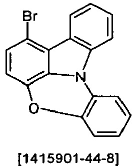 | 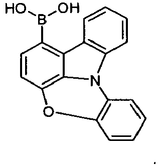 | 70% |
| g8 | 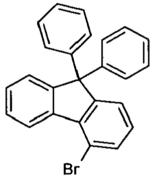 | 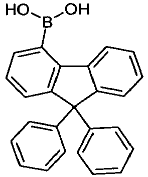 | 78% |
| g9 | 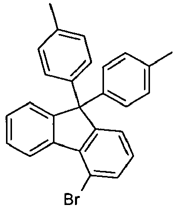 | 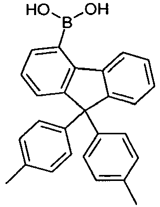 | 81% |
| g10 | 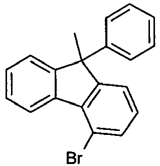 | 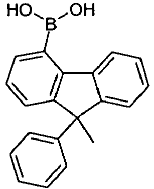 | 86% |
| g11 | 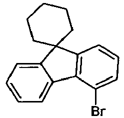 | 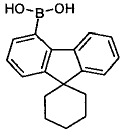 | 83% |
| g12 | 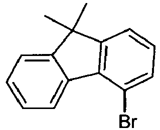 | 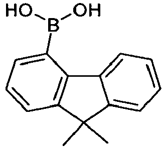 | 85% |
| g13 | 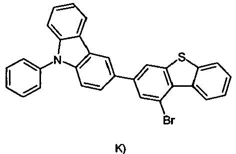 | 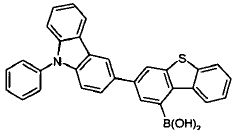 | 82% |
| g14 | 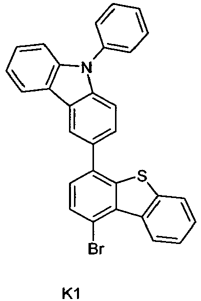 | 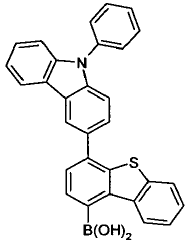 | 80% |
| g15 | 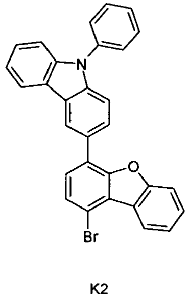 | 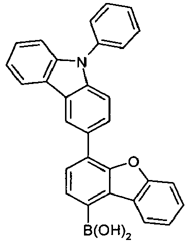 | 83% |
| g16 | 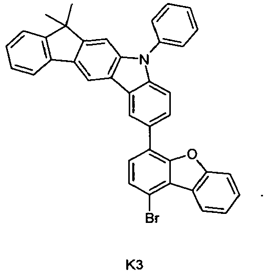 | 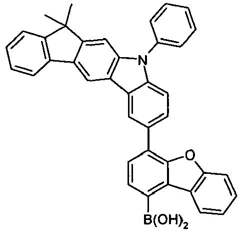 | 82% |
| g17 | 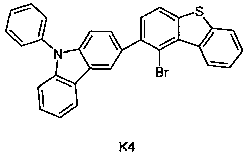 | 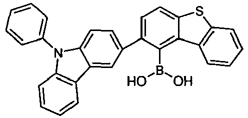 | 81% |
| g18 | 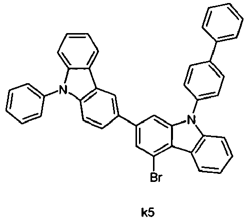 | 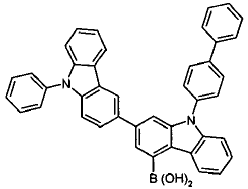 | 84% |

### g19) Synthese von 1-(4,4,5,5-tetramethyl-[1,2,3]dioxaborolan-2yl)-dibenzafuran

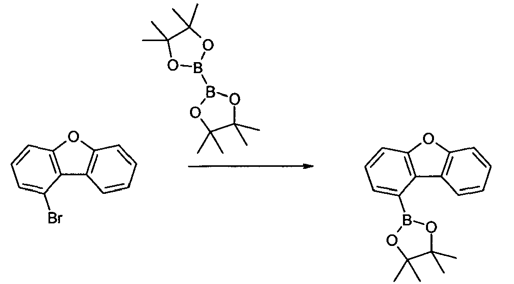

In einem 500 ml-Kolben werden unter Schutzgas 101 g (410 mmol) des 1-Bromdibenzofurans zusammen mit 273 g (1055 mmol) Bis(pinacolato)-diboran (CAS 73183-34-3) in 1500 ml trockenem DMF gelöst und für 30 Minuten entgast. Anschließend werden 121 g (1229 mmol) Kaliumacetat und 8.4 g (37 mmol) Palladiumacetat zugegeben, und der Ansatz wird über Nacht auf 80 °C erhitzt. Nach beendeter Reaktion wird mit 300 ml Toluol verdünnt und das Gemisch mit Wasser extrahiert. Das Lösungsmittel wird am Rotationsverdampfer entfernt und das Produkt wird aus Heotan umkristallisiert. Ausbeute: 118 g (401 mmol), 98 % der Theorie.

### h) 2-Dibenzofuran-1-yl-4,6-dipheniyl-[1,3,5]triazin

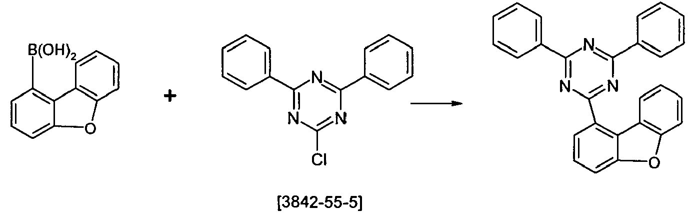

23 g (110.0 mmol) Dibenzofuran-1-boronsäure, 29.5 g (110.0 mmol) 2-Chlor-4,6-diphenyl-1,3,5-triazin und 21 g (210.0 mmol) Natriumcarbonat werden in 500 mL Ethylenglycoldiaminether und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan /Heotan umkristallisiert. Die Ausbeute beträgt 37 g (94 mmol), entsprechend 87 % der Theorie.

Analog dazu werden die folgenden Verbindungen hergestellt:

| | **Edukt 1** | **Edukt 2** | **Produkt** | **Ausbeute** |
|---|---|---|---|---|
| h1 | 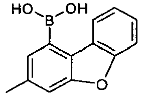 | 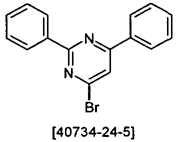 | 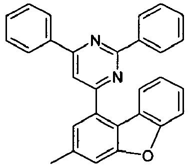 | 73% |
| h2 | 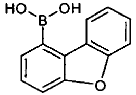 | 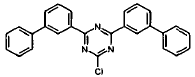 | 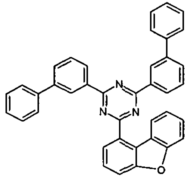 | 82% |
| h3 | 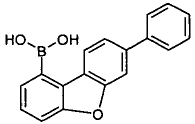 | 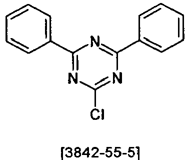 | 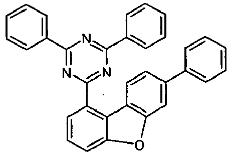 | 73% |
| h4 | 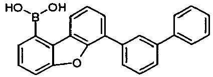 | 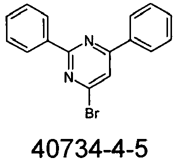 | 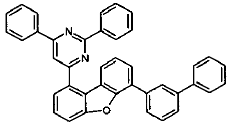 | 72% |
| h5 | 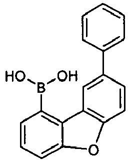 | 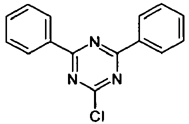 | 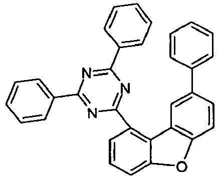 | 65% |
| h6 | 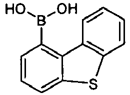 | 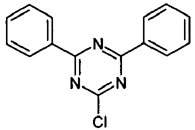 | 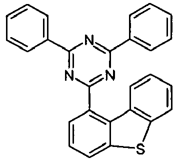 | 63% |
| h7 | 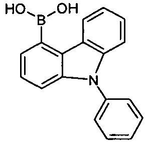 | 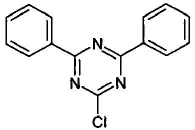 | 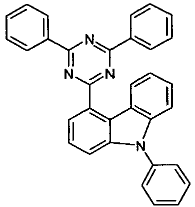 | 72% |
| h8 | 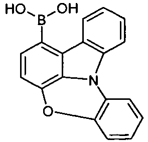 | 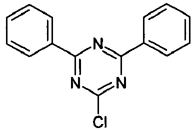 | 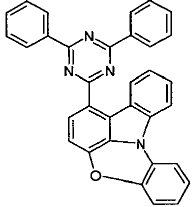 | 75% |
| h9 | 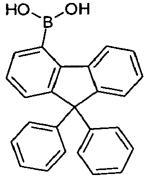 | 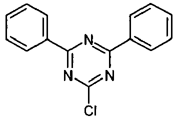 | 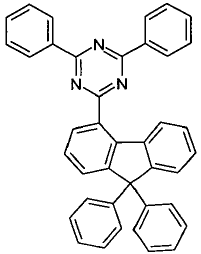 | 79% |
| h10 | 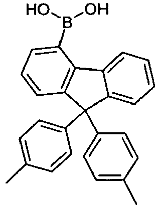 | 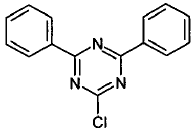 | 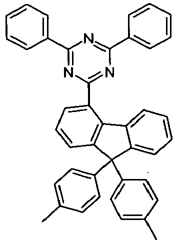 | 81% |
| h11 | 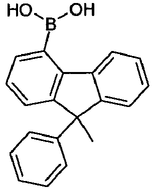 | 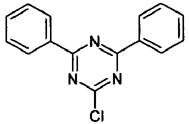 | 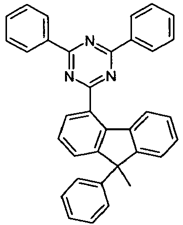 | 85% |
| h12 | 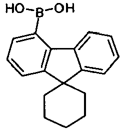 | 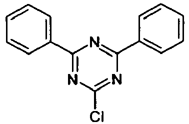 | 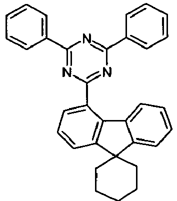 | 80% |
| h13 | 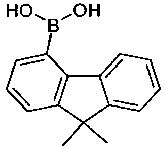 | 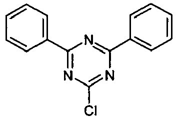 | 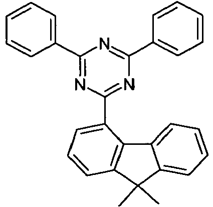 | 79% |
| h14 | 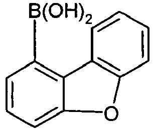 | 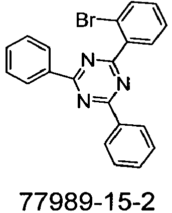 | 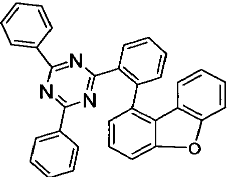 | 77% |
| h15 | 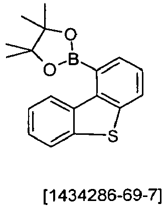 | 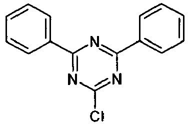 | 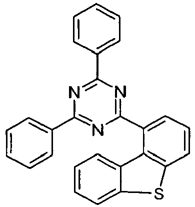 | 76% |
| h16 | 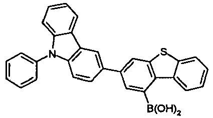 | 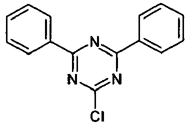 | 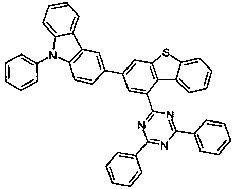 | 71% |
| h17 | 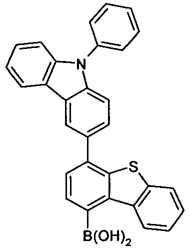 | 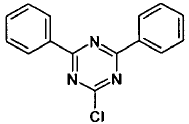 | 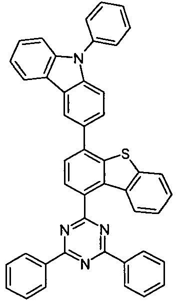 | 75% |
| h18 | 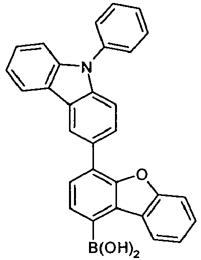 | 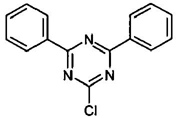 | 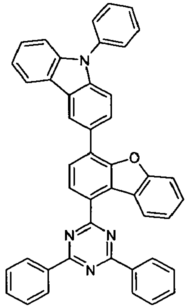 | 76% |
| h19 | 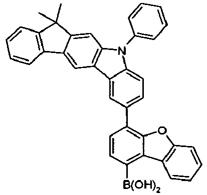 | 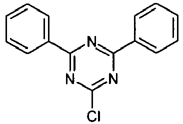 | 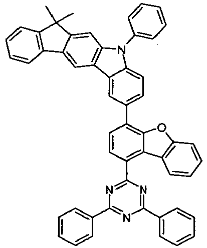 | 80% |
| h20 | 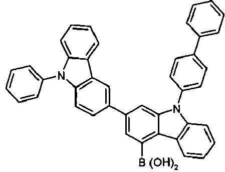 | 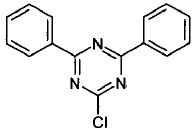 | 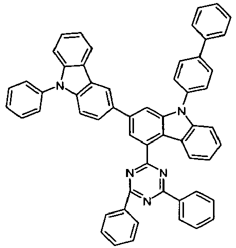 | 79% |
| h21 | 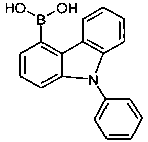 | 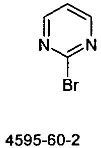 | 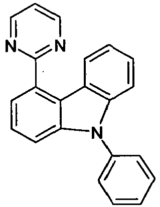 | 82% |

### i) 2-(8-Brom-dibenzofuran-1-yl)-4,6-diphenyl-[1,3,5]triazin

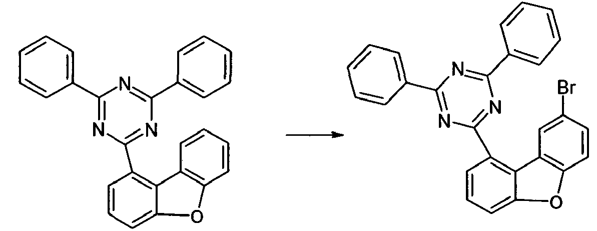

70 g (190.0 mmol) 2-Dibenzofuran-1-yl-4,6-diphenyl-[1,3,5]triazin werden in 2000 mL Essigsäure(100%) und 2000 mL Schwefelsäure (95-98%) suspendiert. Zu dieser Suspension werden portionsweise 34 g (190 mmol) NBS zugegeben und 2 h in Dunkelheit gerührt. Danach mit Wasser/Eis versetzt, der Feststoff abgetrennt und mit Ethanol nachgewaschen. Der Rückstand wird aus Toluol umkristallisiert. Die Ausbeute beträgt 80 g (167 mmol), entsprechend 87 % der Theorie.

Analog dazu werden die folgenden Verbindungen hergestellt:

| | **Edukt 1** | **Produkt** | **Ausbeute** |
|---|---|---|---|
| i1 | 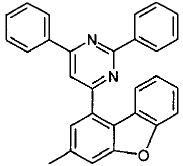 | 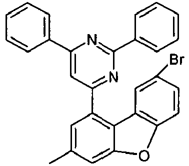 | 80% |
| i2 | 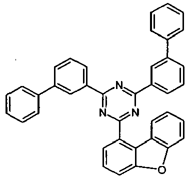 | 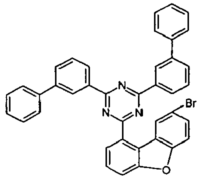 | 41% |
| i3 | 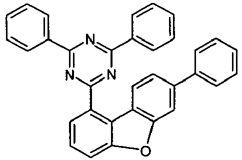 | 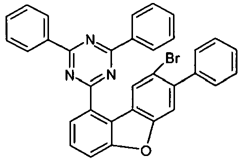 | 52% |
| i4 | 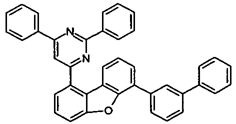 | 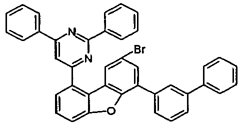 | 64% |
| i5 | 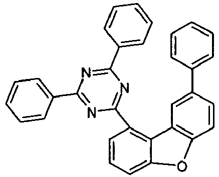 | 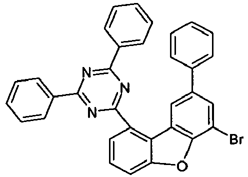 | 33% |
| i6 | 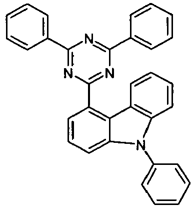 | 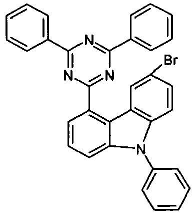 | 73% |
| i7 | 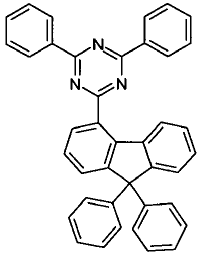 | 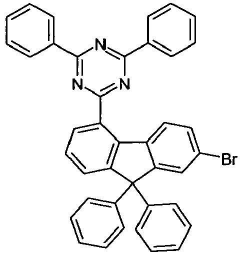 | 78% |
| i8 | 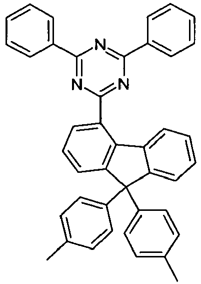 | 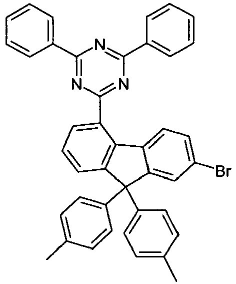 | 80% |
| i9 | 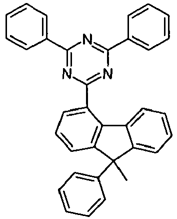 | 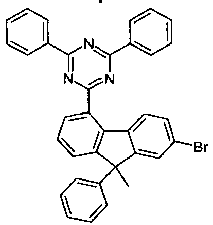 | 70% |
| i10 | 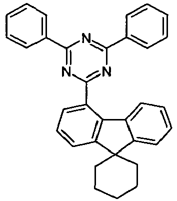 | 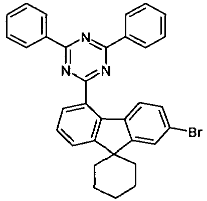 | 86% |
| i11 | 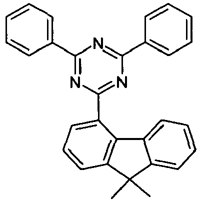 | 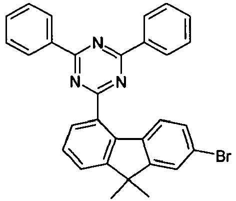 | 88% |
| i12 | 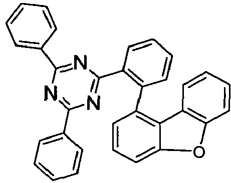 | 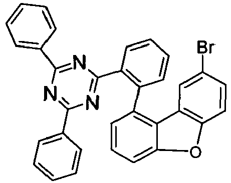 | 41% |
| i13 | 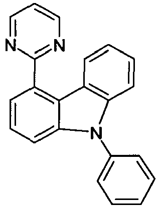 | 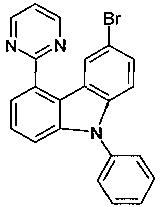 | 73% |

Im Falle der Dibenzothiophen-Derivate wird Nitrobenzol statt Schwefelsäure und elementares Brom an Stelle von NBS eingesetzt:

| | | | |
|---|---|---|---|
| i14 | 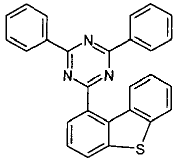 | 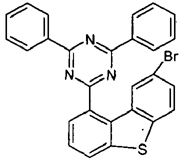 | 55% |
| i15 | 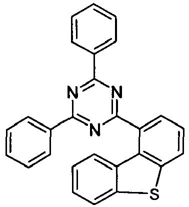 | 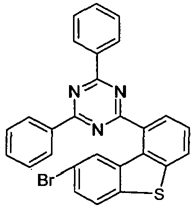 | 52% |

### j) 3-[9-(4,6-Diphenyl-[1,3,5]triazin-2-yl)-dibenzofuran-2-yl]-9-phenyl-9H-carbazol

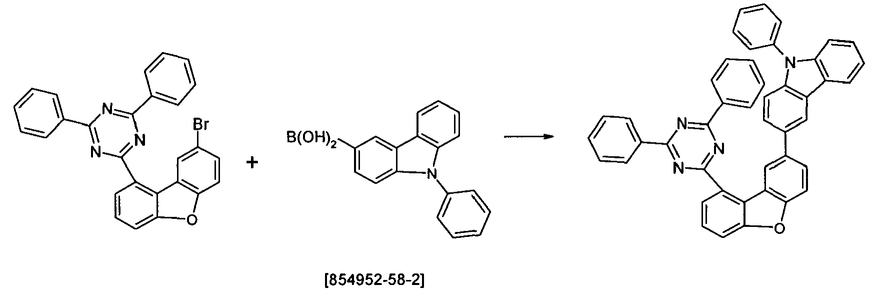

75 g (156 mmol) 2-(8-Brom-dibenzofuran-1-yl)-4,6-diphenyl-[1,3,5]-triazin, 50 g (172 mmol) N-Phenyl-carbazol-3-boronsäure und 36 g (340 mmol) Natriumcarbonat werden in 1000 mL Ethylenglycoldiaminether und 280 mL Wasser suspendiert. Zu dieser Suspension werden 1.8 g (1.5 mmol) Tetrakis(triphenylphosphin)-palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Das Produkt wird via Säulenchromatographie an Kieselgel mit Toluol/Heptan (1:2) gereinigt und abschließend im Hochvakuum (p = 5 x 10⁻⁷ mbar) sublimiert (Reinheit 99,9%). Die Ausbeute beträgt 50 g (78mmol), entsprechend 50 % der Theorie.

Analog dazu werden die folgenden Verbindungen hergestellt:

| | | | | |
|---|---|---|---|---|
| | **Edukt 1** | **Edukt 2** | **Produkt** | **Ausbeute** |
| j1 | 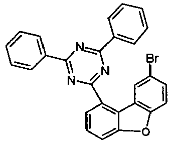 | 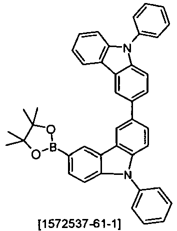 | 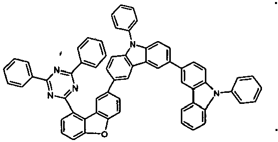 | 61% |
| j2 | 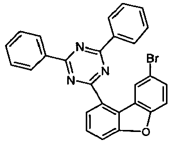 | 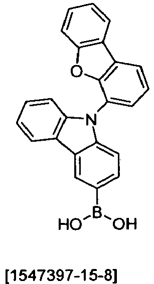 | 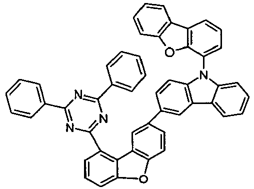 | 67% |
| j3 | 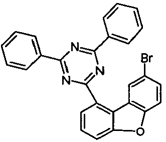 | 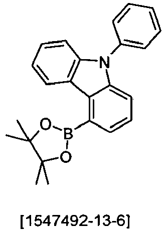 | 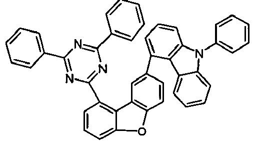 | 65% |
| j4 | 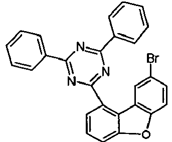 | 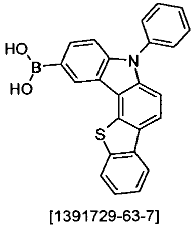 | 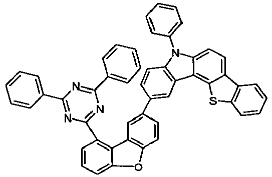 | 53% |
| j5 | 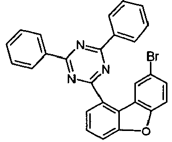 | 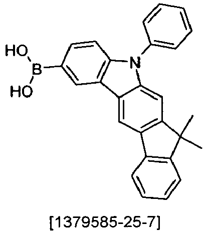 | 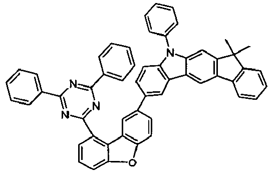 | 58% |
| j6 | 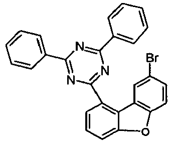 | 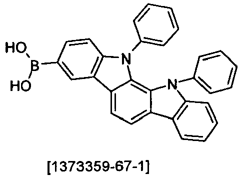 | 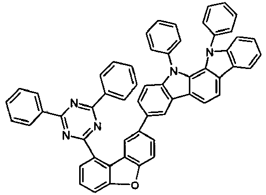 | 54% |
| j7 | 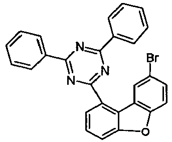 | 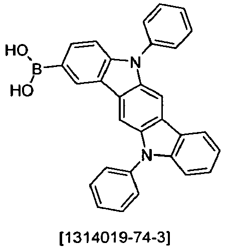 | 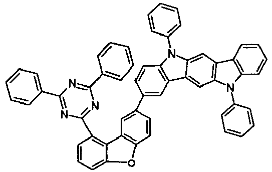 | 65% |
| j8 | 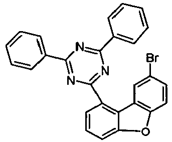 | 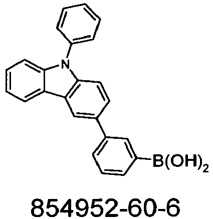 | 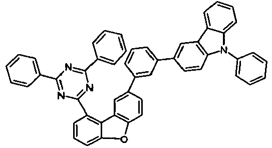 | 71% |
| j9 | 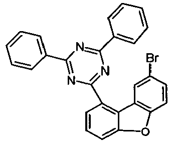 | 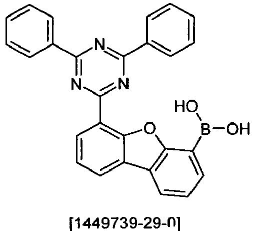 | 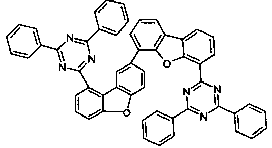 | 56% |
| j10 | 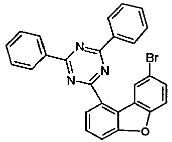 | 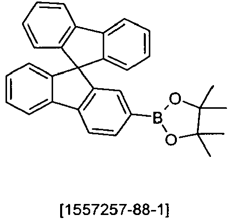 | 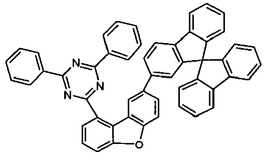 | 79% |
| j11 | 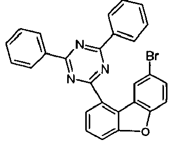 | 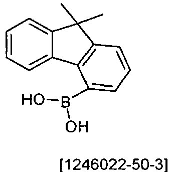 | 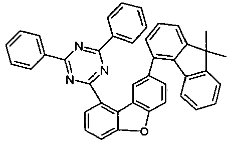 | 70% |
| j12 | 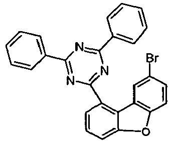 | 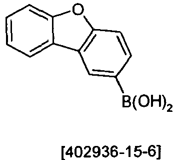 | 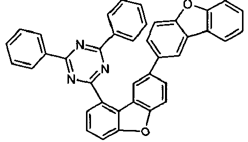 | 82% |
| j13 | 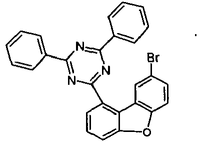 | 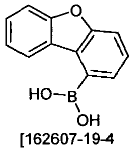 | 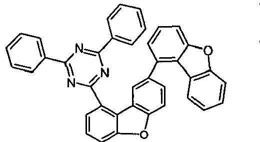 | 69% |
| j14 | 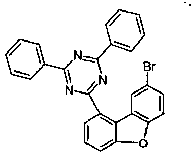 | 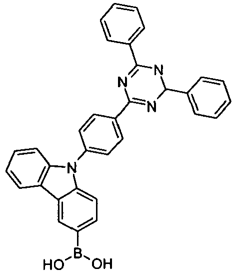 | 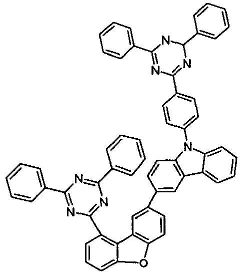 | 65% |
| j15 | 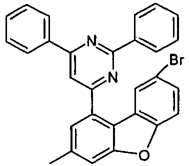 | 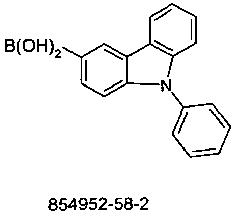 | 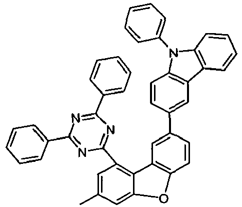 | 77% |
| j16 | 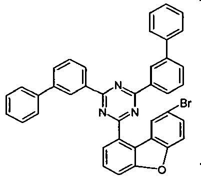 | 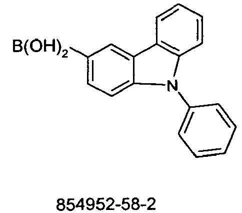 | 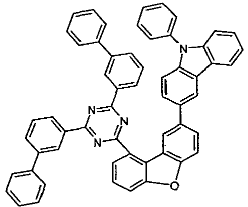 | 82% |
| j17 | 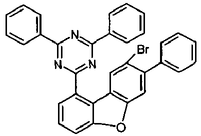 | 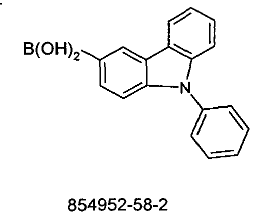 | 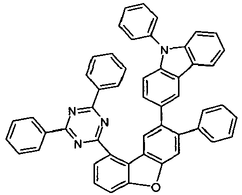 | 54% |
| j18 | 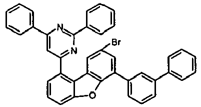 | 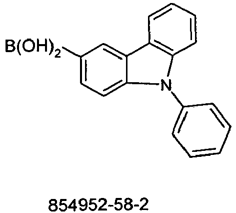 | 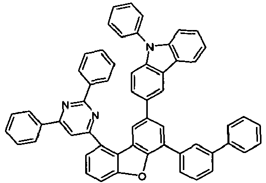 | 67% |
| j19 | 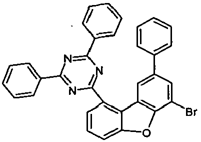 | 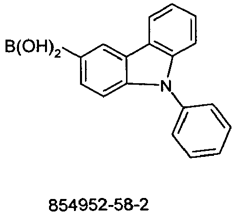 | 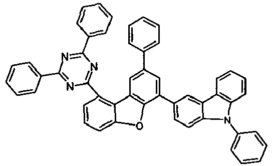 | 65% |
| j20 | 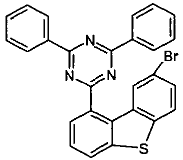 | 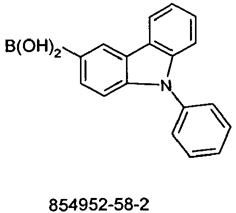 | 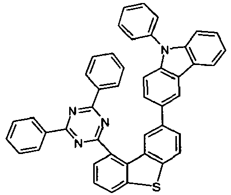 | 63% |
| j21 | 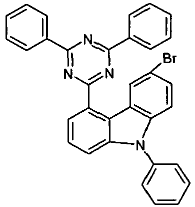 | 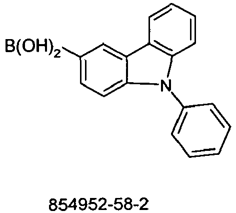 | 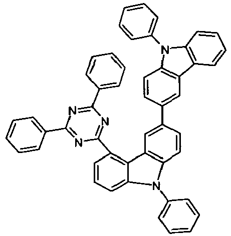 | 79% |
| j22 | 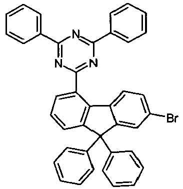 | 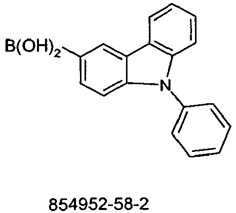 | 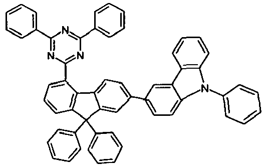 | 65% |
| j23 | 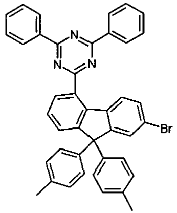 | 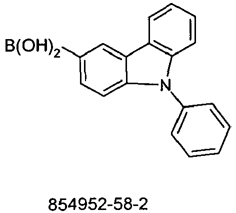 | 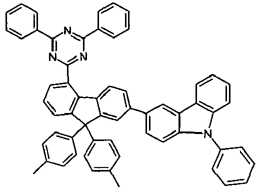 | 55% |
| j24 | 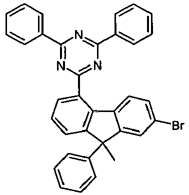 | 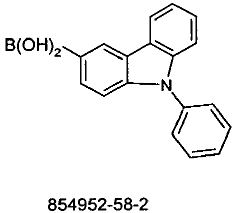 | 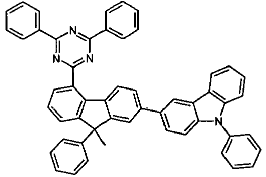 | 67% |
| j25 | 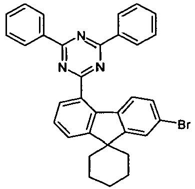 | 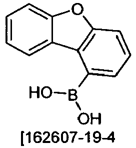 | 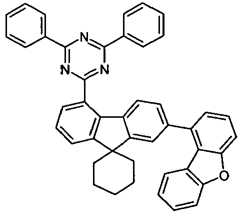 | 73% |
| j26 | 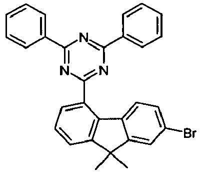 | 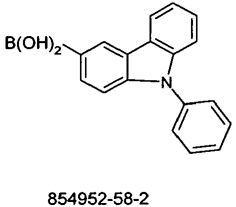 | 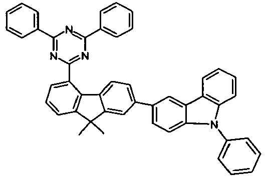 | 66% |
| j27 | 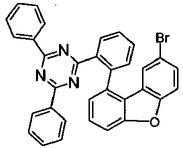 | 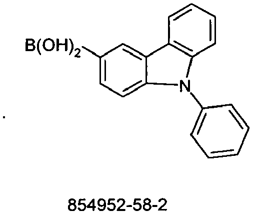 | 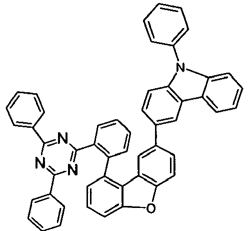 | 59% |
| j28 | 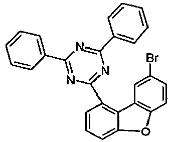 | 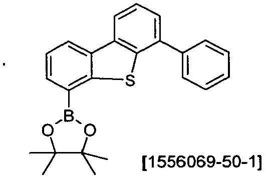 | 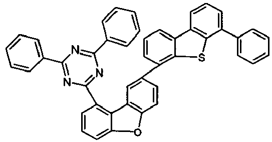 | 64% |
| j29 | 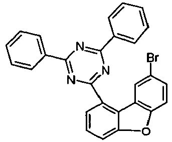 | 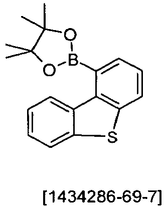 | 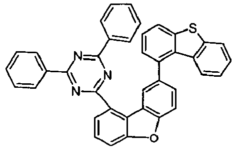 | 71% |
| j30 | 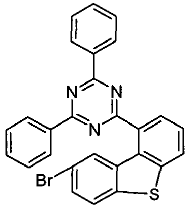 | 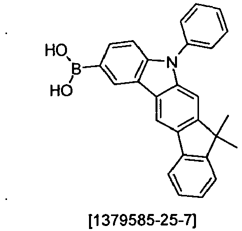 | 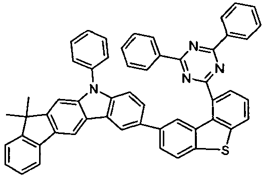 | 62% |

Analog dazu werden die folgenden Verbindungen mit 0.5 Äquivalenten des entsprechenden Bromids hergestellt:

| | | | | |
|---|---|---|---|---|
| j31 Vgl. | 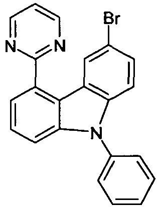 | 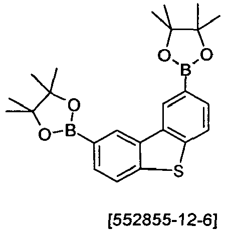 | 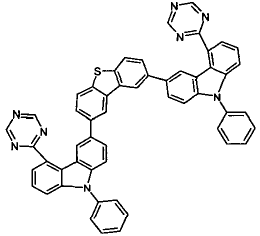 | 72% |
| j32 Vgl. | 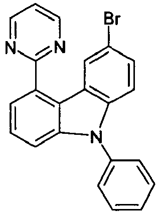 | 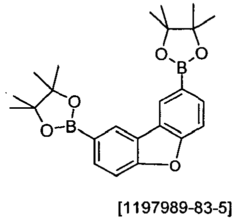 | 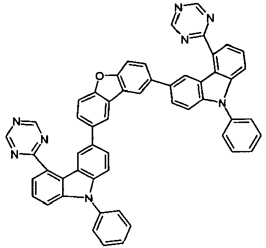 | 73% |
| j33 | 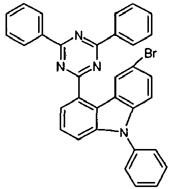 | 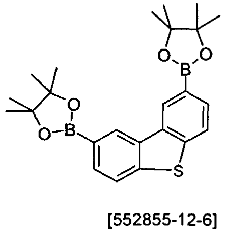 | 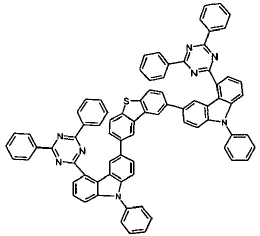 | 74% |
| j34 | 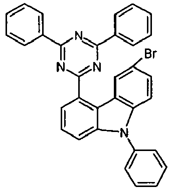 | 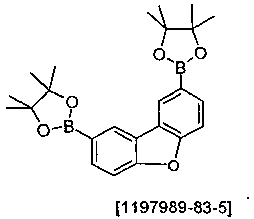 | 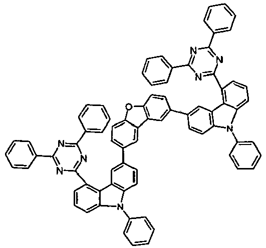 | 77% |

### k) 3-(1-Brom-dibenzothiophen-3-yl)-9-phenyl-9H-carbazol

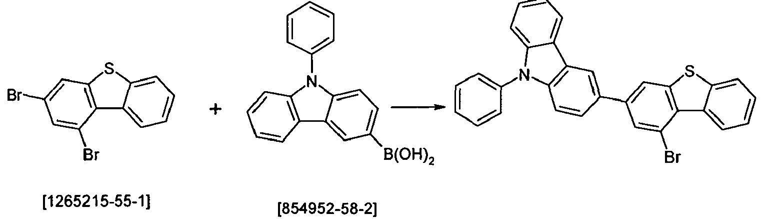

22 g (66 mmol) 1,3-Dibrom-dibenzothiophen, 17 g (664 mmol) N-Phenylcarbazol-3-boronsäure und 13.7 g (100 mmol) Natriumtetraborat werden in 100 mL THF und 60 ml Wasser gelöst und entgast. Es wird mit 0.9 g (1,3 mmol) Bis(triphenylphosphin)palladium(II)chlorid und 1 g (20 mmol) Hydraziniumhydroxid versetzt. Die Reaktionsmischung wird anschließend bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 13.2 g (26 mmol), 40 % der Theorie.

Analog dazu werden die folgenden Verbindungen hergestellt:

| | **Edukt 1** | **Edukt 2** | **Produkt** | **Ausbeute** |
|---|---|---|---|---|
| k1 | 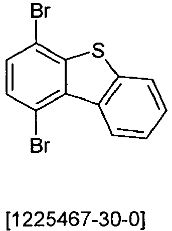 | 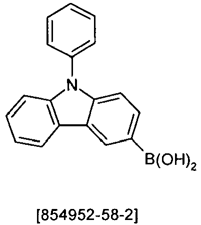 | 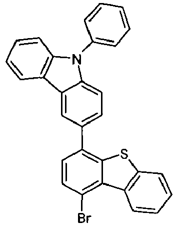 | 27% |
| k2 | 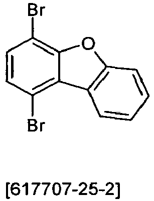 | 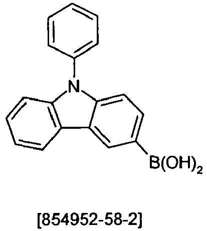 | 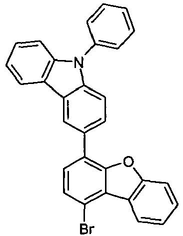 | 29% |
| k3 | 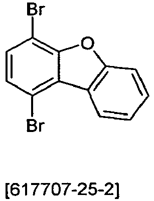 | 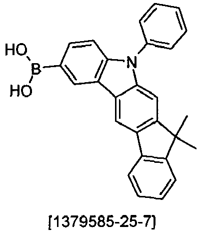 | 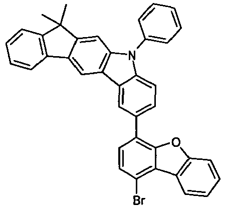 | 34% |
| k4 | 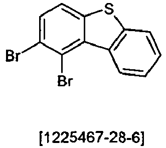 | 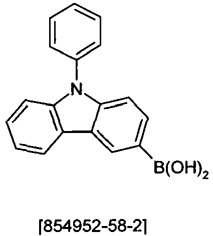 | 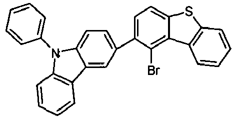 | 24% |
| k5 | 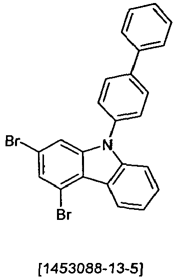 | 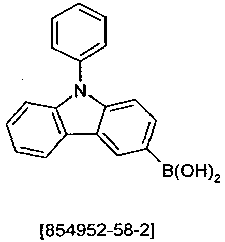 | 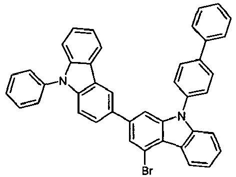 | 31% |

### l) 1-Brom-8-iod-dibenzofuran

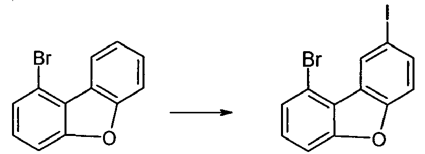

20 g (80 mmol) Dibenzofuran-1-boronsäure, 2.06 g (40.1 mmol) Iod, 3.13 g (17.8 mmol) Iodsäure, 80 ml Essigsäure, 5 ml Schwefelsäure, 5 ml Wasser und 2 ml Chloroform werden 3 h bei 65 °C gerührt. Nach Erkalten wird die Mischung mit Wasser verestzt, der ausgefallene Feststoff abgesaugt und dreimal mit Wasser gewaschen. Der Rückstand wird aus Toluol und aus Dichlormethan /Heptan umkristallisiert. Die Ausbeute beträgt 25.6 g (68 mmol), entsprechend 85 % der Theorie.

Analog dazu werden die folgenden Verbindungen hergestellt:

| | **Edukt 1** | **Produkt** | **Ausbeute** |
|---|---|---|---|
| l1 | 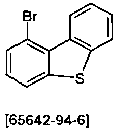 | 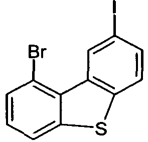 | 81% |
| l2 | 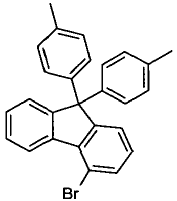 | 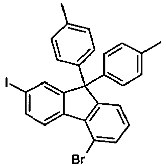 | 84% |
| l3 | 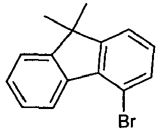 | 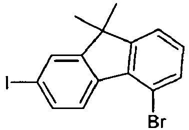 | 78% |

### m) 3-(9-Brom-dibenzofuran-2-yl)-9-phenyl-9H-carbazol

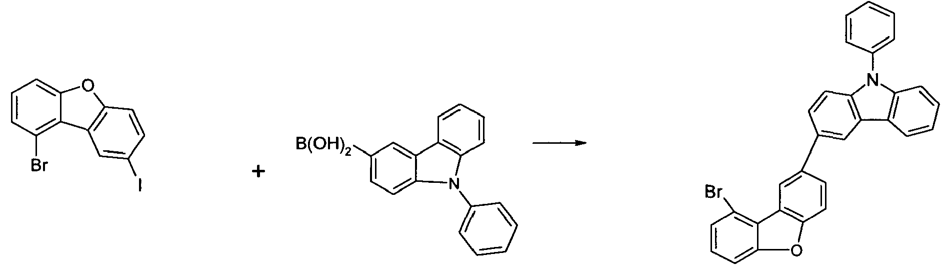

58 g (156 mmol) 1-Brom-8-iod-dibenzofuran, 50 g (172 mmol) N-Phenylcarbazol-3-boronsäure und 36 g (340 mmol) Natriumcarbonat werden in 1000 mL Ethylenglycoldiaminether und 280 mL Wasser suspendiert. Zu dieser Suspension werden 1.8 g (1.5 mmol) Tetrakis(triphenylphosphin)-palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Die Ausbeute beträgt 48 g (89 mmol), entsprechend 64 % der Theorie.

Analog dazu werden die folgenden Verbindungen hergestellt:

| | **Edukt 1** | **Edukt 2** | **Produkt** | **Ausbeute** |
|---|---|---|---|---|
| m1 | 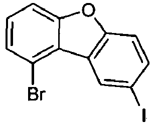 | 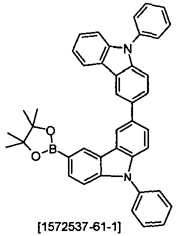 | 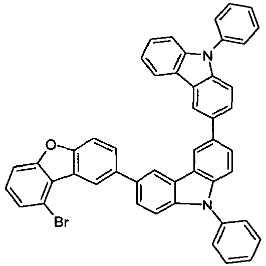 | 60% |
| m2 | 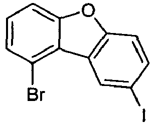 | 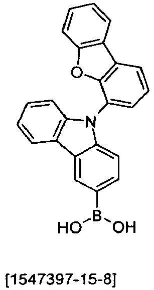 | 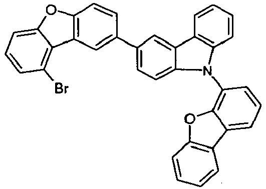 | 62% |
| m3 | 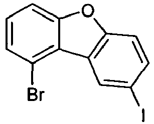 | 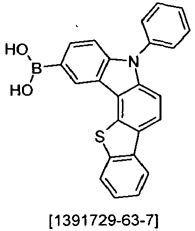 | 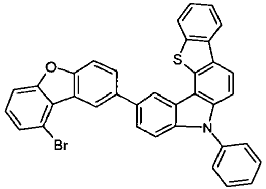 | 54% |
| m4 | 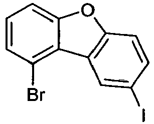 | 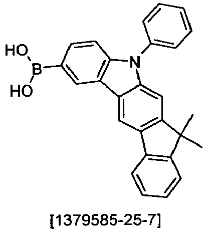 | 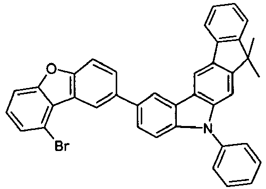 | 50% |
| m5 | 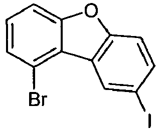 | 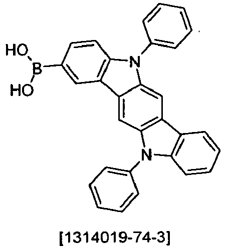 | 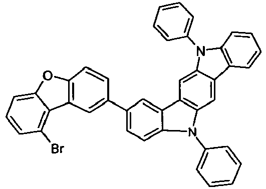 | 55% |
| m6 | 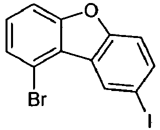 | 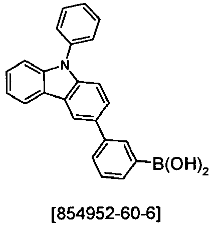 | 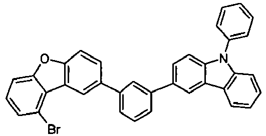 | 56% |
| m7 | 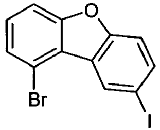 | 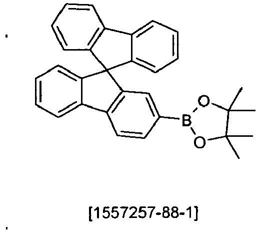 | 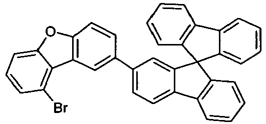 | 57% |
| m8 | 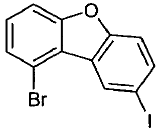 | 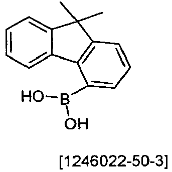 | 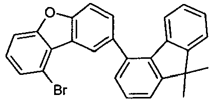 | 61% |
| m9 | 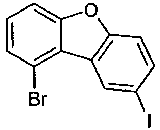 | 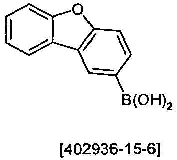 | 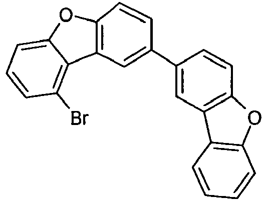 | 52% |
| m10 | 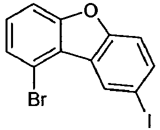 | 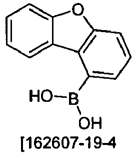 | 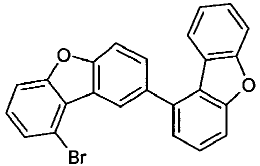 | 50% |
| m11 | 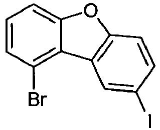 | 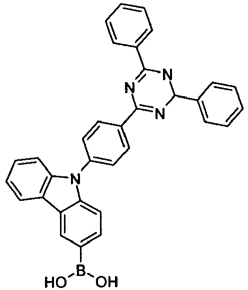 | 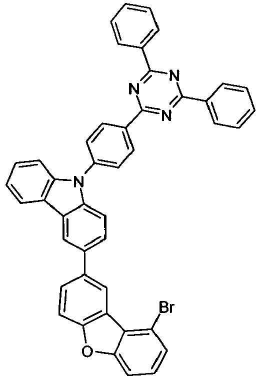 | 48% |
| m12 | 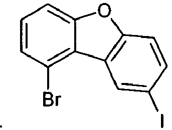 | 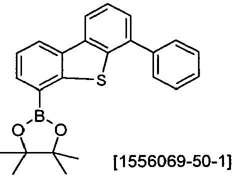 | 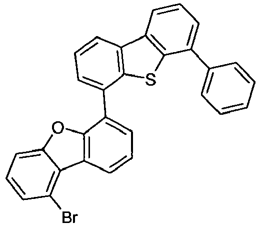 | 52% |
| m13 | 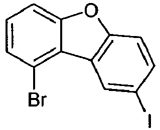 | 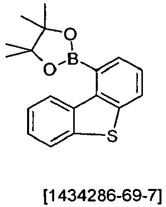 | 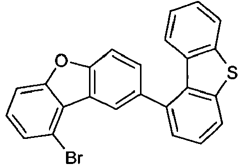 | 54% |
| m14 | 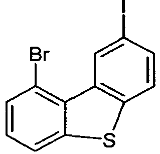 | 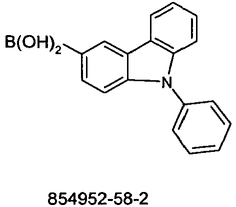 | 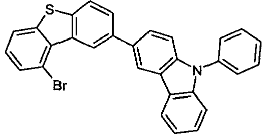 | 57% |
| m15 | 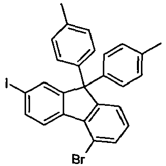 | 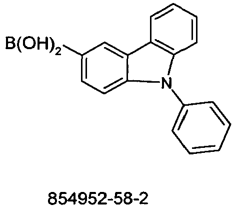 | 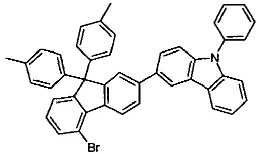 | 48% |
| m16 | 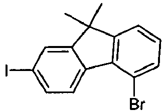 | 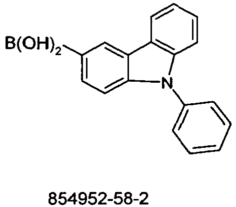 | 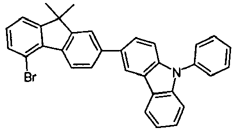 | 46% |

### o) 8-(9-Phenyl-9H-carbazol-3-yl)-dibenzofuran-1-boronsäure

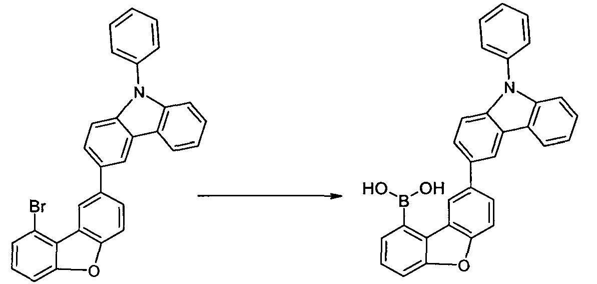

20 g (182 mmol) 3-(9-Brom-dibenzofuran-2-yl)-9-phenyl-9H-carbazol werden in 400 mL trockenem THF gelöst und auf -78 °C gekühlt. Bei dieser Temperatur wird mit 77 mL (190 mmol / 2.5 M in Hexan) n-Butyllithium innerhalb von ca. 5 min. versetzt und anschließend für 2.5 h bei -78 °C nachgerührt. Bei dieser Temperatur wird mit 38 g (365 mmol) Borsäure-trimethylester möglichst zügig versetzt und die Reaktion langsam auf Raumtemperatur kommen gelassen (ca. 18 h). Die Reaktionslösung wird mit Wasser gewaschen und der ausgefallene Feststoff und die organische Phase mit Toluol azeotrop getrocknet. Das Rohprodukt wird mit Toluol/Methylenchlorid bei ca. 40 °C ausgerührt und abgesaugt. Ausbeute: 16.7 g (690 mmol), 90 % der Theorie.

Analog dazu werden die folgenden Verbindungen hergestellt:

| | **Edukt** | **Produkt** | **Ausbeute** |
|---|---|---|---|
| o1 | 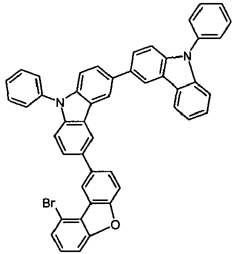 | 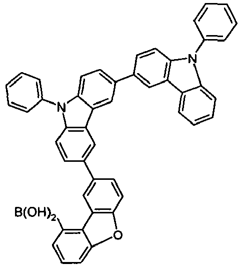 | 81% |
| o2 | 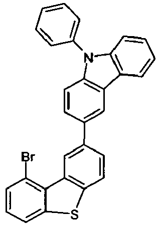 | 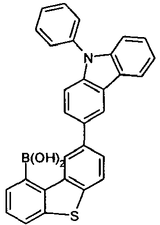 | 84% |
| o3 | 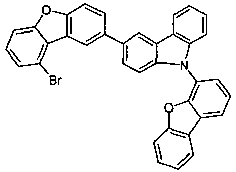 | 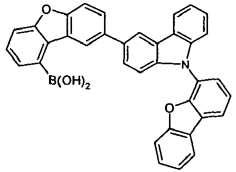 | 82% |
| o4 | 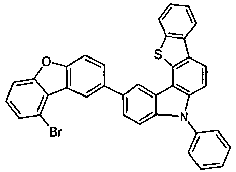 | 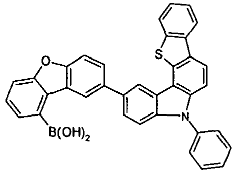 | 81% |
| o5 | 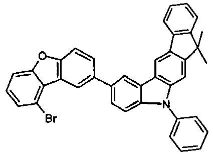 | 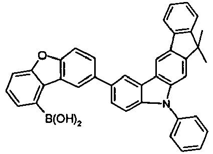 | 79% |
| o6 | 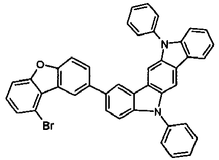 | 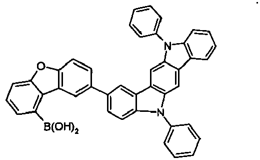 | 77% |
| o7 | 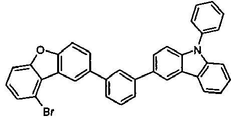 | 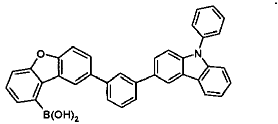 | 75% |
| o8 | 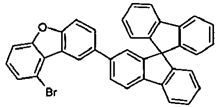 | 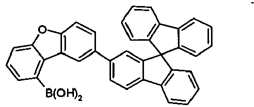 | 78% |
| o9 | 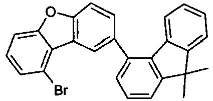 | 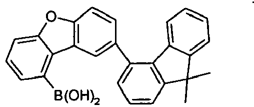 | 76% |
| o10 | 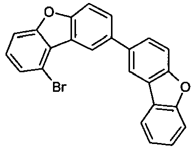 | 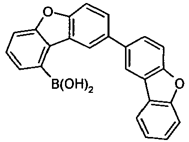 | 81% |
| o11 | 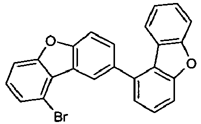 | 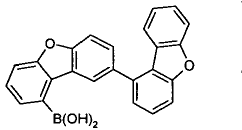 | 80% |
| o12 | 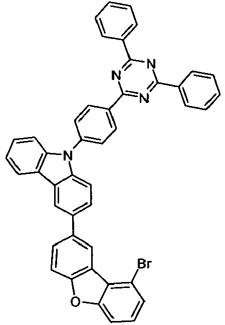 | 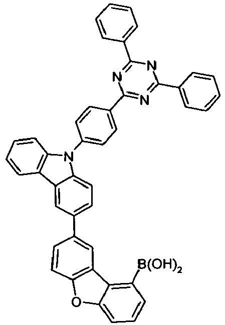 | 71% |
| o13 | 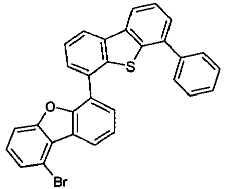 | 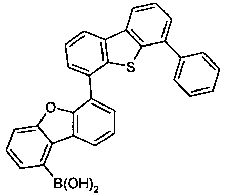 | 69% |
| o14 | 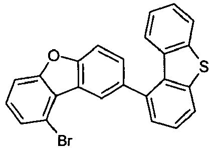 | 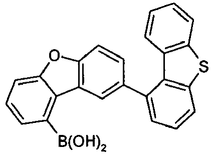 | 88% |
| o15 | 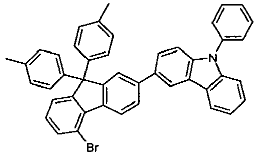 | 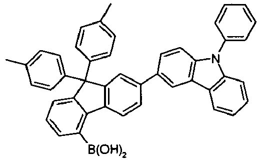 | 78% |
| o16 | 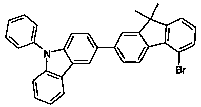 | 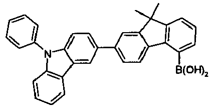 | 77% |

### p) 3-{9-[3-(4,6-Diphenyl-(1,3,5]triazin-2-yl)-phenyl]-dibenzofuran-2-yl-9-phenyl-9H-carbazol

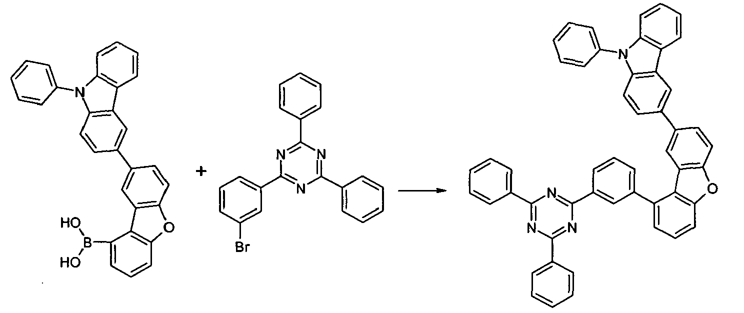

49.8 g (110.0 mmol) 8-(9-Phenyl-9H-carbazol-3-yl)-dibenzofuran-1-boronsäure, 42.6 g (110.0 mmol) 2-(3-Brom-phenyl)-4,6-diphenyl-[1,3,5]triazin und 26 g (210.0 mmol) Natriumcarbonat werden in 500 mL Ethylenglycoldimethylether und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Das Produkt wird via Säulenchromatographie an Kieselgel mit Toluol/Heptan (1:2) gereinigt und abschließend im Hochvakuum (p = 5 x 10⁻⁷ mbar) sublimiert (Reinheit 99,9%). Die Ausbeute beträgt 52 g (72 mmol), entsprechend 78 % der Theorie.

Analog dazu werden die folgenden Verbindungen hergestellt:

| | **Edukt 1** | **Edukt 2** | **Produkt** | **Ausbeute** |
|---|---|---|---|---|
| p1 | 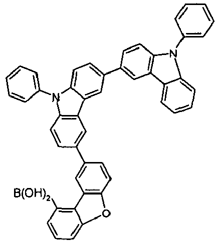 | 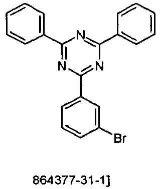 | 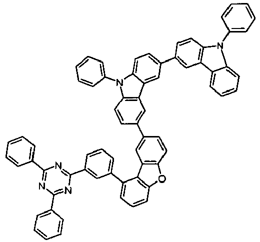 | 59% |
| p2 | 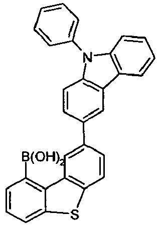 | 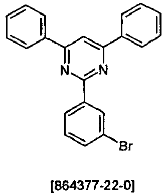 | 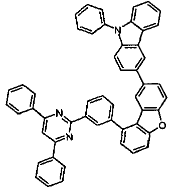 | 67% |
| p3 | 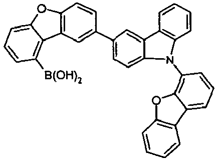 | 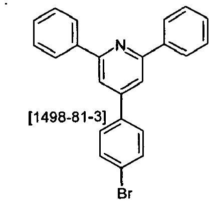 | 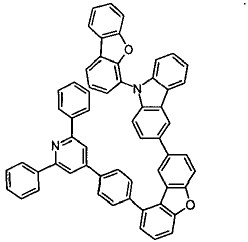 | 62% |
| p4 | 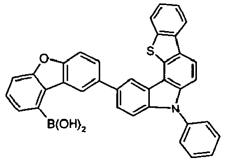 | 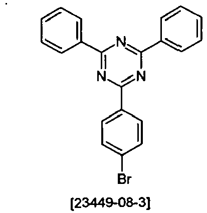 | 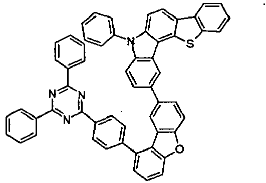 | 69% |
| p5 | 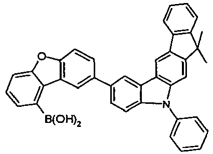 | 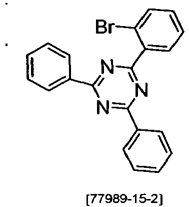 | 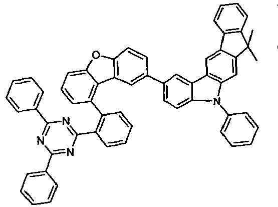 | 67% |
| p6 | 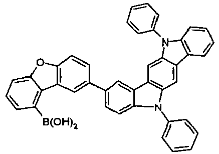 | 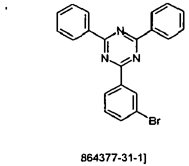 | 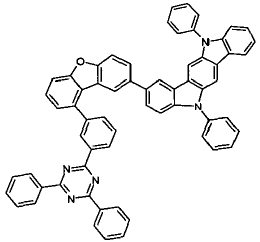 | 68% |
| p7 | 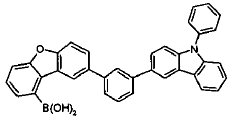 | 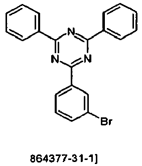 | 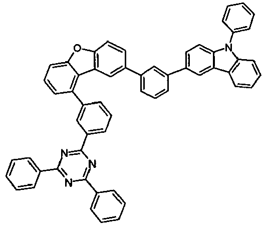 | 69% |
| p8 | 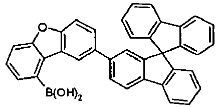 | 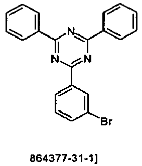 | 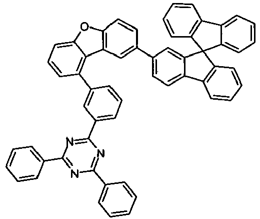 | 60% |
| p9 | 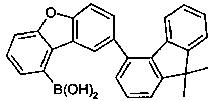 | 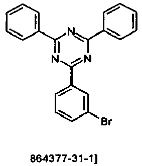 | 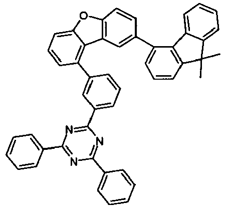 | 63% |
| p10 | 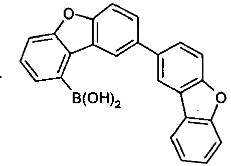 | 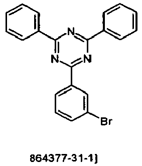 | 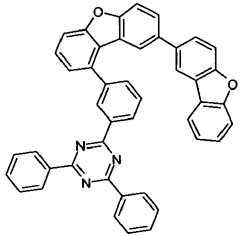 | 66% |
| p11 | 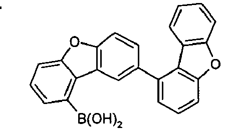 | 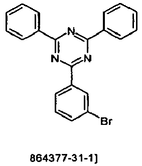 | 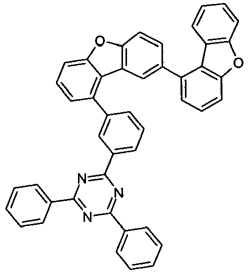 | 60% |
| p12 | 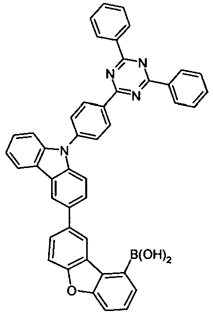 | 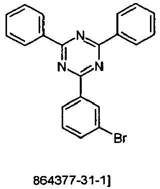 | 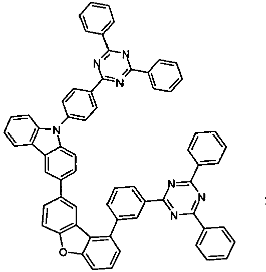 | 61% |
| p13 | 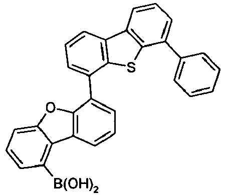 | 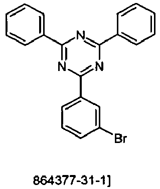 | 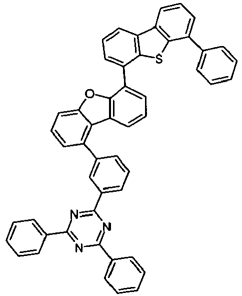 | 65% |
| p14 | 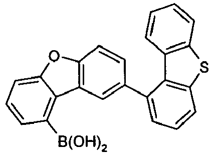 | 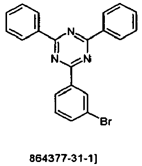 | 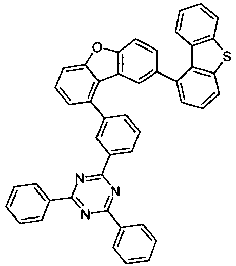 | 63% |
| p15 | 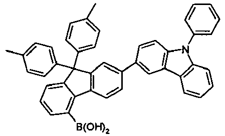 | 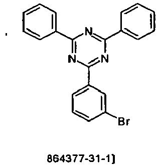 | 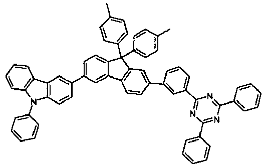 | 65% |
| p16 | 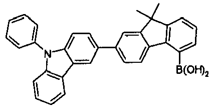 | 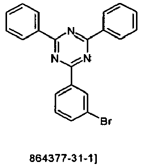 | 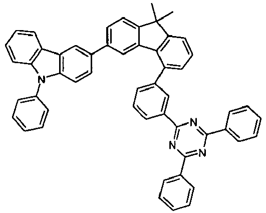 | 63% |
| p17 | 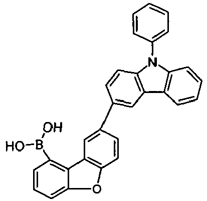 | 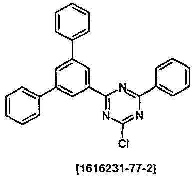 | 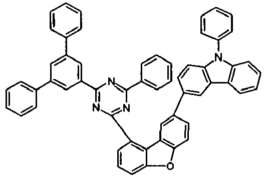 | 65% |
| p18 | 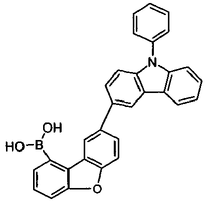 | 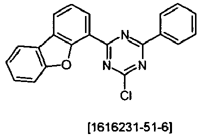 | 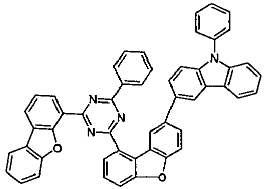 | 67% |
| p19 | 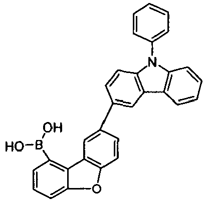 | 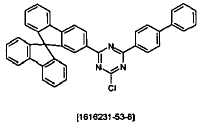 | 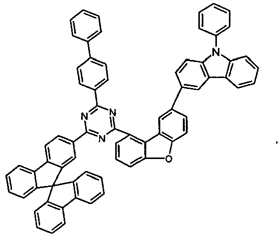 | 72% |
| p20 | 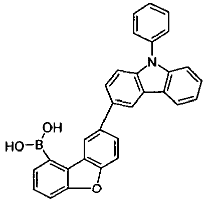 | 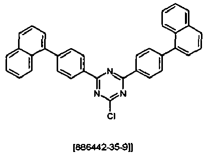 | 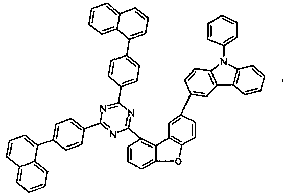 | 66% |

### Herstellung der OLEDs

In den folgenden Beispielen V1 bis E37 (siehe Tabellen 1 und 2) werden die Daten verschiedener OLEDs vorgestellt.

**Vorbehandlung für die Beispiele V1-E37:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen) poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC3:TEG1 (55%:35%:10%) bedeutet hierbei, dass das Material IC1 in einem Volumenanteil von 55%, IC3 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/m² und L1 = 70% in Tabelle 2 bedeutet, dass die in Spalte LD angegebene Lebensdauer der Zeit entspricht, nach der die Anfangsleuchtdichte von 4000 cd/m² auf 2800 cd/m² absinkt. Analog bedeutet L0;j0 = 20mA/cm², L1 = 80%, dass die Leuchtdichte bei Betrieb mit 20mA/cm² nach der Zeit LD auf 80% ihres Anfangswertes absinkt.

Die Daten der verschiedenen OLEDs sind in Tabelle 2 zusammengefasst. Die Beispiele V1- V6 sind Vergleichsbeispiele gemäß dem Stand der Technik, die Beispiele E1-E37 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Mischungen in der Emissionsschicht phosphoreszenter OLEDs

Die erfindungsgemäßen Materialien ergeben bei Einsatz als Matrixmaterialien in phosphoreszierenden OLEDs wesentliche Verbesserungen gegenüber dem Stand der Technik bezüglich der Lebensdauer der Bauteile. Durch Einsatz der erfindungsgemäßen Verbindungen EG1 bis EG4 in Kombination mit dem grün emittierenden Dotanden TEG1 lässt sich eine Steigung der Lebensdauer um über 200% gegenüber dem Stand der Technik beobachten (Vergleich der Beispiele V1 mit E1, E6 und V2 mit E2 sowie V3 mit E3 und V4, V5 mit E4).

**Tabelle 1: Aufbau der OLEDs**

| Bsp | HTL | IL | EBL | EML | HBL | ETL | EIL |
|---|---|---|---|---|---|---|---|
| | Dicke | Dicke | Dicke | Dicke | Dicke | Dicke | Dicke |
| V1 | SpA1 | HATCN | SpMA1 | SdT1:TEG1 | ST2 | ST2:LiQ (50%:50%) | --- |
| | 70nm | 5nm | 90nm | (90%:10%) 30nm | 10nm | 30nm | |
| V2 | SpA1 | HATCN | SpMA1 | SdT2:TEG1 | ST2 | ST2:LiQ (50%:50%) | --- |
| | 70nm | 5nm | 90nm | (90%:10%) 30nm | 10nm | 30nm | |
| V3 | SpA1 | HATCN | SpMA1 | SdT3:TEG1 | ST2 | ST2:LiQ (50%:50%) | --- |
| | 70nm | 5nm | 90nm | (90%:10%) 30nm | 10nm | 30nm | |
| V4 | SpA1 | HATCN | SpMA1 | SdT4:TEG1 | ST2 | ST2:LiQ (50%:50%) | --- |
| | 70nm | 5nm | 90nm | (90%:10%) 30nm | 10nm | 30nm | |
| V5 | SpA1 | HATCN | SpMA1 | SdT5:TEG1 | ST2 | ST2:LiQ (50%:50%) | --- |
| | 70nm | 5nm | 90nm | (90%:10%) 30nm | 10nm | 30nm | |
| V6 | SpA1 | HATCN | SpMA1 | SdT6:TEG1 | ST2 | ST2:LiQ (50%:50%) | --- |
| | 70nm | 5nm | 90nm | (90%:10%) 30nm | 10nm | 30nm | |
| E1 | SpA1 | HATCN | SpMA1 | EG1:TEG1 | ST2 | ST2:LiQ (50%:50%) | --- |
| | 70nm | 5nm | 90nm | (90%:10%) 30nm | 10nm | 30nm | |
| E2 | SpA1 | HATCN | SpMA1 | EG2:TEG1 | ST2 | ST2:LiQ (50%:50%) | --- |
| | 70nm | 5nm | 90nm | (90%:10%) 30nm | 10nm | 30nm | |
| E3 | SpA1 | HATCN | SpMA1 | EG3:TEG1 | ST2 | ST2:LiQ (50%:50%) | --- |
| | 70nm | 5nm | 90nm | (90%:10%) 30nm | 10nm | 30nm | |
| E4 | SpA1 | HATCN | SpMA1 | EG4:TEG1 | ST2 | ST2:LiQ (50%:50%) | --- |
| | 70nm | 5nm | 90nm | (90%:10%) 30nm | 10nm | 30nm | |
| E5 | SpA1 | HATCN | SpMA1 | EG5:TER1 | --- | ST2:LiQ (50%:50%) | --- |
| | 90nm | 5nm | 130nm | (92%:8%) 30nm | | 40nm | |
| E6 | SpA1 | HATCN | SpMA1 | EG6:TER1 | --- | ST2:LiQ (50%:50%) | --- |
| | 90nm | 5nm | 130nm | (92%:8%) 30nm | | 40nm | |
| E7 | SpA1 | HATCN | SpMA1 | EG7:TEG1 | --- | ST2:LiQ (50%:50%) | --- |
| | 70nm | 5nm | 90nm | (90%:10%) 30nm | | 40nm | |
| E8 | SpA1 | HATCN | SpMA1 | EG8:TEG1 | --- | ST2:LiQ (50%:50%) | --- |
| | 70nm | 5nm | 90nm | (90%:10%) 30nm | | 40nm | |
| E9 | SpA1 | HATCN | SpMA1 | EG9:IC3:TEG1 | IC1 | ST2:LiQ (50%:50%) | --- |
| | 70nm | 5nm | 90nm | (45%:45%:10%) 30nm | 10nm | 30nm | |
| E10 | SpA1 | HATCN | SpMA1 | EG10:IC3:TEG1 | IC1 | ST2:LiQ (50%:50%) | --- |
| | 70nm | 5nm | 90nm | (45%:45%:10%) 30nm | 10nm | 30nm | |
| E11 | SpA1 | HATCN | SpMA1 | EG11:IC3:TEG1 | IC1 | ST2:LiQ (50%:50%) | --- |
| | 70nm | 5nm | 90nm | (45%:45%:10%) 30nm | 10nm | 30nm | |
| E12 | SpA1 | HATCN | SpMA1 | IC1:TEG1 | --- | EG12 | LiQ |
| | 70nm | 5nm | 90nm | (90%:10%) 30nm | | 40nm | 3nm |
| E13 | SpA1 | HATCN | SpMA1 | IC1:TEG1 | IC1 | EG13:LiQ | --- |
| | 70nm | 5nm | 90nm | (90%:10%) 30nm | 10nm | (50%:50%) 30nm | |
| E14 | SpA1 | HATCN | SpMA1 | EG14:IC3:TEG1 | IC1 | ST2:LiQ (50%:50%) | --- |
| | 70nm | 5nm | 90nm | (65%:25%:10%) 30nm | 10nm | 30nm | |
| E15 | SpA1 | HATCN | SpMA1 | IC1:TEG1 | EG15 | ST2:LiQ (50%:50%) | --- |
| | 70nm | 5nm | 90nm | (90%:10%) 30nm | 10nm | 30nm | |
| E16 | HATCN | SpMA1 | SpMA2 | EG16:L1:TEY1 | --- | ST1 | LiQ |
| | 5nm | 70nm | 15nm | (45%:45%:10%) 25nm | | 45nm | 3nm |
| E17 | SpA1 | HATCN | SpMA1 | EG17:TEG1 | ST2 | ST2:LiQ (50%:50%) | --- |
| | 70nm | 5nm | 90nm | (90%:10%) 30nm | 10nm | 30nm | |
| E18 | SpA1 | HATCN | SpMA1 | EG18:IC3:TEG1 | IC1 | ST2:LiQ (50%:50%) | --- |
| | 70nm | 5nm | 90nm | (45%:45%:10%) 30nm | 10nm | 30nm | |
| E19 | HATCN | SMA1 | SpMA2 | EG19:L1:TEY1 | --- | ST1 | LiQ |
| | 5nm | 70nm | 15nm | (45%:45%:10%) 25nm | | 45nm | 3nm |
| E20 | SpA1 | HATCN | SpMA1 | IC1:TEG1 | EG20 | ST2:LiQ (50%:50%) | --- |
| | 70nm | 5nm | 90nm | (90%:10%) 30nm | 10nm | 30nm | |
| E21 | SpA1 | HATCN | SpMA1 | EG21:TEG1 | --- | ST2:LiQ (50%:50%) | --- |
| | 70nm | 5nm | 90nm | (90%:10%) 30nm | | 40nm | |
| E22 | SpA1 | HATCN | SpMA1 | EG22:TEG1 | --- | ST2:LiQ (50%:50%) | --- |
| | 70nm | 5nm | 90nm | (90%:10%) 30nm | | 30nm | |
| E23 | SpA1 | HATCN | SpMA1 | IC1:TEG1 | --- | EG23:ST1 | LiQ |
| | 70nm | 5nm | 90nm | (90%:10%) 30nm | | 40nm | 3nm |
| E24 | SpA1 | HATCN | SpMA1 | EG24:TEG1 | --- | ST2:LiQ (50%:50%) | --- |
| | 70nm | 5nm | 90nm | (90%:10%) 30nm | | 30nm | |
| E25 | SpA1 | HATCN | SpMA1 | EG25:IC3:TEG1 | ST2 | ST2:LiQ (50%:50%) | --- |
| | 70nm | 5nm | 90nm | (50%:40%:10%) 30nm | 10nm | 30nm | |
| E26 | SpA1 | HATCN | SpMA1 | EG26:IC3:TEG1 | ST2 | ST2:LiQ (50%:50%) | --- |
| | 70nm | 5nm | 90nm | (55%:35%:10%) 30nm | 10nm | 30nm | |
| E27 | SpA1 | HATCN | SpMA1 | EG27:IC3:TEG1 | ST2 | ST2:LiQ (50%:50%) | --- |
| | 70nm | 5nm | 90nm | (45%:45%:10%) 30nm | 10nm | 30nm | |
| E28 | SpA1 | HATCN | SpMA1 | IC1:TEG1 | IC1 | EG28:LiQ (50%:50%) | --- |
| | 70nm | 5nm | 90nm | (90%:10%) 30nm | 10nm | 30nm | |
| E29 | SpA1 | HATCN | SpMA1 | EG29:TER1 | --- | ST2:LiQ (50%:50%) | --- |
| | 90nm | 5nm | 130nm | (92%:8%) 30nm | | 40nm | |
| E30 | SpA1 | HATCN | SpMA1 | EG30:IC3:TEG1 | ST2 | ST2:LiQ (50%:50%) | --- |
| | 70nm | 5nm | 90nm | (45%:45%:10%) 30nm | 10nm | 30nm | |
| E31 | SpA1 | HATCN | SpMA1 | EG1:IC3:TEG1 | ST2 | ST2:LiQ (50%:50%) | --- |
| | 70nm | 5nm | 90nm | (50%:40%:10%) 30nm | 10nm | 30nm | |
| E32 | SpA1 | HATCN | SpMA1 | EG1:IC4:TEG1 | ST2 | ST2:LiQ (50%:50%) | --- |
| | 70nm | 5nm | 90nm | (40%:45%:15%) 30nm | 10nm | 30nm | |
| E33 | SpA1 | HATCN | SpMA1 | EG1:IC5:TEG1 | ST2 | ST2:LiQ (50%:50%) | --- |
| | 70nm | 5nm | 90nm | (70%:25%:5%) 30nm | 10nm | 30nm | |
| E34 | SpA1 | HATCN | SpMA1 | EG1:IC6:TEG1 | ST2 | ST2:LiQ (50%:50%) | --- |
| | 70nm | 5nm | 90nm | (45%:45%:10%) 30nm | 10nm | 30nm | |
| E35 | SpA1 | HATCN | SpMA1 | EG1:IC7:TEG1 | ST2 | ST2:LiQ (50%:50%) | --- |
| | 70nm | 5nm | 90nm | (40%:50%:10%) 30nm | 10nm | 30nm | |
| E36 | SpA1 | HATCN | SpMA1 | EG1:IC8:TEG1 | ST2 | ST2:LiQ (50%:50%) | --- |
| | 70nm | 5nm | 90nm | (30%:50%:20%) 30nm | 10nm | 30nm | |
| E37 | SpA1 | HATCN | SpMA1 | EG1:L1:TEG1 | ST2 | ST2:LiQ (50%:50%) | --- |
| | 70nm | 5nm | 90nm | (25%:55%:20%) 30nm | 10nm | 30nm | |

**Tabelle 2: Daten der OLEDs**

| Bsp. | U1000 (V) | SE1000 (cd/A) | LE1000 (lm/W) | EQE 1000 | CIE x/y bei 1000 cd/m² | L₀; j₀ | L1 % | LD (h) |
|---|---|---|---|---|---|---|---|---|
| V1 | 3.6 | 51 | 44 | 13.7% | 0.33/0.63 | 20mA/cm² | 80 | 95 |
| V2 | 4.2 | 50 | 37 | 14.3% | 0.33/0.62 | 20mA/cm² | 80 | 10 |
| V3 | 4.3 | 55 | 40 | 14.7% | 0.33/0.64 | 20mA/cm² | 80 | 15 |
| V4 | 3.5 | 48 | 43 | 12.8% | 0.32/0.64 | 20mA/cm² | 80 | 190 |
| V5 | 3.7 | 59 | 50 | 15.7% | 0.33/0.64 | 20mA/cm² | 80 | 125 |
| V6 | 3.4 | 44 | 41 | 11.8% | 0.31/0.65 | 20mA/cm² | 80 | 20 |
| E1 | 3.5 | 40 | 36 | 11.6% | 0.33/0.62 | 20mA/cm² | 80 | 290 |
| E2 | 4.3 | 51 | 37 | 14.5% | 0.33/0.62 | 20mA/cm² | 80 | 20 |
| E3 | 4.4 | 55 | 39 | 15.0% | 0.33/0.63 | 20mA/cm² | 80 | 35 |
| E4 | 3.6 | 41 | 36 | 11.9% | 0.32/0.63 | 20mA/cm² | 80 | 300 |
| E5 | 4.4 | 13 | 9 | 12.4% | 0.66/0.34 | 4000 cd/m² | 80 | 340 |
| E6 | 4.6 | 11 | 8 | 11.4% | 0.67/0.34 | 4000 cd/m² | 80 | 370 |
| E7 | 3.4 | 59 | 55 | 15.9% | 0.33/0.63 | 20mA/cm² | 80 | 115 |
| E8 | 3.6 | 56 | 49 | 15.2% | 0.33/0.62 | 20mA/cm² | 80 | 125 |
| E9 | 3.4 | 62 | 57 | 16.5% | 0.34/0.63 | 20 mA/cm² | 80 | 240 |
| E10 | 3.5 | 60 | 54 | 16.1% | 0.33/0.63 | 20 mA/cm² | 80 | 350 |
| E11 | 3.6 | 57 | 50 | 15.5% | 0.33/0.63 | 20 mA/cm² | 80 | 290 |
| E12 | 3.3 | 64 | 61 | 17.1% | 0.33/0.63 | 20 mA/cm² | 80 | 125 |
| E13 | 3.7 | 62 | 53 | 16.5% | 0.34/0.63 | 20 mA/cm² | 80 | 165 |
| E14 | 3.3 | 60 | 57 | 16.7% | 0.32/0.63 | 20 mA/cm² | 80 | 270 |
| E15 | 3.5 | 59 | 53 | 16.0% | 0.34/0.63 | 20 mA/cm² | 80 | 145 |
| E16 | 2.9 | 75 | 81 | 22.4% | 0.44/0.55 | 50mA/cm² | 90 | 85 |
| E17 | 3.4 | 41 | 37 | 11.7% | 0.33/0.63 | 20mA/cm² | 80 | 140 |
| E18 | 3.5 | 60 | 53 | 16.3% | 0.33/0.63 | 20 mA/cm² | 80 | 260 |
| E19 | 2.8 | 77 | 86 | 23.1% | 0.45/0.55 | 50mA/cm² | 90 | 100 |
| E20 | 3.7 | 59 | 50 | 15.8% | 0.33/0.63 | 20 mA/cm² | 80 | 155 |
| E21 | 3.7 | 55 | 47 | 14.7% | 0.36/0.61 | 20mA/cm² | 80 | 135 |
| E22 | 3.8 | 58 | 48 | 15.6% | 0.33/0.63 | 20mA/cm² | 80 | 140 |
| E23 | 3.4 | 62 | 57 | 17.0% | 0.31/0.64 | 20mA/cm² | 80 | 130 |
| E24 | 3.8 | 56 | 46 | 15.3% | 0.33/0.63 | 20mA/cm² | 80 | 125 |
| E25 | 3.6 | 60 | 52 | 16.0% | 0.35/0.62 | 20mA/cm² | 80 | 360 |
| E26 | 3.7 | 57 | 48 | 15.2% | 0.33/0.63 | 20mA/cm² | 80 | 275 |
| E27 | 3.6 | 54 | 47 | 15.5% | 0.34/0.61 | 20mA/cm² | 80 | 255 |
| E28 | 3.6 | 60 | 52 | 16.4% | 0.34/0.62 | 20mA/cm² | 80 | 170 |
| E29 | 4.5 | 13 | 9 | 11.6% | 0.67/0.33 | 4000cd/m² | 80 | 340 |
| E30 | 3.5 | 58 | 52 | 15.6% | 0.33/0.63 | 20mA/cm² | 80 | 270 |
| E31 | 3.4 | 59 | 55 | 15.9% | 0.32/0.64 | 20mA/cm² | 80 | 380 |
| E32 | 3.4 | 61 | 56 | 16.2% | 0.33/0.64 | 20mA/cm² | 80 | 360 |
| E33 | 3.3 | 59 | 56 | 15.7% | 0.33/0.63 | 20mA/cm² | 80 | 335 |
| E34 | 3.5 | 61 | 55 | 16.3% | 0.33/0.63 | 20mA/cm² | 80 | 355 |
| E35 | 3.4 | 62 | 57 | 16.6% | 0.31/0.64 | 20mA/cm² | 80 | 340 |
| E36 | 3.4 | 59 | 55 | 16.1% | 0.33/0.63 | 20mA/cm² | 80 | 345 |
| E37 | 3.3 | 54 | 51 | 15.0% | 0.32/0.63 | 20mA/cm² | 80 | 395 |

**Tabelle 3: Strukturformeln der Materialien für die OLEDs**

| | |
|---|---|
| 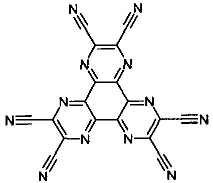 | 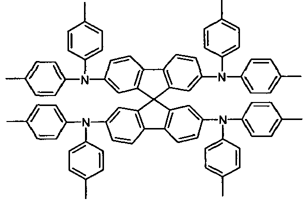 |
| HATCN | SpA1 |
| 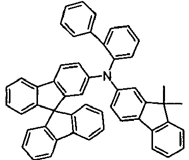 | 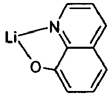 |
| SpMA1 | LiQ |
| 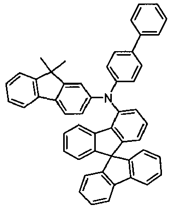 | 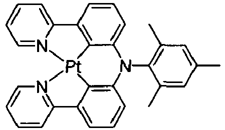 |
| SpMA2 | TER1 |
| 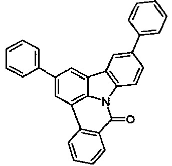 | 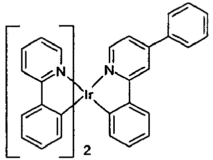 |
| L1 | TEY1 |
| 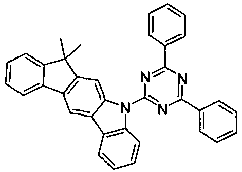 | 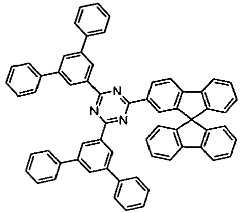 |
| IC1 | ST2 |
| 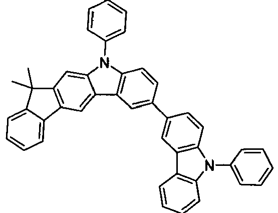 | 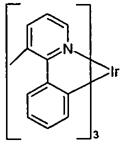 |
| IC3 | TEG1 |
| 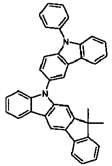 | 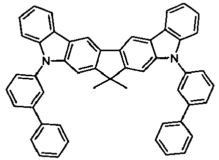 |
| IC4 | IC5 |
| 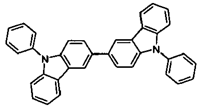 | 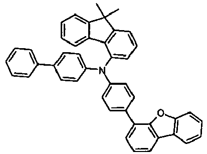 |
| IC6 | IC7 |
| 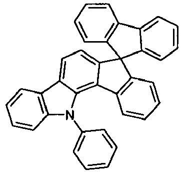 | 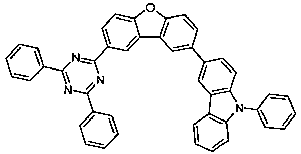 |
| IC8 | SdT1 |
| 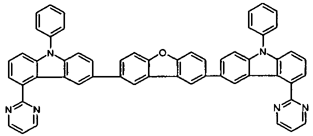 | 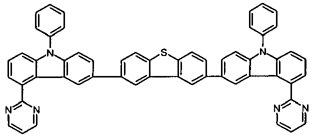 |
| SdT2 | SdT3 |
| 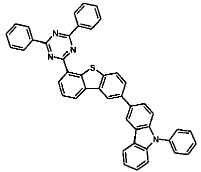 | 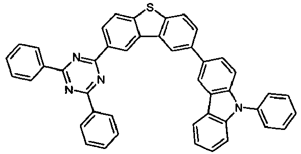 |
| SdT4 | SdT5 |
| 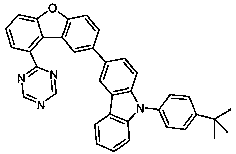 | |
| SdT6 | |
| 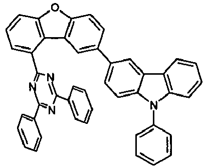 | 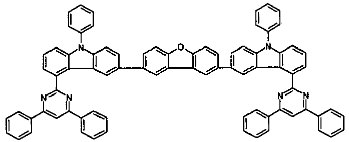 |
| EG1 | EG2 |
| 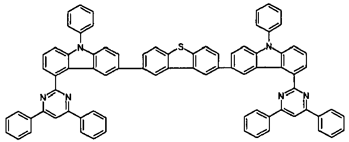 | 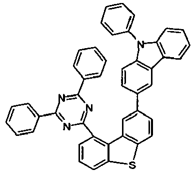 |
| EG3 | EG4 |
| 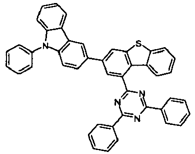 | 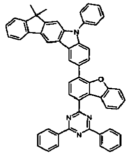 |
| EG5 | EG6 |
| 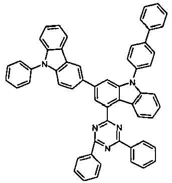 | 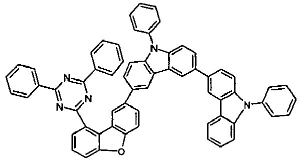 |
| EG7 | EG8 |
| 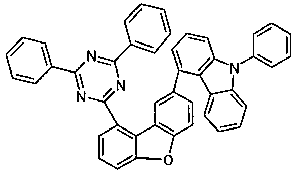 | 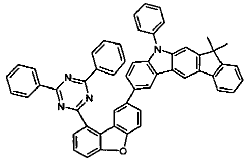 |
| EG9 | EG10 |
| 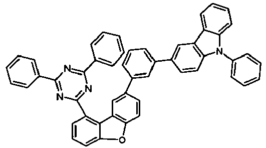 | 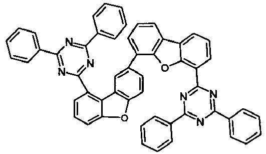 |
| EG11 | E12 |
| 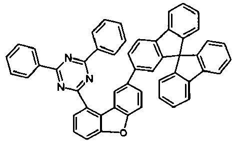 | 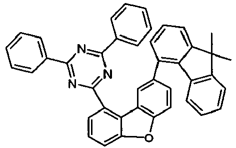 |
| EG13 | EG14 |
| 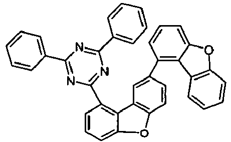 | 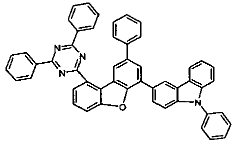 |
| EG15 | EG16 |
| 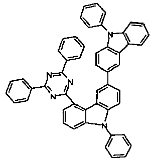 | 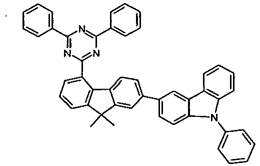 |
| EG17 | EG18 |
| 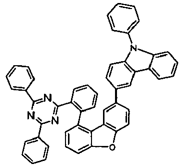 | 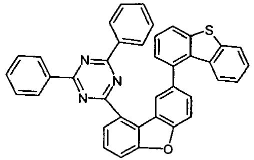 |
| EG19 | EG20 |
| 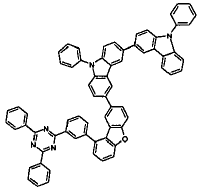 | 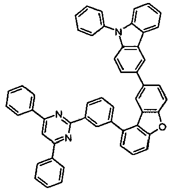 |
| EG21 | EG22 |
| 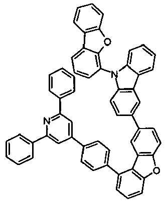 | 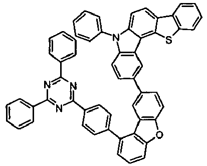 |
| EG23 | EG24 |
| 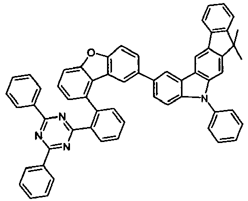 | 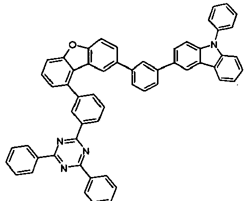 |
| EG25 | EG26 |
| 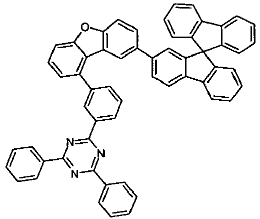 | 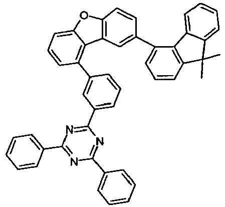 |
| EG27 | EG28 |
| 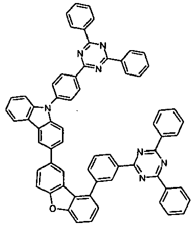 | 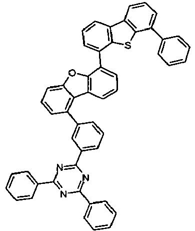 |
| EG29 | EG30 |

## Patentansprüche

1. Verbindung gemäß Formel (1) oder (2), 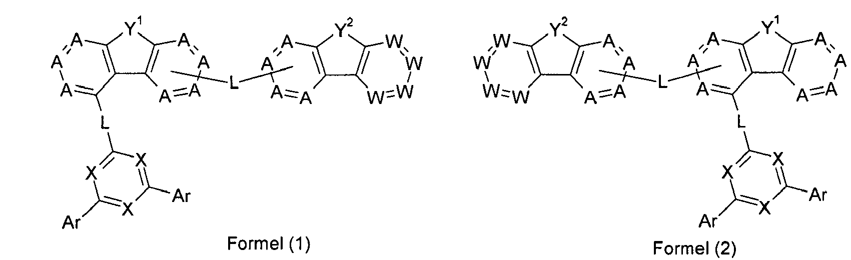 wobei für die verwendeten Symbole und Indizes gilt:
A ist bei jedem Auftreten gleich oder verschieden CR oder N, wobei maximal zwei Gruppen A pro Cyclus für N stehen und wobei A für C steht, wenn an diese Position eine Gruppe L gebunden ist;
W ist bei jedem Auftreten gleich oder verschieden CR oder N, wobei maximal zwei Gruppen W für N stehen, oder zwei benachbarte Gruppen W stehen zusammen für eine Gruppe der folgenden Formel (3), wobei die Verbindung der Formel (1) bzw. Formel (2) maximal eine Gruppe der Formel (3) aufweist, 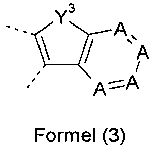 wobei die gestrichelten Bindungen die Verknüpfung dieser Gruppe andeuten und A die oben genannten Bedeutungen aufweist; mit der Maßgabe, dass die Verbindung der Formel (2) keine Gruppe der Formel (3) aufweist, wenn Y¹ für CR₂ steht;
X ist bei jedem Auftreten gleich oder verschieden CR oder N mit der Maßgabe, dass mindestens eine Gruppe X für N steht;
Ar ist bei jedem Auftreten gleich oder verschieden ein aromatisches oder heteroaromatisches Ringsystem mit 5 bis 30 aromatischen Ringatomen, welches durch einen oder mehrere Reste R substituiert sein kann;
Y¹, Y², Y³ sind bei jedem Auftreten gleich oder verschieden O, NR, S oder CR₂, wobei der Rest R, der an N gebunden ist, ungleich H ist;
L ist bei jedem Auftreten gleich oder verschieden eine Einfachbindung oder ein aromatisches oder heteroaromatisches Ringsystem mit 5 bis 30 aromatischen Ringatomen, das durch einen oder mehrere Reste R substituiert sein kann;
R ist bei jedem Auftreten gleich oder verschieden ausgewählt aus der Gruppe bestehend aus H, D, F, Cl, Br, I, CN, NO₂, N(Ar¹)₂, N(R¹)₂, C(=O)Ar¹, C(=O)R¹, P(=O)(Ar¹)₂, P(Ar¹)₂, B(Ar¹)₂, Si(Ar¹)₃, Si(R¹)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R¹ substituiert sein kann, wobei eine oder mehrere nichtbenachbarte CH₂-Gruppen durch R¹C=CR¹, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R¹ substituiert sein kann, einer Aryloxy- oder Heteroaryloxygruppe mit 5 bis 40 aromatischen Ringatomen, die mit einem oder mehreren Resten R¹ substituiert sein kann, oder einer Aralkyl- oder Heteroaralkylgruppe mit 5 bis 40 aromatischen Ringatomen, die mit einem oder mehreren Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann;
Ar¹ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können zwei Reste Ar¹, welche an dasselbe N-Atom, P-Atom oder B-Atom binden, auch durch eine Einfachbindung oder eine Brücke, ausgewählt aus N(R¹), C(R¹)₂, O oder S, miteinander verbrückt sein;
R¹ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten R¹ miteinander ein mono- oder polycyclisches, aliphatisches Ringsystem bilden;
dabei sind die folgenden Verbindungen von der Erfindung ausgenommen:

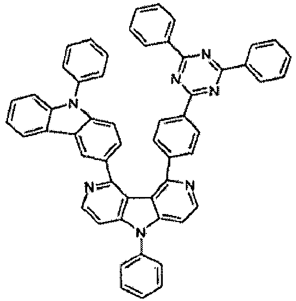

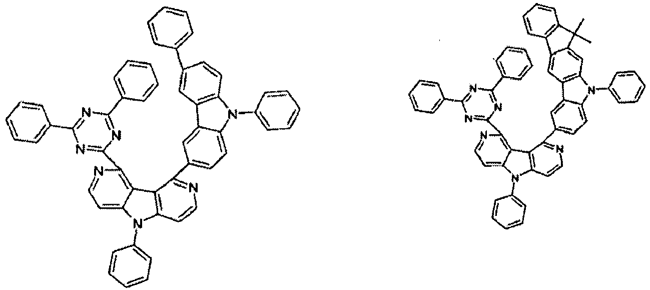

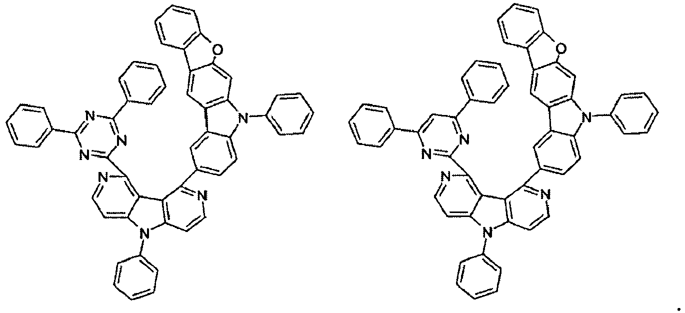

2. Verbindung nach Anspruch 1, **dadurch gekennzeichnet, dass** W gleich oder verschieden bei jedem Auftreten für CR steht und dass A gleich oder verschieden bei jedem Auftreten für CR steht, ausgewählt aus den Verbindungen der Formeln (1a), (1b), (2a) bzw. (2b), 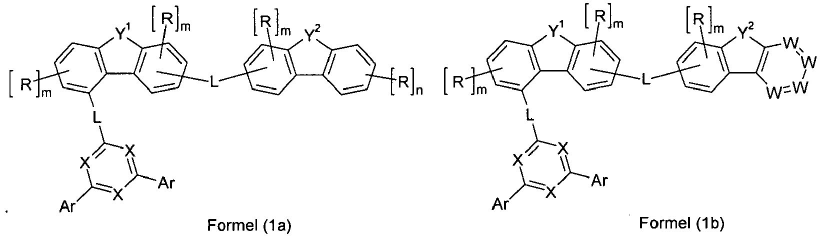 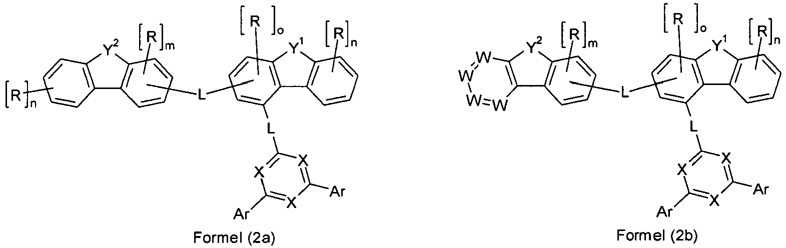 wobei gilt:
zwei benachbarte Gruppen W stehen zusammen für eine Gruppe der folgenden Formel (3a) und die anderen beiden Gruppen W stehen für CR, 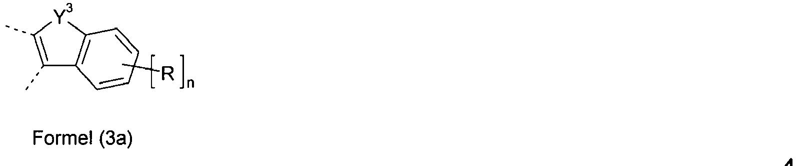 wobei die gestrichelten Bindungen die Verknüpfung dieser Gruppe andeuten;
n ist bei jedem Auftreten gleich oder verschieden 0, 1, 2, 3 oder 4;
m ist bei jedem Auftreten gleich oder verschieden 0, 1, 2 oder 3;
o ist 0, 1 oder 2;
die weiteren verwendeten Symbole weisen die in Anspruch 1 genannten Bedeutungen auf;
wobei Verbindungen der Formel (2b), in denen Y¹ für CR₂ steht, von der Erfindung ausgenommen sind.

3. Verbindung nach Anspruch 1 oder 2, **dadurch gekennzeichnet, dass** mindestens zwei Gruppen X für N stehen.

4. Verbindung nach einem oder mehreren der Ansprüche 1 bis 3, **dadurch gekennzeichnet, dass** Y¹ und Y² gleich oder verschieden bei jedem Auftreten für O, NR, wobei der an den Stickstoff gebundene Rest R ungleich H ist, oder S stehen.

5. Verbindung nach einem oder mehreren der Ansprüche 1 bis 4, für die gilt:
X ist gleich oder verschieden bei jedem Auftreten CR oder N, wobei mindestens zwei Gruppen X für N stehen;
Y¹, Y² stehen gleich oder verschieden bei jedem Auftreten für O, NR, wobei der an den Stickstoff gebundene Rest R ungleich H ist, oder S;
Y³ steht für O, NR, wobei der an den Stickstoff gebundene Rest R ungleich H ist, oder CR₂;
L steht gleich oder verschieden bei jedem Auftreten für eine Einfachbindung oder ein aromatisches oder heteroaromatisches Ringsystem mit 5 bis 24 aromatischen Ringatomen, das durch einen oder mehrere Reste R substituiert sein kann;
Ar steht gleich oder verschieden bei jedem Auftreten für ein aromatisches oder heteroaromatisches Ringsystem mit 6 bis 24 aromatischen Ringatomen, das durch einen oder mehrere Reste R substituiert sein kann;
n ist in Formel (1a) bzw. (2a) gleich oder verschieden bei jedem Auftreten 0, 1, 2 oder 3;
m ist in Formel (1a) bzw. (2a) gleich oder verschieden bei jedem Auftreten 0, 1 oder 2;
o ist in Formel (1a) bzw. (2a) 0 oder 1;
R ist gleich oder verschieden bei jedem Auftreten gewählt aus der Gruppe bestehend aus H, D, F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R¹ substituiert sein kann, oder einer Aralkyl- oder Heteroaralkylgruppe mit 5 bis 25 aromatischen Ringatomen, die mit einem oder mehreren Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann.

6. Verbindung nach einem oder mehreren der Ansprüche 1 bis 5, für die gilt:
X ist N;
Y¹, Y² stehen gleich oder verschieden bei jedem Auftreten für O oder NR, wobei der an den Stickstoff gebundene Rest R ungleich H ist;
Y³ steht für NR, wobei der an den Stickstoff gebundene Rest R ungleich H ist, oder CR₂;
L steht für eine Einfachbindung;
Ar steht gleich oder verschieden bei jedem Auftreten für ein aromatisches oder heteroaromatisches Ringsystem mit 6 bis 18 aromatischen Ringatomen, das jeweils durch einen oder mehrere Reste R substituiert sein kann;
n ist in Formel (1a) bzw. (2a) bei jedem Auftreten gleich oder verschieden 0 oder 1;
m ist in Formel (1a) bzw. (2a) 0;
o ist in Formel (1a) bzw. (2a) 0;
R ist gleich oder verschieden bei jedem Auftreten ausgewählt aus der Gruppe bestehend aus H, D, F, CN, N(Ar¹)₂, einer geradkettigen Alkylgruppe mit 1 bis 8 C-Atomen, oder einer verzweigten oder cyclischen Alkylgruppe mit 3 bis 8 C-Atomen, oder einer Alkenylgruppe mit 2 bis 8 C-Atomen, die jeweils mit einem oder mehreren Resten R¹ substituiert sein kann, oder einem aromatischen oder heteroaromatischen Ringsystem mit 6 bis 24 aromatischen Ringatomen, das jeweils mit einem oder mehreren nicht-aromatischen Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R¹ substituiert sein kann.

7. Verbindung nach einem oder mehreren der Ansprüche 1 bis 6, **dadurch gekennzeichnet, dass** die Verbindung ausgewählt ist aus den Formeln (6), (7), (8), (9) und (10), 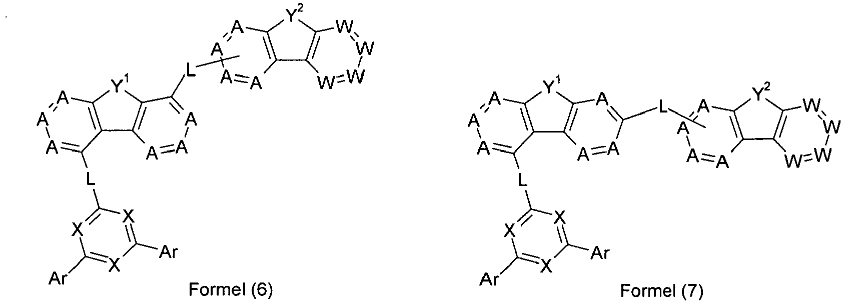 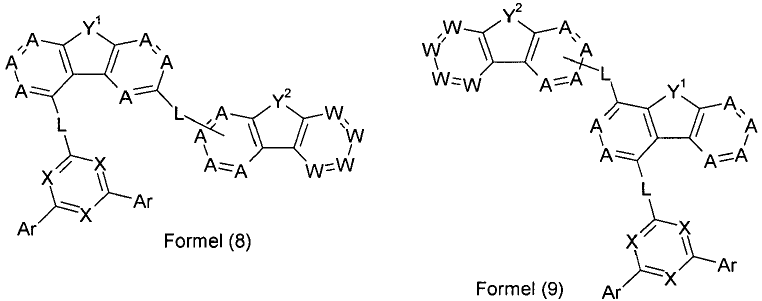 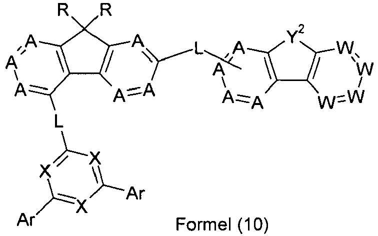 wobei Y¹ für O, NR oder S steht, die weiteren verwendeten Symbole die in Anspruch 1 genannten Bedeutungen aufweisen und A und W bevorzugt gleich oder verschieden bei jedem Auftreten für CR stehen.

8. Verbindung nach Anspruch 7, gewählt aus den Verbindungen der Formeln (8a) und (8b), 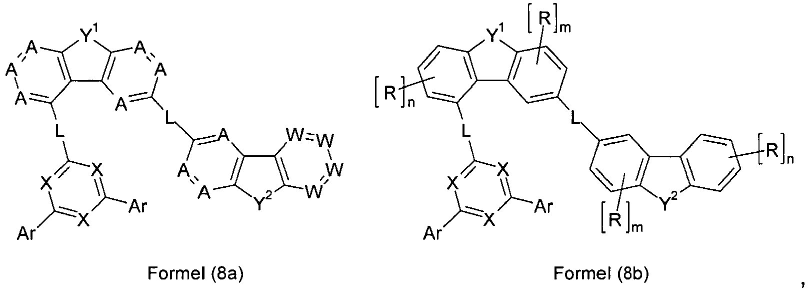 wobei Y¹ und Y² gleich oder verschieden bei jedem Auftreten für O, NR oder S stehen und die weiteren verwendeten Symbole und Indizes die in Anspruch 1 genannten Bedeutungen aufweisen.

9. Verbindung nach Anspruch 8 gemäß Formel (8c), 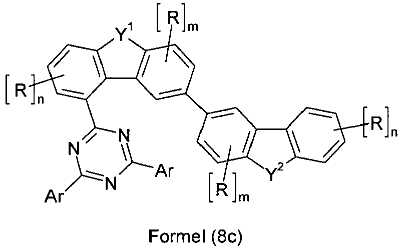 wobei für die verwendeten Symbole und Indizes gilt:
Y¹, Y² stehen gleich oder verschieden bei jedem Auftreten für O, NR, wobei der an den Stickstoff gebundene Rest R ungleich H ist, oder S, mit der Maßgabe, dass mindestens eine der Gruppen Y¹ und/oder Y² für NR steht;
Ar steht gleich oder verschieden bei jedem Auftreten für ein aromatisches oder heteroaromatisches Ringsystem mit 6 bis 24 aromatischen Ringatomen, das jeweils durch einen oder mehrere Reste R substituiert sein kann;
n ist gleich oder verschieden bei jedem Auftreten 0, 1, 2 oder 3;
m ist gleich oder verschieden bei jedem Auftreten 0, 1 oder 2;
o ist 0 oder 1;
R ist gleich oder verschieden bei jedem Auftreten gewählt aus der Gruppe bestehend aus H, D, F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R¹ substituiert sein kann, oder einer Aralkyl- oder Heteroaralkylgruppe mit 5 bis 25 aromatischen Ringatomen, die mit einem oder mehreren Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann.

10. Verbindung nach Anspruch 9 gemäß Formel (8d), 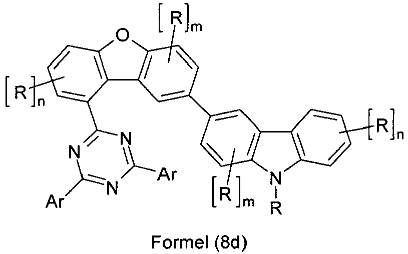 wobei für die verwendeten Symbole und Indizes gilt:
n ist gleich oder verschieden bei jedem Auftreten 0, 1 oder 2;
m ist gleich oder verschieden bei jedem Auftreten 0 oder 1; der an den Stickstoff gebundene Rest R steht für ein aromatisches oder heteroaromatisches Ringsystem mit 5 bis 24 aromatischen Ringatomen steht, das jeweils mit einem oder mehreren Resten R¹ substituiert sein kann;
die weiteren verwendeten Symbole und Indizes weisen die unter Anspruch 9 genannten Bedeutungen auf.

11. Mischung enthaltend mindestens eine Verbindung nach einem oder mehreren der Ansprüche 1 bis 10 und mindestens eine weitere Verbindung und/oder mindestens eine Lösemittel.

12. Verwendung einer Verbindung nach einem oder mehreren der Ansprüche 1 bis 10 oder einer Mischung nach Anspruch 11 in einer elektronischen Vorrichtung.

13. Elektronische Vorrichtung enthaltend mindestens eine Verbindung nach einem oder mehreren der Ansprüche 1 bis 10 oder einer Mischung nach Anspruch 11.

14. Elektronische Vorrichtung nach Anspruch 13, **dadurch gekennzeichnet, dass** es sich um einer organische Elektrolumineszenzvorrichtung handelt.

15. Elektronische Vorrichtung nach Anspruch 14, **dadurch gekennzeichnet, dass** die Verbindung nach einem oder mehreren der Ansprüche 1 bis 10 in einer emittierenden Schicht, bevorzugt in Kombination mit einem phosphoreszierenden Dotanden und gegebenenfalls einem oder mehreren weiteren Matrixmaterialien, oder in einer Lochblockierschicht oder in einer Elektronentransportschicht eingesetzt wird.

## Claims

1. Compound of the formula (1) or (2), 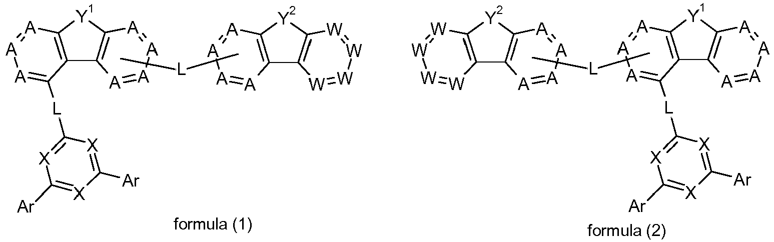 where the following applies for the symbols and indices used:
A is on each occurrence, identically or differently, CR or N, where a maximum of two groups A per ring stand for N and where A stands for C if a group L is bonded at this position;
W is on each occurrence, identically or differently, CR or N, where a maximum of two groups W stand for N, or two adjacent groups W together stand for a group of the following formula (3), where the compound of the formula (1) or formula (2) contains a maximum of one group of the formula (3), 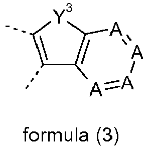 where the dashed bonds indicate the linking of this group and A has the meanings given above; with the proviso that the compound of the formula (2) does not contain a group of the formula (3) if Y¹ stands for CR₂;
X is on each occurrence, identically or differently, CR or N, with the proviso that at least one group X stands for N;
Ar is on each occurrence, identically or differently, an aromatic or heteroaromatic ring system having 5 to 30 aromatic ring atoms, which may be substituted by one or more radicals R;
Y¹, Y², Y³ are on each occurrence, identically or differently, O, NR, S or CR2, where the radical R that is bonded to N is not equal to H;
L is on each occurrence, identically or differently, a single bond or an aromatic or heteroaromatic ring system having 5 to 30 aromatic ring atoms, which may be substituted by one or more radicals R;
R is selected on each occurrence, identically or differently, from the group consisting of H, D, F, Cl, Br, I, CN, NO₂, N(Ar¹)₂, N(R¹)₂, C(=O)Ar¹, C(=O)R¹, P(=O)(Ar¹)₂, P(Ar¹)₂, B(Ar¹)₂, Si(Ar¹)₃, Si(R¹)₃, a straight-chain alkyl, alkoxy or thioalkyl group having 1 to 20 C atoms or a branched or cyclic alkyl, alkoxy or thioalkyl group having 3 to 20 C atoms or an alkenyl group having 2 to 20 C atoms, which may in each case be substituted by one or more radicals R¹, where one or more non-adjacent CH₂ groups may be replaced by R¹C=CR¹, Si(R¹)₂, C=O, C=S, C=NR¹, P(=O)(R¹), SO, SO₂, NR¹, O, S or CONR¹ and where one or more H atoms may be replaced by D, F, Cl, Br, I, CN or NO₂, an aromatic or heteroaromatic ring system having 5 to 40 aromatic ring atoms, which may in each case be substituted by one or more radicals R¹, an aryloxy or heteroaryloxy group having 5 to 40 aromatic ring atoms, which may be substituted by one or more radicals R¹, or an aralkyl or heteroaralkyl group having 5 to 40 aromatic ring atoms, which may be substituted by one or more radicals R¹; two substituents R that are bonded to the same carbon atom or to adjacent carbon atoms may optionally form a monocyclic or polycyclic, aliphatic, aromatic or heteroaromatic ring system, which may be substituted by one or more radicals R¹;
Ar¹ is on each occurrence, identically or differently, an aromatic or heteroaromatic ring system having 5 to 30 aromatic ring atoms, which may be substituted by one or more non-aromatic radicals R¹; two radicals Ar¹ that are bonded to the same N atom, P atom or B atom may also be bridged to one another by a single bond or a bridge selected from N(R¹), C(R¹)₂, O or S;
R¹ is selected on each occurrence, identically or differently, from the group consisting of H, D, F, CN, an aliphatic hydrocarbon radical having 1 to 20 C atoms or an aromatic or heteroaromatic ring system having 5 to 30 aromatic ring atoms, in which one or more H atoms may be replaced by D, F, Cl, Br, I or CN and which may be substituted by one or more alkyl groups, each having 1 to 4 carbon atoms; two or more adjacent substituents R¹ may form a mono- or polycyclic, aliphatic ring system with one another;
the following compounds are excluded from the invention:

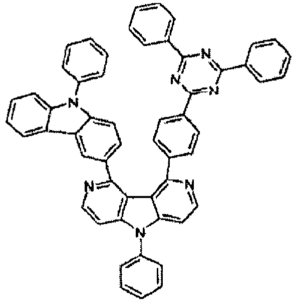

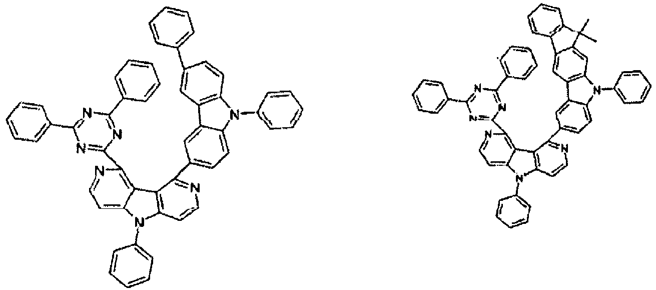

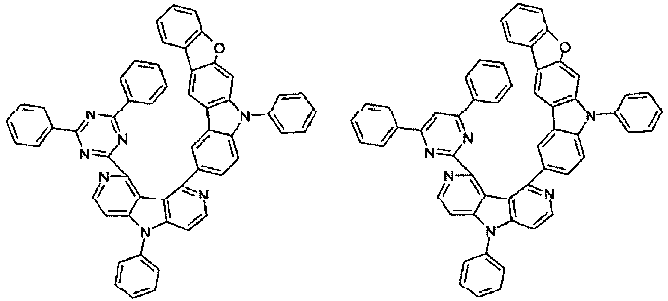

2. Compound according to Claim 1, **characterised in that** W stands, identically or differently on each occurrence, for CR and **in that** A stands, identically or differently on each occurrence, for CR, selected from the compounds of the formulae (1a), (1b), (2a) and (2b), 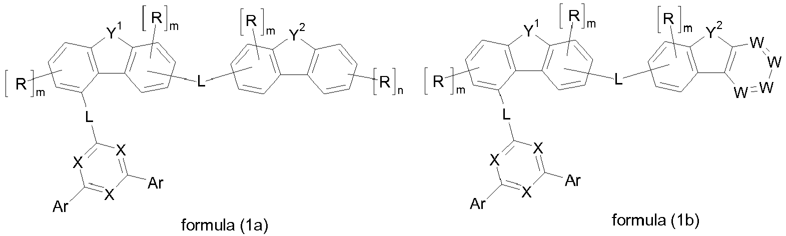 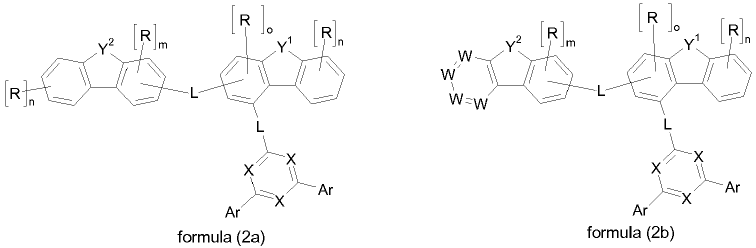 where the following applies:
two adjacent groups W together stand for a group of the following formula (3a) and the other two groups W stand for CR, 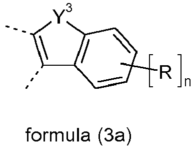 where the dashed bonds indicate the linking of this group;
n is on each occurrence, identically or differently, 0, 1, 2, 3 or 4;
m is on each occurrence, identically or differently, 0, 1, 2 or 3;
o is 0, 1 or 2;
the other symbols used have the meanings given in Claim 1;
where compounds of the formula (2b) in which Y¹ stands for CR₂ are excluded from the invention.

3. Compound according to Claim 1 or 2, **characterised in that** at least two groups X stand for N.

4. Compound according to one or more of Claims 1 to 3, **characterised in that** Y¹ and Y² stand, identically or differently on each occurrence, for O, NR, where the radical R bonded to the nitrogen is not equal to H, or S.

5. Compound according to one or more of Claims 1 to 4, for which the following applies:
X is, identically or differently on each occurrence, CR or N, where at least two groups X stand for N;
Y¹, Y² stand, identically or differently on each occurrence, for O, NR, where the radical R bonded to the nitrogen is not equal to H, or S;
Y³ stands for O, NR, where the radical R bonded to the nitrogen is not equal to H, or CR₂;
L stands, identically or differently on each occurrence, for a single bond or an aromatic or heteroaromatic ring system having 5 to 24 aromatic ring atoms, which may be substituted by one or more radicals R;
Ar stands, identically or differently on each occurrence, for an aromatic or heteroaromatic ring system having 6 to 24 aromatic ring atoms, which may be substituted by one or more radicals R;
n in formula (1a) or (2a) is, identically or differently on each occurrence, 0, 1, 2 or 3;
m in formula (1a) or (2a) is, identically or differently on each occurrence, 0, 1 or 2;
o in formula (1a) or (2a) is 0 or 1;
R is selected, identically or differently on each occurrence, from the group consisting of H, D, F, CN, N(Ar¹)₂, C(=O)Ar¹, P(=O)(Ar¹)2, a straight-chain alkyl or alkoxy group having 1 to 10 C atoms or a branched or cyclic alkyl or alkoxy group having 3 to 10 C atoms or an alkenyl group having 2 to 10 C atoms, which may in each case be substituted by one or more radicals R¹, where one or more non-adjacent CH2 groups may be replaced by O and where one or more H atoms may be replaced by D or F, an aromatic or hetero-aromatic ring system having 5 to 24 aromatic ring atoms, which may in each case be substituted by one or more radicals R¹, or an aralkyl or heteroaralkyl group having 5 to 25 aromatic ring atoms, which may be substituted by one or more radicals R¹; two substituents R that are bonded to the same carbon atom or to adjacent carbon atoms may optionally form a monocyclic or polycyclic, aliphatic, aromatic or heteroaromatic ring system, which may be substituted by one or more radicals R¹.

6. Compound according to one or more of Claims 1 to 5, for which the following applies:
X is N;
Y¹, Y² stand, identically or differently on each occurrence, for O or NR, where the radical R bonded to the nitrogen is not equal to H;
Y³ stands for NR, where the radical R bonded to the nitrogen is not equal to H, or CR₂;
L stands for a single bond;
Ar stands, identically or differently on each occurrence, for an aromatic or heteroaromatic ring system having 6 to 18 aromatic ring atoms, which may in each case be substituted by one or more radicals R;
n in formula (1a) or (2a) is on each occurrence, identically or differently, 0 or 1;
m in formula (1a) or (2a) is 0;
o in formula (1a) or (2a) is 0;
R is selected, identically or differently on each occurrence, from the group consisting of H, D, F, CN, N(Ar¹)₂, a straight-chain alkyl group having 1 to 8 C atoms, or a branched or cyclic alkyl group having 3 to 8 C atoms, or an alkenyl group having 2 to 8 C atoms, which may in each case be substituted by one or more radicals R¹, or an aromatic or heteroaromatic ring system having 6 to 24 aromatic ring atoms, which may in each case be substituted by one or more non-aromatic radicals R¹; two substituents R that are bonded to the same carbon atom or to adjacent carbon atoms may optionally form a monocyclic or polycyclic, aliphatic ring system, which may be substituted by one or more radicals R¹.

7. Compound according to one or more of Claims 1 to 6, **characterised in that** the compound is selected from the formulae (6), (7), (8), (9) and (10), 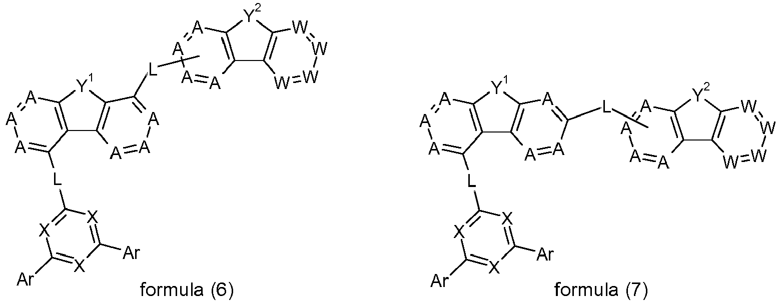 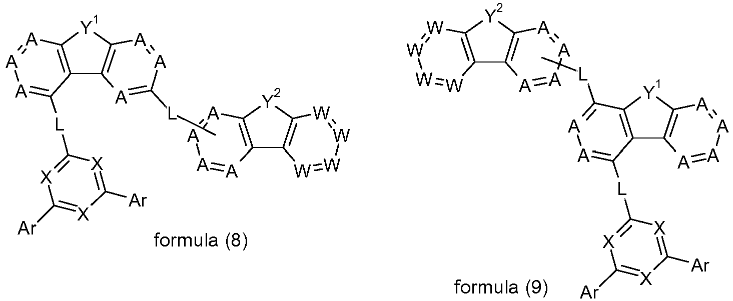 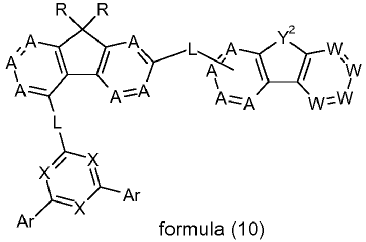 where Y¹ stands for O, NR or S, the other symbols used have the meanings given in Claim 1 and A and W preferably stand, identically or differently on each occurrence, for CR.

8. Compound according to Claim 7, selected from the compounds of the formulae (8a) and (8b), 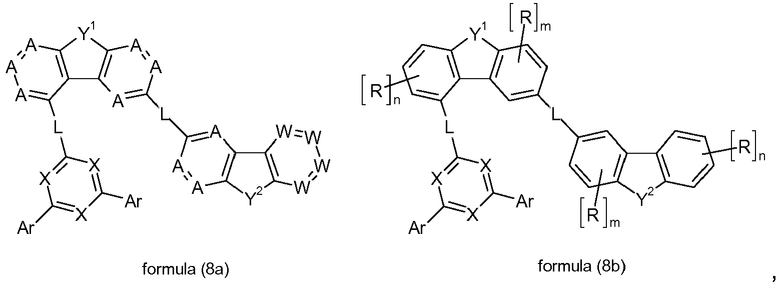 where Y¹ and Y² stand, identically or differently on each occurrence, for O, NR or S and the other symbols and indices used have the meanings given in Claim 1.

9. Compound according to Claim 8 of the formula (8c), 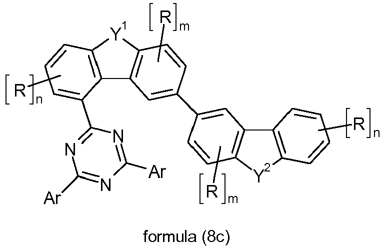 where the following applies for the symbols and indices used:
Y¹, Y² stand, identically or differently on each occurrence, for O, NR, where the radical R bonded to the nitrogen is not equal to H, or S, with the proviso that at least one of the groups Y¹ and/or Y² stands for NR;
Ar stands, identically or differently on each occurrence, for an aromatic or heteroaromatic ring system having 6 to 24 aromatic ring atoms, which may in each case be substituted by one or more radicals R;
n is, identically or differently on each occurrence, 0, 1, 2 or 3;
m is, identically or differently on each occurrence, 0, 1 or 2;
o is 0 or 1;
R is selected, identically or differently on each occurrence, from the group consisting of H, D, F, CN, N(Ar¹)₂, C(=O)Ar¹, P(=O)(Ar¹)₂, a straight-chain alkyl or alkoxy group having 1 to 10 C atoms or a branched or cyclic alkyl or alkoxy group having 3 to 10 C atoms or an alkenyl group having 2 to 10 C atoms, which may in each case be substituted by one or more radicals R¹, where one or more non-adjacent CH2 groups may be replaced by O and where one or more H atoms may be replaced by D or F, an aromatic or hetero-aromatic ring system having 5 to 24 aromatic ring atoms, which may in each case be substituted by one or more radicals R¹, or an aralkyl or heteroaralkyl group having 5 to 25 aromatic ring atoms, which may be substituted by one or more radicals R¹; two substituents R that are bonded to the same carbon atom or to adjacent carbon atoms may optionally form a monocyclic or polycyclic, aliphatic, aromatic or heteroaromatic ring system, which may be substituted by one or more radicals R¹.

10. Compound according to Claim 9 of the formula (8d), 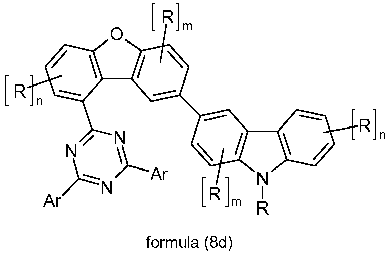 where the following applies for the symbols and indices used:
n is, identically or differently on each occurrence, 0, 1 or 2;
m is, identically or differently on each occurrence, 0 or 1;
the radical R bonded to the nitrogen stands for an aromatic or heteroaromatic ring system having 5 to 24 aromatic ring atoms, which may in each case be substituted by one or more radicals R¹;
the other symbols and indices used have the meanings given under Claim 9.

11. Mixture comprising at least one compound according to one or more of Claims 1 to 10 and at least one further compound and/or at least one solvent.

12. Use of a compound according to one or more of Claims 1 to 10 or a mixture according to Claim 11 in an electronic device.

13. Electronic device containing at least one compound according to one or more of Claims 1 to 10 or a mixture according to Claim 11.

14. Electronic device according to Claim 13, **characterised in that** it is an organic electroluminescent device.

15. Electronic device according to Claim 14, **characterised in that** the compound according to one or more of Claims 1 to 10 is employed in an emitting layer, preferably in combination with a phosphorescent dopant and optionally one or more further matrix materials, or in a hole-blocking layer or in an electron-transport layer.

## Revendications

1. Composé de la formule (1) ou (2) : 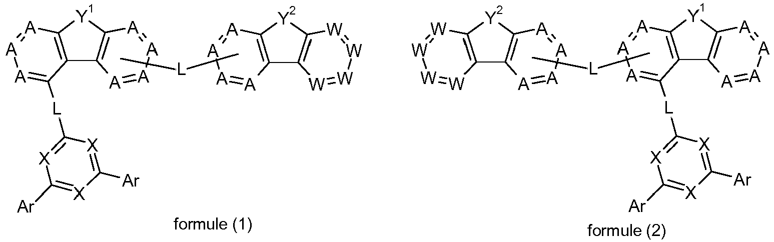 dans lesquelles ce qui suit s'applique aux symboles et aux indices qui sont utilisés :
A est pour chaque occurrence, de manière identique ou différente, CR ou N, où un maximum de deux groupes A par cycle représentent N et où A représente C si un groupe L est lié au niveau de cette position ;
W est pour chaque occurrence, de manière identique ou différente, CR ou N, où un maximum de deux groupes W représentent N, ou deux groupes adjacents W représentent en association un groupe de la formule qui suit (3), où le composé de la formule (1) ou de la formule (2) contient un maximum d'un groupe de la formule (3) : 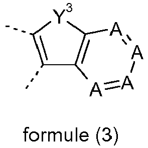 dans laquelle les liens en pointillés indiquent la liaison de ce groupe et A présente les significations qui ont été données ci-avant ; étant entendu que le composé de la formule (2) ne contient pas un groupe de la formule (3) si Y¹ représente CR₂ ;
X est pour chaque occurrence, de manière identique ou différente, CR ou N, étant entendu qu'au moins un groupe X représente N ;
Ar est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R ;
Y¹, Y², Y³ sont pour chaque occurrence, de manière identique ou différente, O, NR, S ou CR₂, où le radical R qui est lié à N n'est pas égal à H ;
L est pour chaque occurrence, de manière identique ou différente, une liaison simple ou un système de cycle aromatique ou hétéroaromatique qui comporte de 5 à 30 atomes de cycle aromatique, lequel peut être substitué par un radical ou par plusieurs radicaux R;
R est sélectionné pour chaque occurrence, de manière identique ou différente, parmi le groupe qui est constitué par H, D, F, Cl, Br, I, CN, NO₂, N(Ar¹)₂, N(R¹)₂, C(=O)Ar¹, C(=O)R¹, P(=O)(Ar')₂, P(Ar¹)₂, B(Ar¹)₂, Si(Ar¹)₃, Si(R¹)₃, un groupe alkyle, alcoxy ou thioalkyle en chaîne droite qui comporte de 1 à 20 atome(s) de C ou un groupe alkyle, alcoxy ou thioalkyle ramifié ou cyclique qui comporte de 3 à 20 atomes de C ou un groupe alkényle qui comporte de 2 à 20 atomes de C, lequel peut dans chaque cas être substitué par un radical ou par plusieurs radicaux R¹, où un ou plusieurs groupe(s) CH2 non adjacents peut/peuvent être remplacé(s) par R¹C=CR¹, Si(R¹)₂, C=O, C=S, C=NR¹, P(=O)(R¹), SO, SO₂, NR¹, O, S ou CONR¹ et où un ou plusieurs atome(s) de H peut/peuvent être remplacé(s) par D, F, Cl, Br, I, CN ou NO₂, un système de cycle aromatique ou hétéroaromatique qui comporte de 5 à 40 atomes de cycle aromatique, lequel peut dans chaque cas être substitué par un radical ou par plusieurs radicaux R¹, un groupe aryloxy ou hétéroaryloxy qui comporte de 5 à 40 atomes de cycle aromatique, lequel peut être substitué par un radical ou par plusieurs radicaux R¹, ou un groupe aralkyle ou hétéroaralkyle qui comporte de 5 à 40 atomes de cycle aromatique, lequel peut être substitué par un radical ou par plusieurs radicaux R¹ ; deux substituants R qui sont liés au même atome de carbone ou à des atomes de carbone adjacents peuvent en option former un système de cycle aliphatique, aromatique ou hétéroaromatique monocyclique ou polycyclique, lequel peut être substitué par un radical ou par plusieurs radicaux R¹;
Ar¹ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux non aromatique(s) R¹ ; deux radicaux Ar¹ qui sont liés au même atome de N, au même atome de P ou au même atome de B peuvent également être pontés l'un à l'autre au moyen d'une liaison simple ou d'un pont qui est sélectionné parmi N(R¹), C(R¹)₂, O ou S ;
R¹ est sélectionné pour chaque occurrence, de manière identique ou différente, parmi le groupe qui est constitué par H, D, F, CN, un radical hydrocarbone aliphatique qui comporte de 1 à 20 atome(s) de C ou un système de cycle aromatique ou hétéroaromatique qui comporte de 5 à 30 atomes de cycle aromatique, où un ou plusieurs atome(s) de H peut/peuvent être remplacé(s) par D, F, Cl, Br, I ou CN, et lequel peut être substitué par un ou plusieurs groupe(s) alkyle, dont chacun comporte de 1 à 4 atome(s) de carbone ; deux substituants R¹ adjacents ou plus peuvent former un système de cycle aliphatique mono- ou polycyclique l'un avec l'autre ou les uns avec les autres ;
les composés qui suivent sont exclus de l'invention :

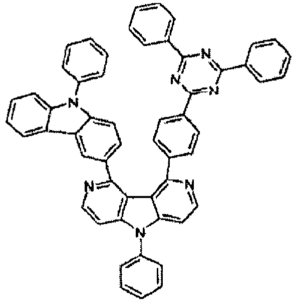

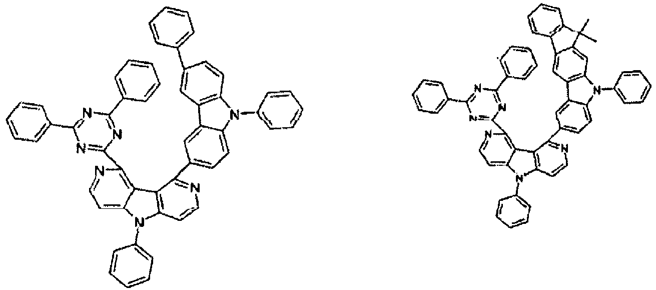

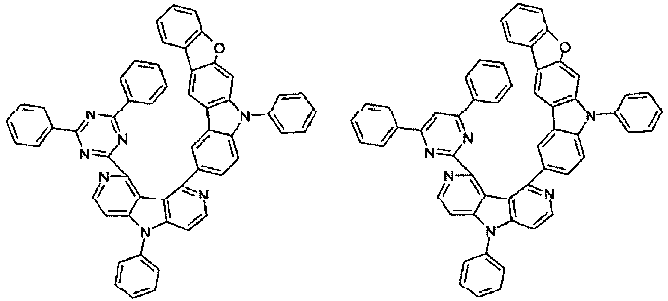

2. Composé selon la revendication 1, **caractérisé en ce que** W représente, de manière identique ou différente pour chaque occurrence, CR et **en ce que** A représente, de manière identique ou différente pour chaque occurrence, CR, sélectionné parmi les composés des formules (1a), (1b), (2a) et (2b): 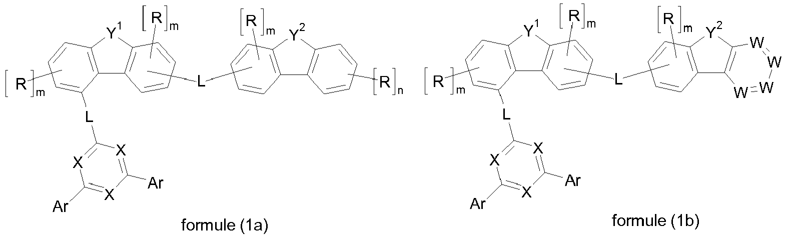 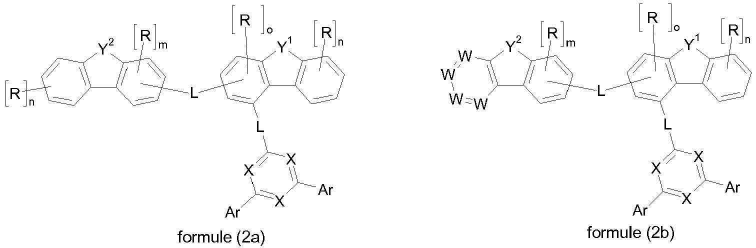 dans lesquelles ce qui suit s'applique :
deux groupes adjacents W représentent en association un groupe de la formule (3a) qui suit et les deux autres groupes W représentent CR : 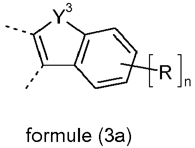 dans laquelle les liens en pointillés indiquent la liaison de ce groupe ;
n est pour chaque occurrence, de manière identique ou différente, 0, 1, 2, 3 ou 4 ;
m est pour chaque occurrence, de manière identique ou différente, 0, 1, 2 ou 3 ;
o est 0, 1 ou 2 ;
les autres symboles qui sont utilisés présentent les significations qui ont été données selon la revendication 1 ;
où les composés de la formule (2b) pour lesquels Y¹ représente CR₂ sont exclus de l'invention.

3. Composé selon la revendication 1 ou 2, **caractérisé en ce qu'**au moins deux groupes X représentent N.

4. Composé selon une ou plusieurs des revendications 1 à 3, **caractérisé en ce que** Y¹ et Y² représentent, de manière identique ou différente pour chaque occurrence, O, NR, où le radical R qui est lié à l'azote n'est égal ni à H, ni à S.

5. Composé selon une ou plusieurs des revendications 1 à 4, pour lequel ce qui suit s'applique :
X est, de manière identique ou différente pour chaque occurrence, CR ou N, où au moins deux groupes X représentent N ;
Y¹, Y² représentent, de manière identique ou différente pour chaque occurrence, O, NR, où le radical R qui est lié à l'azote n'est égal ni à H, ni à S ;
Y³ représente O, NR, où le radical R qui est lié à l'azote n'est égal ni à H, ni à CR₂;
L représente, de manière identique ou différente pour chaque occurrence, une liaison simple ou un système de cycle aromatique ou hétéroaromatique qui comporte de 5 à 24 atomes de cycle aromatique, lequel peut être substitué par un radical ou par plusieurs radicaux R ;
Ar représente, de manière identique ou différente pour chaque occurrence, un système de cycle aromatique ou hétéroaromatique qui comporte de 6 à 24 atomes de cycle aromatique, lequel peut être substitué par un radical ou par plusieurs radicaux R ;
n au niveau de la formule (1a) ou (2a), est, de manière identique ou différente pour chaque occurrence, 0, 1, 2 ou 3 ;
m au niveau de la formule (1a) ou (2a), est, de manière identique ou différente pour chaque occurrence, 0, 1 ou 2 ;
o au niveau de la formule (1a) ou (2a), est 0 ou 1 ;
R est sélectionné, de manière identique ou différente pour chaque occurrence, parmi le groupe qui est constitué par H, D, F, CN, N(Ar¹)₂, C(=O)Ar¹, P(=O)(Ar¹)₂, un groupe alkyle ou alcoxy en chaîne droite qui comporte de 1 à 10 atome(s) de C ou un groupe alkyle ou alcoxy ramifié ou cyclique qui comporte de 3 à 10 atomes de C ou un groupe alkényle qui comporte de 2 à 10 atomes de C, lequel peut dans chaque cas être substitué par un radical ou par plusieurs radicaux R¹, où un ou plusieurs groupe(s) CH2 non adjacents peut/peuvent être remplacé(s) par O et où un ou plusieurs atome(s) de H peut/peuvent être remplacé(s) par D ou par F, un système de cycle aromatique ou hétéroaromatique qui comporte de 5 à 24 atomes de cycle aromatique, lequel peut dans chaque cas être substitué par un radical ou par plusieurs radicaux R¹, ou un groupe aralkyle ou hétéroaralkyle qui comporte de 5 à 25 atomes de cycle aromatique, lequel peut être substitué par un radical ou par plusieurs radicaux R¹ ; deux substituants R qui sont liés au même atome de carbone ou à des atomes de carbone adjacents peuvent en option former un système de cycle aliphatique, aromatique ou hétéroaromatique monocyclique ou polycyclique, lequel peut être substitué par un radical ou par plusieurs radicaux R¹.

6. Composé selon une ou plusieurs des revendications 1 à 5, pour lequel ce qui suit s'applique :
X est N ;
Y¹, Y² représentent, de manière identique ou différente pour chaque occurrence, O ou NR, où le radical R qui est lié à l'azote n'est pas égal à H ;
Y³ représente NR, où le radical R qui est lié à l'azote n'est égal ni à H, ni à CR₂;
L représente une liaison simple ;
Ar représente, de manière identique ou différente pour chaque occurrence, un système de cycle aromatique ou hétéroaromatique qui comporte de 6 à 18 atomes de cycle aromatique, lequel peut dans chaque cas être substitué par un radical ou par plusieurs radicaux R ;
n au niveau de la formule (1a) ou (2a), est pour chaque occurrence, de manière identique ou différente, 0 ou 1 ;
m au niveau de la formule (1a) ou (2a), est 0 ;
o au niveau de la formule (1a) ou (2a), est 0 ;
R est sélectionné, de manière identique ou différente pour chaque occurrence, parmi le groupe qui est constitué par H, D, F, CN, N(Ar¹)₂, un groupe alkyle en chaîne droite qui comporte de 1 à 8 atome(s) de C, ou un groupe alkyle ramifié ou cyclique qui comporte de 3 à 8 atomes de C, ou un groupe alkényle qui comporte de 2 à 8 atomes de C, lequel peut dans chaque cas être substitué par un radical ou par plusieurs radicaux R¹, ou un système de cycle aromatique ou hétéroaromatique qui comporte de 6 à 24 atomes de cycle aromatique, lequel peut dans chaque cas être substitué par un radical ou par plusieurs radicaux non aromatique(s) R¹ ; deux substituants R qui sont liés au même atome de carbone ou à des atomes de carbone adjacents peuvent en option former un système de cycle aliphatique monocyclique ou polycyclique, lequel peut être substitué par un radical ou par plusieurs radicaux R¹.

7. Composé selon une ou plusieurs des revendications 1 à 6, **caractérisé en ce que** le composé est sélectionné parmi les formules (6), (7), (8), (9) et (10) : 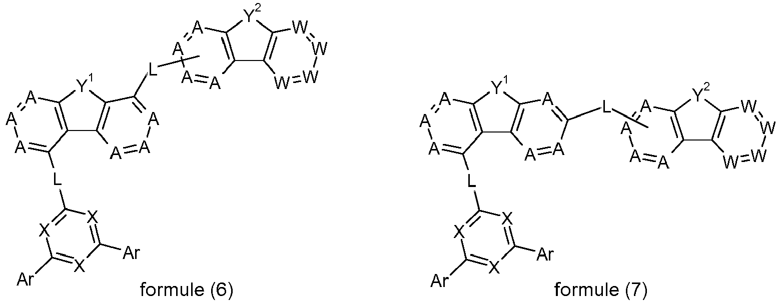 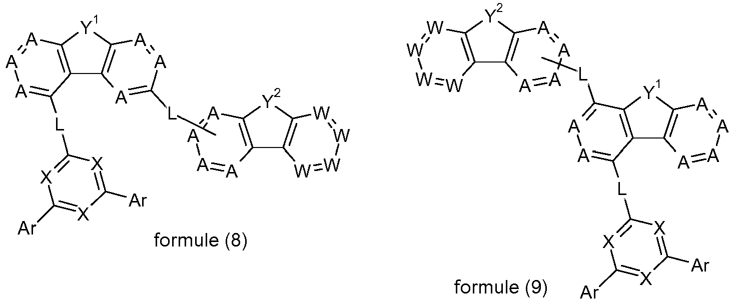 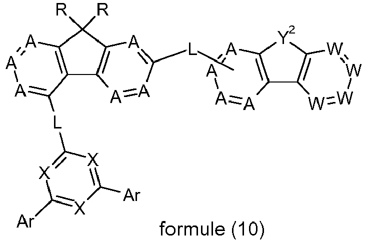 dans lesquelles Y¹ représente O, NR ou S, les autres symboles qui sont utilisés présentent les significations qui ont été données selon la revendication 1, et A et W représentent de préférence, de manière identique ou différente pour chaque occurrence, CR.

8. Composé selon la revendication 7, sélectionné parmi les composés des formules (8a) et (8b) : 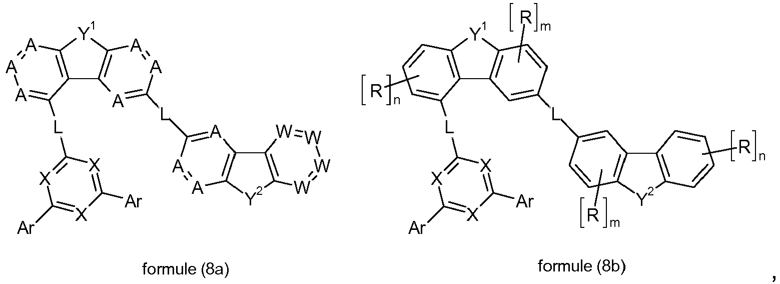 dans lesquelles Y¹ et Y² représentent, de manière identique ou différente pour chaque occurrence, O, NR ou S et les autres symboles et les autres indices qui sont utilisés présentent les significations qui ont été données selon la revendication 1.

9. Composé selon la revendication 8 de la formule (8c) : 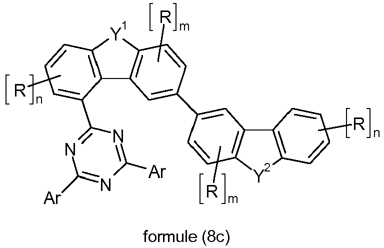 dans laquelle ce qui suit s'applique aux symboles et aux indices qui sont utilisés :
Y¹, Y² représentent, de manière identique ou différente pour chaque occurrence, O, NR, où le radical R qui est lié à l'azote n'est égal ni à H, ni à S, étant entendu qu'au moins l'un des groupes Y¹ et/ou Y² représente NR ;
Ar représente, de manière identique ou différente pour chaque occurrence, un système de cycle aromatique ou hétéroaromatique qui comporte de 6 à 24 atomes de cycle aromatique, lequel peut dans chaque cas être substitué par un radical ou par plusieurs radicaux R ;
n est, de manière identique ou différente pour chaque occurrence, 0, 1, 2 ou 3 ;
m est, de manière identique ou différente pour chaque occurrence, 0, 1 ou 2 ;
o est 0 ou 1 ;
R est sélectionné, de manière identique ou différente pour chaque occurrence, parmi le groupe qui est constitué par H, D, F, CN, N(Ar¹)₂, C(=O)Ar¹, P(=O)(Ar¹)₂, un groupe alkyle ou alcoxy en chaîne droite qui comporte de 1 à 10 atome(s) de C ou un groupe alkyle ou alcoxy ramifié ou cyclique qui comporte de 3 à 10 atomes de C ou un groupe alkényle qui comporte de 2 à 10 atomes de C, lequel peut dans chaque cas être substitué par un radical ou par plusieurs radicaux R¹, où un ou plusieurs groupe(s) CH2 non adjacents peut/peuvent être remplacé(s) par O et où un ou plusieurs atome(s) de H peut/peuvent être remplacé(s) par D ou par F, un système de cycle aromatique ou hétéroaromatique qui comporte de 5 à 24 atomes de cycle aromatique, lequel peut dans chaque cas être substitué par un radical ou par plusieurs radicaux R¹, ou un groupe aralkyle ou hétéroaralkyle qui comporte de 5 à 25 atomes de cycle aromatique, lequel peut être substitué par un radical ou par plusieurs radicaux R¹ ; deux substituants R qui sont liés au même atome de carbone ou à des atomes de carbone adjacents peuvent en option former un système de cycle aliphatique, aromatique ou hétéroaromatique monocyclique ou polycyclique, lequel peut être substitué par un radical ou par plusieurs radicaux R¹.

10. Composé selon la revendication 9 de la formule (8d) : 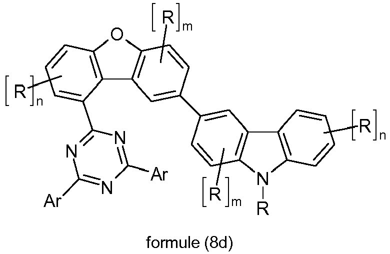 dans laquelle ce qui suit s'applique aux symboles et aux indices qui sont utilisés :
n est, de manière identique ou différente pour chaque occurrence, 0, 1 ou 2 ;
m est, de manière identique ou différente pour chaque occurrence, 0 ou 1 ;
le radical R qui est lié à l'azote représente un système de cycle aromatique ou hétéroaromatique qui comporte de 5 à 24 atomes de cycle aromatique, lequel peut dans chaque cas être substitué par un radical ou par plusieurs radicaux R¹ ;
les autres symboles et les autres indices qui sont utilisés présentent les significations qui ont été données selon la revendication 9.

11. Mélange comprenant au moins un composé selon une ou plusieurs des revendications 1 à 10 et au moins un autre composé et/ou au moins un solvant.

12. Utilisation d'un composé selon une ou plusieurs des revendications 1 à 10 ou d'un mélange selon la revendication 11 à l'intérieur d'un dispositif électronique.

13. Dispositif électronique contenant au moins un composé selon une ou plusieurs des revendications 1 à 10 ou un mélanges selon la revendication 11.

14. Dispositif électronique selon la revendication 13, **caractérisé en ce qu'**il s'agit d'un dispositif électroluminescent organique.

15. Dispositif électronique selon la revendication 14, **caractérisé en ce que** le composé selon une ou plusieurs des revendications 1 à 10 est utilisé à l'intérieur d'une couche d'émission, de préférence en combinaison avec un dopant phosphorescent et en option, avec un autre ou plusieurs autres matériau(x) de matrice, ou à l'intérieur d'une couche de blocage de trous ou à l'intérieur d'une couche de transport d'électrons.
